(19) Europäisches Patentamt
European Patent Office
Office européen des brevets

(11) EP 2 993 236 A1

# (12) EUROPEAN PATENT APPLICATION

(43) Date of publication:
**09.03.2016 Bulletin 2016/10**

(51) Int Cl.:
***C12Q 1/37*** *(2006.01)*

(21) Application number: **15185484.1**

(22) Date of filing: **15.04.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.04.2010 US 324579 P**

(62) Document number(s) of the earlier application(s) in accordance with Art. 76 EPC:
**11769642.7 / 2 558 589**

(71) Applicant: **GlaxoSmithKline LLC**
**Wilmington DE 19808 (US)**

(72) Inventors:
- **DAI, Han**
  **Cambridge, MA 02139 (US)**
- **RIERA, Thomas V.**
  **Cambridge, MA 02139 (US)**
- **STEIN, Ross L.**
  **Cambridge, MA 02139 (US)**
- **SZCZEPANKIEWICZ, Bruce**
  **Cambridge, MA 02139 (US)**

(74) Representative: **Valentine, Jill Barbara et al**
**GlaxoSmithKline**
**Global Patents (CN925.1)**
**980 Great West Road**
**Brentford, Middlesex TW8 9GS (GB)**

Remarks:
This application was filed on 16-09-2015 as a divisional application to the application mentioned under INID code 62.

## (54) SIRTUIN ACTIVATORS AND ACTIVATION ASSAYS

(57) Provided are methods and compositions for detecting a compound that activates a sirtuin deacetylase activity on a fluorescent-free activation substrate in vitro. Further provided are sirtuin modulating compounds of the formulas (I) - (XXI), and related compounds (XXXI), (XXXII), (XXXIII), and (XXXIV), including the fluorescent free-substrate SIRT1 activator compounds of formulas (XL), (XI), (XII), and (XIII).

EP 2 993 236 A1

SEQUENCE LISTING

<110> SIRTRIS PHARMACEUTICALS, INC.
STEIN, Ross L.
DAI, Han
RIERA, Thomas V.
SZCZEPANKIEWICZ, Bruce G.

<120> SIRTUIN ACTIVATORS AND ACTIVATION ASSAYS

<130> SIRT062

<140> PCT/US2011/032628
<141> 2011-04-15

<150> 61/324,579
<151> 2010-04-15

<160> 22

<170> FastSEQ for Windows Version 4.0

<210> 1
<211> 4
<212> PRT
<213> Artificial Sequence

<220>
<223> P53 Artificial sequence

<220>
<221> ACETYLATION
<222> (1)...(1)

<220>
<221> ACETYLATION
<222> (4)...(4)

<220>
<221> UNSURE
<222> (4)...(4)
<223> 7-amido-4-methyl coumarin

<400> 1
Arg His Lys Lys
 1

<210> 2
<211> 5
<212> PRT
<213> Artificial Sequence

<220>
<223> P53 Artificial sequence

<220>
<221> ACETYLATION
<222> 1, 4

<400> 2

Arg His Lys Lys Phe
1 5

<210> 3
<211> 5
<212> PRT
<213> Artificial Sequence

<220>
<223> P53 Artificial sequence

<220>
<221> ACETYLATION
<222> 1, 4

<400> 3
Arg His Lys Lys Trp
1 5

<210> 4
<211> 5
<212> PRT
<213> Artificial Sequence

<220>
<223> P53 Artificial sequence

<220>
<221> ACETYLATION
<222> 1, 4

<400> 4
Arg His Lys Lys Ala
1 5

<210> 5
<211> 20
<212> PRT
<213> Artificial Sequence

<220>
<223> P53 Artificial sequence

<220>
<221> ACETYLATION
<222> 1, 12

<220>
<221> MOD_RES
<222> 13
<223> Nle

<220>
<221> MOD_RES
<222> 3
<223> biotin

<220>
<221> MOD_RES
<222> 18

<223> tetramethyl-6-carboxyrhodamine

<220>
<221> AMIDATION
<222> 20
<223> NH2

<400> 5
Glu Glu Lys Gly Gln Ser Thr Ser Ser His Ser Lys Leu Ser Thr Glu
1               5                   10                  15
Gly Lys Glu Glu
            20

<210> 6
<211> 20
<212> PRT
<213> Artificial Sequence

<220>
<223> P53 Artificial sequence

<220>
<221> ACETYLATION
<222> 1, 12

<220>
<221> MOD_RES
<222> 3
<223> biotin

<220>
<221> MOD_RES
<222> 13
<223> Nle

<220>
<221> AMIDATION
<222> 20
<223> NH2

<400> 6
Glu Glu Lys Gly Gln Ser Thr Ser Ser His Ser Lys Leu Ser Thr Glu
1               5                   10                  15
Gly Lys Glu Glu
            20

<210> 7
<211> 5
<212> PRT
<213> Artificial Sequence

<220>
<223> P53 Artificial sequence

<220>
<221> ACETYLATION
<222> 1, 4

<220>
<221> AMIDATION

<222> 5
<223> NH2

<400> 7
Arg His Lys Lys Trp
1 5

<210> 8
<211> 5
<212> PRT
<213> Artificial Sequence

<220>
<223> P53 Artificial sequence

<220>
<221> ACETYLATION
<222> 1, 4

<220>
<221> AMIDATION
<222> 5
<223> NH2

<400> 8
Arg His Lys Lys Phe
1 5

<210> 9
<211> 5
<212> PRT
<213> Artificial Sequence

<220>
<223> P53 Artificial sequence

<220>
<221> ACETYLATION
<222> 1, 4

<220>
<221> VARIANT
<222> 5
<223> Xaa = Any Amino Acid

<400> 9
Arg His Lys Lys Xaa
1 5

<210> 10
<211> 5
<212> PRT
<213> Artificial Sequence

<220>
<223> P53 Artificial sequence

<220>
<221> ACETYLATION
<222> 1, 4

<220>
<221> AMIDATION
<222> 5
<223> NH2

<400> 10
Arg His Lys Lys Trp
 1               5

<210> 11
<211> 4
<212> PRT
<213> Artificial Sequence

<220>
<223> P53 Artificial sequence

<400> 11
Arg His Lys Lys
 1

<210> 12
<211> 5
<212> PRT
<213> Artificial Sequence

<220>
<223> P53 Artificial sequence

<220>
<221> ACETYLATION
<222> 1, 4

<220>
<221> AMIDATION
<222> 5
<223> NH2

<400> 12
Arg His Lys Lys Phe
 1               5

<210> 13
<211> 19
<212> PRT
<213> Artificial Sequence

<220>
<223> P53 Artificial sequence

<220>
<221> MOD_RES
<222> 3
<223> biotin

<220>
<221> ACETYLATION
<222> 11

<220>
<221> MOD_RES
<222> (12)...(12)
<223> Nle

<220>
<221> MOD_RES
<222> 17
<223> tetramethyl-6-carboxyrhodamine

<400> 13
Glu Glu Lys Gly Gln Ser Thr Ser Ser His Lys Leu Ser Thr Glu Gly
1 5 10 15
Lys Glu Glu

<210> 14
<211> 19
<212> PRT
<213> Artificial Sequence

<220>
<223> P53 Artificial sequence

<220>
<221> MOD_RES
<222> 3
<223> biotin

<220>
<221> ACETYLATION
<222> 11

<220>
<221> MOD_RES
<222> 12
<223> Nle

<400> 14
Glu Glu Lys Gly Gln Ser Thr Ser Ser His Lys Leu Ser Thr Glu Gly
1 5 10 15
Lys Glu Glu

<210> 15
<211> 19
<212> PRT
<213> Artificial Sequence

<220>
<223> P53 Artificial sequence

<220>
<221> ACETYLATION
<222> 11

<220>
<221> MOD_RES
<222> 12
<223> Nle

<400> 15
Glu Glu Lys Gly Gln Ser Thr Ser Ser His Lys Leu Ser Thr Glu Gly
 1               5                   10                  15
Lys Glu Glu

<210> 16
<211> 19
<212> PRT
<213> Artificial Sequence

<220>
<223> P53 Artificial sequence

<220>
<221> ACETYLATION
<222> 12

<220>
<221> MOD_RES
<222> 14
<223> Nle

<400> 16
Ser Lys Lys Gly Gln Ser Thr Ser Arg His Lys Lys Leu Leu Lys Thr
 1               5                   10                  15
Glu Gly Pro

<210> 17
<211> 4
<212> PRT
<213> Artificial Sequence

<220>
<223> P53 Artificial sequence

<220>
<221> ACETYLATION
<222> 4

<220>
<221> UNSURE
<222> 4
<223> 7-amido-4-methyl coumarin

<400> 17
Arg His Lys Lys
 1

<210> 18
<211> 4
<212> PRT
<213> Artificial Sequence

<220>
<223> P53 Artificial sequence

<220>
<221> ACETYLATION

<222> 4

<400> 18
Arg His Lys Lys
 1

<210> 19
<211> 5
<212> PRT
<213> Artificial Sequence

<220>
<223> P53 Artificial sequence

<220>
<221> ACETYLATION
<222> 4

<400> 19
Arg His Lys Lys Ala
 1               5

<210> 20
<211> 5
<212> PRT
<213> Artificial Sequence

<220>
<223> P53 Artificial sequence

<220>
<221> ACETYLATION
<222> 4

<400> 20
Arg His Lys Lys Phe
 1               5

<210> 21
<211> 5
<212> PRT
<213> Artificial Sequence

<220>
<223> P53 Artificial sequence

<220>
<221> ACETYLATION
<222> 4

<400> 21
Arg His Lys Lys Trp
 1               5

<210> 22
<211> 4
<212> PRT
<213> Artificial Sequence

<220>
<223> P53 Artificial sequence

<220>
<221> ACETYLATION
<222> 1, 4

<220>
<221> AMIDATION
<222> 4
<223> NH2

<400> 22
Arg His Lys Lys
1

**Description**

## BACKGROUND

The Silent Information Regulator (SIR) family of genes represents a highly conserved group of genes present in the genomes of organisms ranging from archaebacteria to eukaryotes. The encoded SIR proteins are involved in diverse processes from regulation of gene silencing to DNA repair. The proteins encoded by members of the SIR gene family show high sequence conservation in a 250 amino acid core domain. A well-characterized gene in this family is S. cerevisiae SIR2, which is involved in silencing HM loci that contain information specifying yeast mating type, telomere position effects and cell aging. The yeast Sir2 protein belongs to a family of histone deacetylases. The Sir2 homolog, CobB, in Salmonella typhimurium, functions as an NAD (nicotinamide adenine dinucleotide)-dependent ADP-ribosyl transferase.

The Sir2 protein is a class III deacetylase which uses $NAD^+$ as a cosubstrate. Unlike other deacetylases, many of which are involved in gene silencing, Sir2 is insensitive to class I and II histone deacetylase inhibitors like trichostatin A (TSA).

Deacetylation of acetyl-lysine by Sir2 is tightly coupled to $NAD^+$ hydrolysis, producing nicotinamide and a novel acetyl-ADP ribose compound. The $NAD^+$-dependent deacetylase activity of Sir2 is essential for its functions which can connect its biological role with cellular metabolism in yeast. Mammalian Sir2 homo logs have $NAD^+$-dependent histone deacetylase activity.

Biochemical studies have shown that Sir2 can readily deacetylate the amino-terminal tails of histones H3 and H4, resulting in the formation of 1-O-acetyl-ADP-ribose and nicotinamide. Strains with additional copies of SIR2 display increased rDNA silencing and a 30% longer life span. It has recently been shown that additional copies of the C. elegans SIR2 homolog, sir-2.1, and the *D. melanogaster* dSir2 gene greatly extend life span in those organisms. This implies that the SIR2-dependent regulatory pathway for aging arose early in evolution and has been well conserved. Today, Sir2 genes are believed to have evolved to enhance an organism's health and stress resistance to increase its chance of surviving adversity.

In humans, there are seven Sir2-like genes (SIRT1-SIRT7) that share the conserved catalytic domain of Sir2. SIRT1 is a nuclear protein with the highest degree of sequence similarity to Sir2. SIRT1 regulates multiple cellular targets by deacetylation including the tumor suppressor p53, the cellular signaling factor NF-κB, and the FOXO transcription factor.

SIRT3 is a homolog of SIRT1 that is conserved in prokaryotes and eukaryotes. The SIRT3 protein is targeted to the mitochondrial cristae by a unique domain located at the N-terminus. SIRT3 has $NAD^+$-dependent protein deacetylase activity and is ubiquitously expressed, particularly in metabolically active tissues. Upon transfer to the mitochondria, SIRT3 is believed to be cleaved into a smaller, active form by a mitochondrial matrix processing peptidase (MPP).

Caloric restriction has been known for over 70 years to improve the health and extend the lifespan of mammals. Yeast life span, like that of metazoans, is also extended by interventions that resemble caloric restriction, such as low glucose. The discovery that both yeast and flies lacking the SIR2 gene do not live longer when calorically restricted provides evidence that SIR2 genes mediate the beneficial health effects of a restricted calorie diet. Moreover, mutations that reduce the activity of the yeast glucose-responsive cAMP (adenosine 3',5'-monophosphate)-dependent (PKA) pathway extend life span in wild type cells but not in mutant sir2 strains, demonstrating that SIR2 is likely to be a key downstream component of the caloric restriction pathway.

Furthermore, studies in which SIRT1 protein and activity levels have been manipulated, through gene-deletion or over-expression in mice, have validated the beneficial impact of increased SIRT1 activity in several models of disease including those involving metabolic stress (Haigis, M. C., and Sinclair, D. (2010) Annu Rev Pathol 5, 253-259; Baur, J. A. (2010) Mech Aging Dev). This has recently been observed in humans as well, where reduced SIRT1 expression in insulin-sensitive tissues was associated with reduced energy expenditure (Rutanen et. al. (2010) Diabetes). Therefore, for many of the diseases in which SIRT1 is thought to play a role, therapeutic effects are predicted to follow from the administration of activators of this enzyme's deacetylase activity. Over the past several years, SIRT1 activating compounds (STACs), including resveratrol and more specific, chemically distinct molecules, have been developed (Milne et al. (2007) Nature 450, 712-716; Bemis et al. (2009) Bioorg Med Chem Lett 19, 2350-2353; Vu et al. (2009) J. Med. Chem.). When tested in cell-based and animal models of these diseases, STACs produce effects consistent with direct activation of this enzyme (Milne et al. (2007) Nature 450, 712-716; Feige et al. (2008) Cell Metab 8, 347-358; Funk et al. J Pharmacol Exp Ther; Jin et al. (2009) Protein Sci 18, 514-525; Liu et al. (2008) Nature 456, 269-273; Smith et al. (2009) BMC Syst Biol 3, 31; Yamazaki et al. (2009) Am J Physiol Endocrinol Metab; Yoshizaki et al. (2009) Mol Cell Biol 29, 1363-1374).

At the molecular level, much remains to be learned concerning the mechanism by which these compounds accelerate SIRT1-catalyzed deacetylation. One area of interest is the dependence of activation on structural features of peptide substrates.

This aspect of SIRT1 activation first came to light in 2005, when two papers reported that resveratrol can activate the SIRT1-catalyzed deacetylation of Ac-Arg-His-Lys-Lys$^{Ac}$-AMC, but not the simple amide or acid of this peptide (see Howitz et al. (2003) Nature 425, 191-196; Borra et al. (2005) J. Biol. Chem. 280, 17187-17195; Kaeberlein et al. (2005) J. Biol. Chem. 280, 17038-17045). Recently, the results with resveratrol were confirmed (Beher et al. (2009) Chem Biol Drug Des), and extended by Pacholec et al. (Pacholec et al. (2010) J. Biol. Chem. 285, 8340-8351) to include three previously reported STACs, SRT1460, SRT1720, and SRT2183, originally described by Milne et al. ((2007) Nature 450, 712-716) (see Figure 1). Pacholec et al. ((2010) J. Biol. Chem. 285, 8340-8351) investigated the STAC-mediated activation of SIRT1 using several acetylated peptide substrate, including the TAMRA-labeled 20mer (TAMRA-peptide; see Table 3 for structure of this and other peptide substrates) used by Milne et al. ((2007) Nature 450, 712-716), and two protein substrates of SIRT1. The primary observations of this study were (i) the presence of the TAMRA-label is necessary for activation, since no activation was observed with unmodified peptides or the protein substrates, (ii) SRT1460 and SRT1720 can bind to TAMRA-peptide, and (iii) SRT1460 interacts with the SIRT1:TAMRA-peptide complex. These observations led the authors to conclude that SIRT1 activation by STACs must be through an "indirect" mechanism involving the formation of a complex between activator and TAMRA-peptide. Although Pacholec et al. did not advance a specific mechanistic hypothesis, activation of SIRT1 presumably results from favorable kinetics of the SIRT1-catalyzed turnover of this complex. This proposal, which we term 'activation by substrate enhancement', is shown in schematic form in Figure 2A. A thorough analysis of the mechanistic proposal of Figure 2A to determine if it can account for STAC-mediated activation of SIRT1 is needed.

The invention provides a better understanding of the mechanism of action of sirtuin activating compounds which demonstrates the inadequacy of the indirect mechanism of activation by substrate enhancement to explain activation of SIRT1 by STACs. In particular, while the mechanistic proposal of Figure 2A demands a correlation between the activation efficacy of STACs and their affinity for substrates, none exists. In addition, some STACs can be shown to bind to free SIRT1. Such binding is not accommodated by the mechanism of Figure 2A. Finally, data sets for the activation of SIRT1 by STACs cannot be successfully fit to the rate law for the mechanism of Figure 2A. In combination, these results point to the inadequacy of the indirect mechanism of activation by substrate enhancement, to explain activation of SIRT1 by STACs.

Accordingly, a better understanding of the mechanism of action of sirtuin activating compounds is needed to advance an understanding of sirtuin function and to develop enhanced screens for new activator compounds.

## SUMMARY

The invention is based, in part, upon the discovery of unique features of sirtuin substrate structure that determine the features of SIRT1 activator compound (STAC) sirtuin enzyme activation. In particular, certain STACs can accelerate the deacetylation of the TAMRA-peptide analog that lacks a TAMRA fluorescent group (i.e., desTAMRA-peptide) but contains another bulky group such as biotin, as well as certain unlabeled peptides comprising only natural amino acids (e.g., Ac-Arg-His-Lys-Lys$^{Ac}$-Phe and Ac-Arg-His-Lys-Lys$^{Ac}$-Trp), but not others (e.g., Ac-Arg-His-Lys-Lys$^{Ac}$-Ala). Accordingly, the invention provides fluorescent group-free peptide, polypeptide, and protein substrates that include an "activation cofactor" moiety, such as an indole (Trp) or phenyl group (Phe), or an activation cofactor-bearing accessory protein such as DBC1 (deleted in breast cancer 1), HIC1 (hypermethylated in cancer 1), AROS (active regulator of SIRT1), or CLOCK (Hirayma et al. (2007) Nature 450: 1086-90; Sassone-Corsi et al. (2008) Cell 134, 329-340), as well as associated methods of use of these substrates to detect compounds that activate a sirtuin deacetylase activity, such as SIRT1. The invention further provides certain novel STAC compounds that can activate the fluorescent-free activation substrates.

In addition, the invention provides novel SIRT1 activator compounds, salts thereof, and associated pharmaceutical compositions.

In one aspect, the invention provides a method of detecting a compound that activates a sirtuin deacetylase activity on a fluorescent-free activation substrate *in vitro*. The method includes the step of contacting the sirtuin deacetylase with a candidate compound and a fluorescent-free activation substrate, such as an acetylated peptide, polypeptide, or protein substrate of the sirtuin, and then detecting the level of sirtuin deacetylase activity on the fluorescent-free activation substrate in the presence of the candidate compound. The level of sirtuin deacetylase activity on the fluorescent-free activation substrate in the presence of the candidate compound is then compared to the level of sirtuin deacetylase activity on the fluorescent-free activation substrate in the absence of the candidate compound. Accordingly, an increase in the level of sirtuin deacetylase activity on the fluorescent-free activation substrate in the presence of the candidate compound compared to the level of sirtuin deacetylase activity on the fluorescent-free activation substrate in the absence of the candidate compound indicates that the compound is a sirtuin activator. In preferred embodiments, the fluorescent-free acetylated peptide, polypeptide or protein is acetylated on an epsilon amino group of a lysine amino acid residue.

In certain preferred embodiments, the sirtuin deacetylase used in this method of the invention is SIRT1. In other embodiments, the sirtuin deacetylase is SIRT2, SIRT3, SIRT4, or SIRT5.

In particular embodiments, the candidate compound is a SIRT1 activator of a fluorescent group-containing peptide substrate (e.g., TAMRA-peptide Ac-EEK$^{(biotin)}$GQSTSSHSK$^{Ac}$Nle-STEGK$^{(5-TMR)}$EE-$NH_2$).

In preferred embodiments, the sirtuin deacetylase is contacted with a fluorescent-free activation substrate and a candidate compound in the presence of $NAD^+$. In other embodiments the sirtuin deacetylase is contacted with a fluorescent-free activation substrate and a candidate compound in the presence of a hydrolysable $NAD^+$analog.

In certain preferred embodiments, the level of sirtuin deacetylase activity is detected by measuring the rate of fluorescent-free activation substrate deacetylation. In other embodiments, the level of sirtuin deacetylase activity is detected by measuring the rate of $NAD^+$ hydrolysis.

In further embodiments, the fluorescent-free activation substrate is a biotinylated polypeptide. In preferred embodiments, the fluorescent-free activation substrate is an acetylated peptide or polypeptide free of the fluorescent groups TAMRA (tetramethyl-6-carboxyrhodamine) and AMC (7-amido-4-methyl coumarin). In particular embodiments, the fluorescent-free activation substrate is the acetylated desTAMRA-peptide Ac-EEK$^{(biotin)}$GQSTSSHSK$^{Ac}$NleSTEG-KEE-$NH_2$. In further embodiments, the fluorescent-free activation substrate is an acetylated peptide selected from the group consisting of Ac-RHKK$^{Ac}$F-$NH_2$ and Ac-RHKK$^{Ac}$W-$NH_2$. In other embodiments, the fluorescent-free activation substrate for use in the above method of the invention comprises an acetylated peptide, polypeptide, or protein substrate of the sirtuin in combination with an activation cofactor-bearing accessory protein or ligand. For example, the activation cofactor-bearing accessory protein might be the DBC1 (deleted in breast cancer 1) protein, the HIC1 (hypermethylated in cancer 1) protein, the AROS (active regulator of SIRT1) protein, or the CLOCK and/or BMAL protein. In particular embodiments, the acetylated protein is histone H1, histone H3, histone H4, p53, p300, FOXO 1, FOXO 3a, FOXO 4, p65, HIVTat, PGC-1$\alpha$, PCAF, MyoD, PPAR$\gamma$, or Ku70.

In certain embodiments of the method of the invention, the compound is an activator of SIRT1 deacetylation of a fluorescent-free activation substrate. In particular embodiments, the activator of SIRT1 deacetylation of a fluorescent-free activation substrate has a structure according to the following formula (XL):

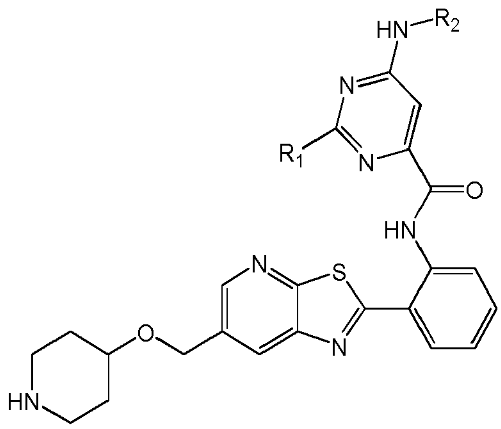

(XL),

or a salt thereof, wherein $R_1$ is selected from $-(CH_2)_3-CH_3$, and $-(CH_2)CH(CH_3)_2$; and $R_2$ is-piperidine or $-(CH_2)_2-NH-CH_3$. In certain preferred embodiments, the compound, or salt thereof, has the formula (XI), (XII), or (XIII):

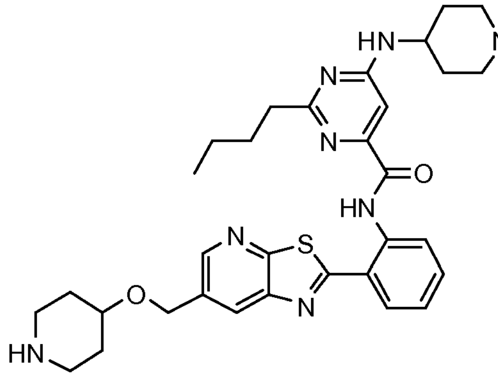

(XI),

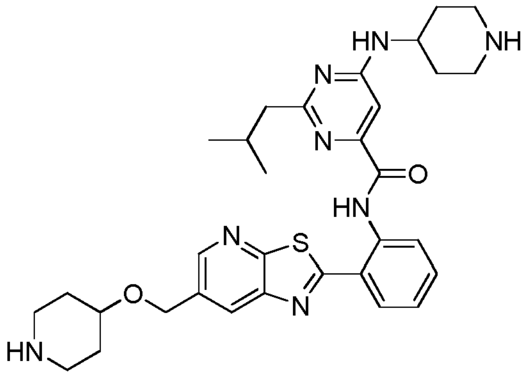

(XII), and

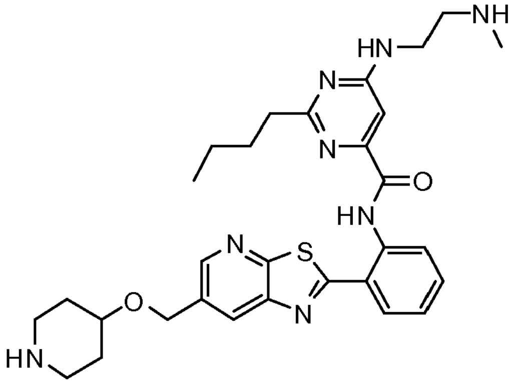

(XIII).

In still further embodiments of the method of the invention, the invention further includes the steps of selecting a candidate compound that is a SIRT1 activator, and contacting the SIRT1 activator with a test cell and detecting a SIRT1 activation-specific change in the test cell. In certain embodiments, the SIRT1 activation-specific change is an increase in FGF21 production. In other embodiments, the SIRT1 activation-specific change is a decrease in LPS-induced TNFα production.

In other embodiments, the method of the invention further includes the steps of selecting a candidate compound that is a SIRT1 activator, and administering the SIRT1 activator to a test subject and detecting a SIRT1 activation-specific change in the test subject. In certain embodiments, the test subject is a diabetic model mouse and the SIRT1 activation specific change is a lowering of blood glucose. In other embodiments the test subject is a neurodegenerative disease model mouse and the SIRT1 activation specific change is a decrease in a neurodegenerative disease process or marker (e.g. for Alzheimer's, Huntington's, amyotrophic lateral sclerosis (ALS), Parkinson's disease, Huntington's disease, or multiple sclerosis (MS)).

In another aspect, the invention provides a fluorescent-free sirtuin substrate having the structural formula Ac-RHKK$^{Ac}$F-$NH_2$ or Ac-RHKK$^{Ac}$W-$NH_2$.

In further aspects, the invention provides sirtuin-modulating compounds of Structural Formulas (I), (II), (III), (IV), (V), (VI), (VII), (VIII), (IX), (X), (XI), (XII) (XIII), (XIV), (XV), (XVI), (XVII), (XVIII), (XIX), (XX), (XXI):

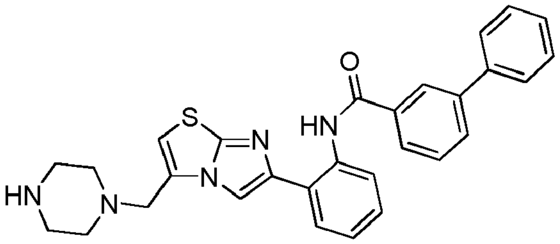

(I),

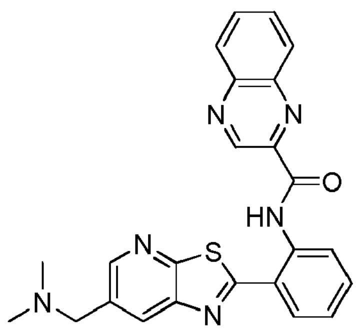

(II),

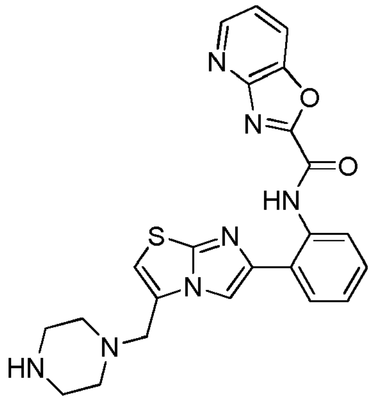
(III),

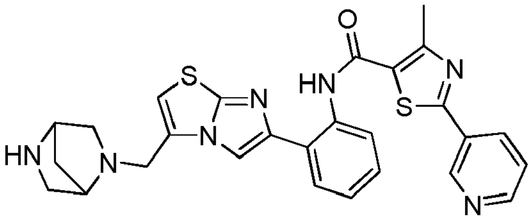
(IV),

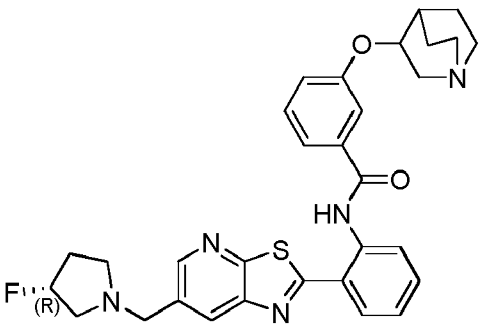
(V),

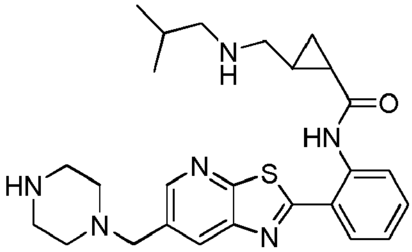
(VI),

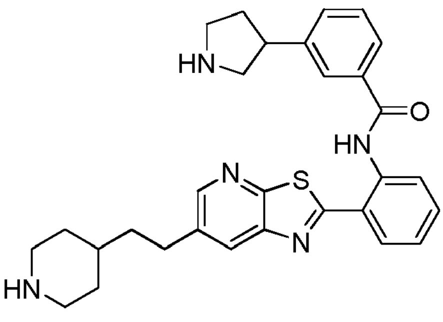
(VII),

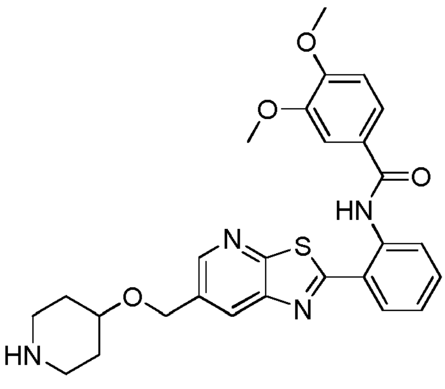
(VIII),

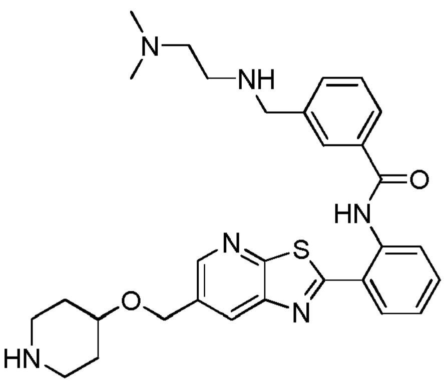
(IX),

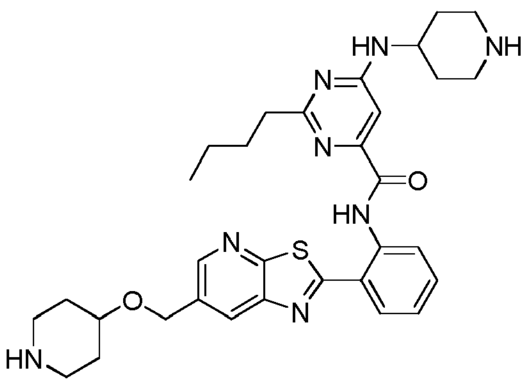
(X),

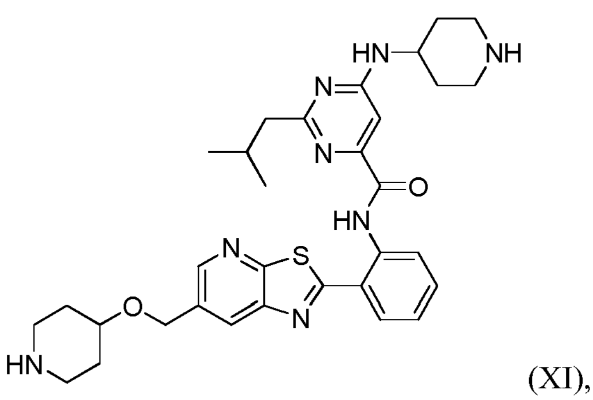

(XI),

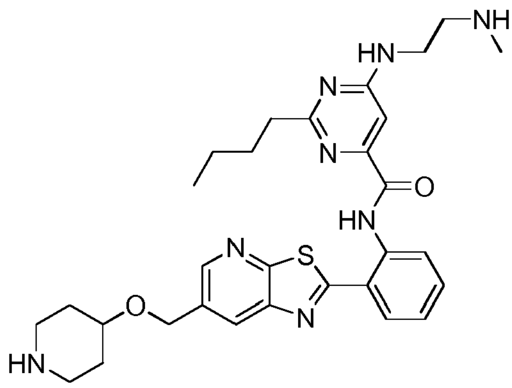

(XII),

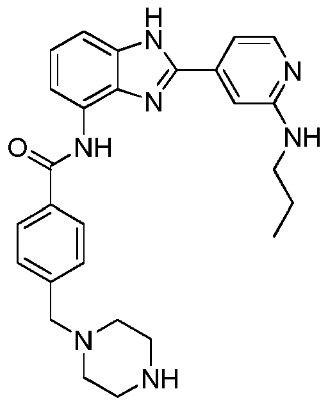

(XIII),

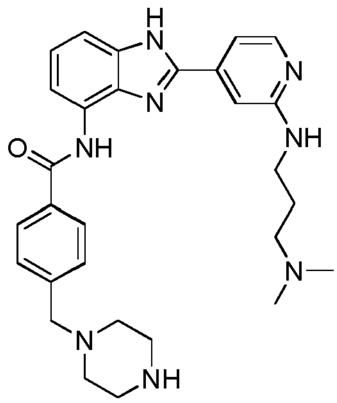

(XIV),

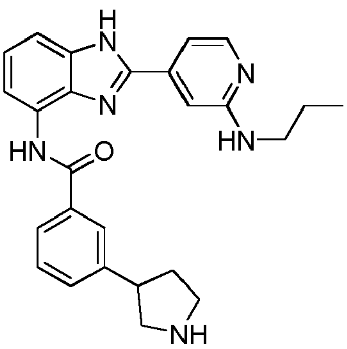

(XV),

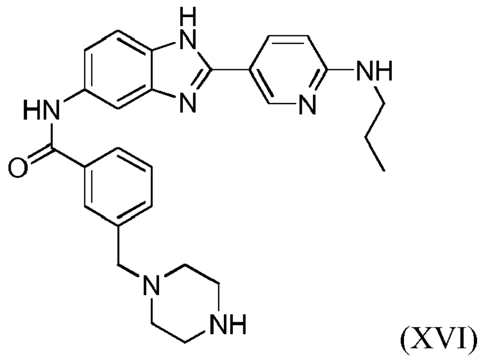

(XVI),

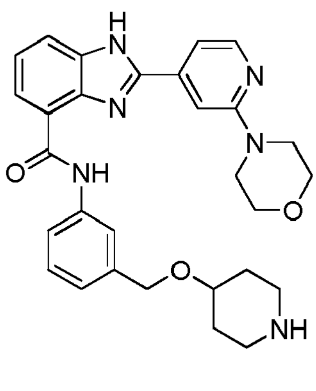

(XVII),

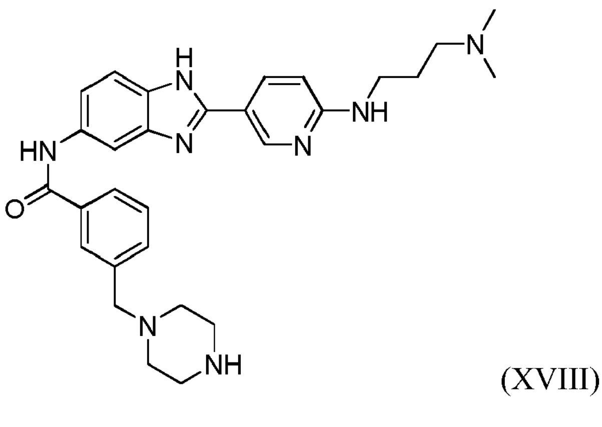

(XVIII),

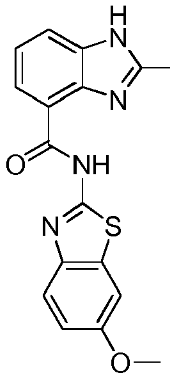

(XIX),

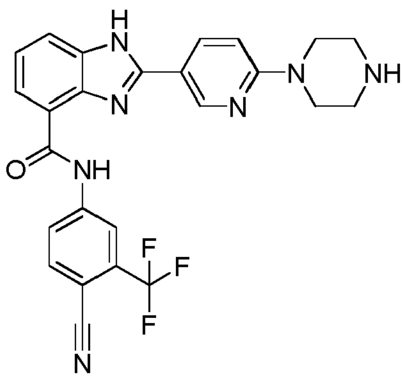

(XX),

and

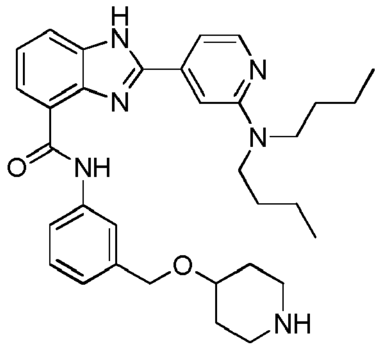

(XXI).

In further aspects, the invention provides pharmaceutical compositions comprising a compound of any one of formulas (I)-(XXI), or a pharmaceutically acceptable salt thereof; and a pharmaceutically acceptable carrier. In certain embodiments, the pharmaceutical composition further includes an additional active agent.

In yet another aspect, the invention provides a method for treating a subject suffering from or susceptible to insulin resistance, a metabolic syndrome, diabetes, or complications thereof, or for increasing insulin sensitivity in a subject, by administering a pharmaceutical composition comprising a compound of any one of formulas (I)-(XXI) to a subject in need thereof.

In still further aspects, the invention provides sirtuin-modulating compounds of structural formula (XXXI):

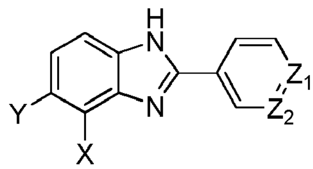

(XXXI),

or a salt thereof, wherein:

one of X and Y is selected from-NH-C(=O)-$R^1$ or -C(=O)-NH-$R^1$, and the other of X and Y is H, wherein $R^1$ is selected from phenyl or a fused hetereocycle, and $R^1$ is optionally substituted with one to two substituents independently selected from -C≡N, $C_1$-$C_2$ fluoro-substituted alkyl and -($C_0$-$C_2$ alkyl)-saturated hetereocycle, and when $R^1$ is phenyl, $R^1$ is optionally substituted with -$CH_2$-O-saturated hetereocycle, wherein the saturated hetereocycle is selected from piperidine, pyrrolidine or piperazine, and when $R^1$ is selected from a fused hetereocycle, the fused hetereocycle is selected from benzo[d]thiazole and $R^1$ is also optionally substituted with -$OCH_3$; and one of $Z^1$ and $Z^2$ is N, and the other is $CR^2$, wherein $R^2$ is selected from -NH($C_1$-$C_3$ alkyl), -NH($C_1$-$C_3$ alkyl)-N($CH_3$)$_2$, -($C_0$-$C_2$alkyl)-morpholine, -($C_0$-$C_2$ alkyl)-piperazine or -N($C_1$-$C_4$ alkyl)$_2$, wherein when $R^2$ is -($C_0$-$C_2$ alkyl)-saturated hetereocycle, the saturated heterocycle is selected from morpholine or piperazine.

In specific embodiments, the invention provides compounds, or salts thereof, of structural formula (XXXI), and

having the structural formulas (XIII)-(XXI):

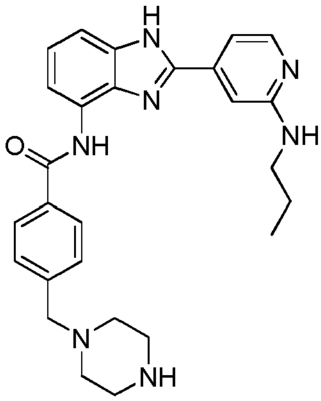 (XIII), 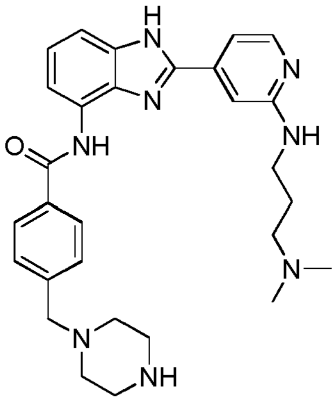 (XIV),

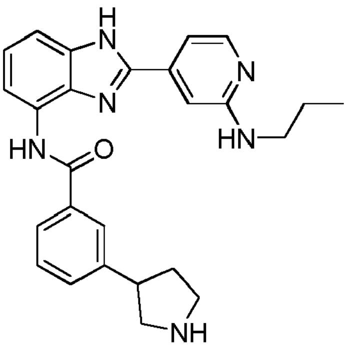 (XV), 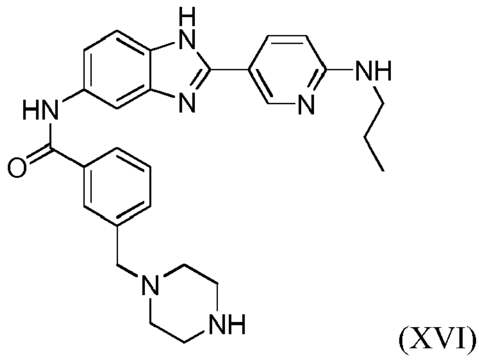 (XVI),

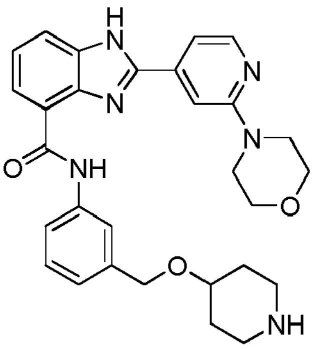 (XVII), 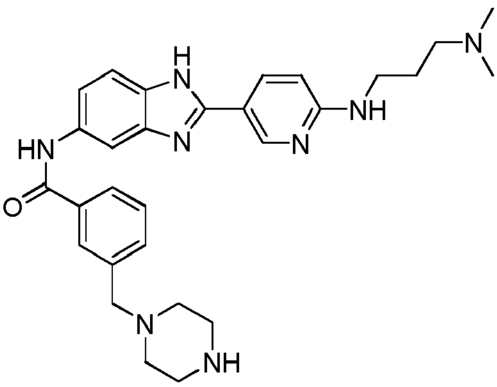 (XVIII),

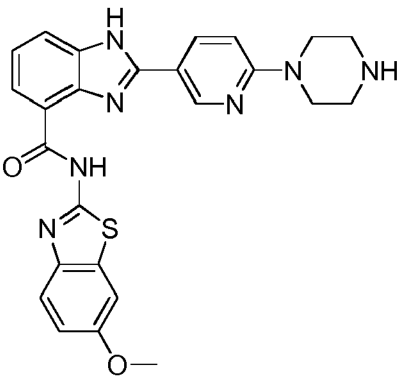 (XIX), 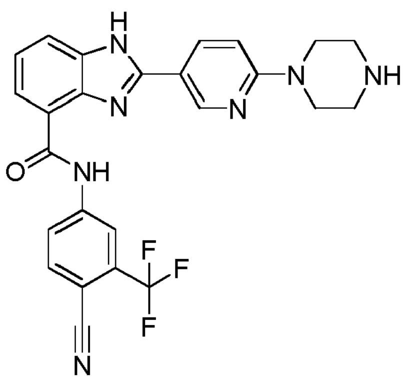 (XX),

and

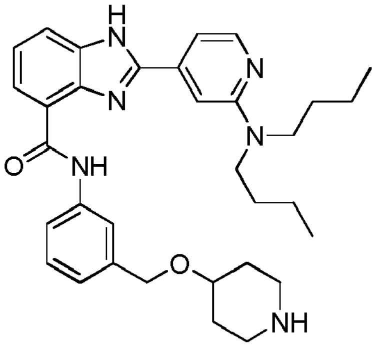

(XXI).

In particular aspects, the invention provides sirtuin-modulating compounds of structural formula (XXXII):

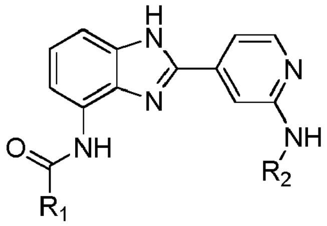

(XXXII),

or a salt thereof, wherein:

$R^1$ is phenyl optionally substituted with -($C_0$-$C_2$ alkyl)-pyrrolidine or -($C_0$-$C_2$ alkyl)-piperazine; and $R^2$ is selected from -$C_1$-$C_3$ alkyl or -($C_1$-$C_3$ alkyl)-$N(CH_3)_2$.

In specific embodiments, the invention provides compounds, or salts thereof, of structural formula (XXXII), having the structural formulas (XIII), (XIV), and (XV):

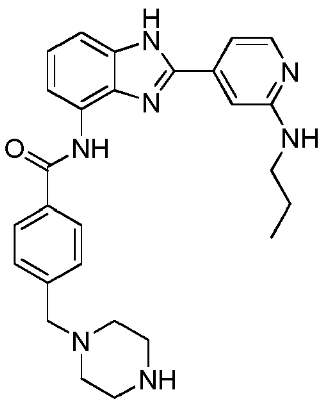

(XIII),

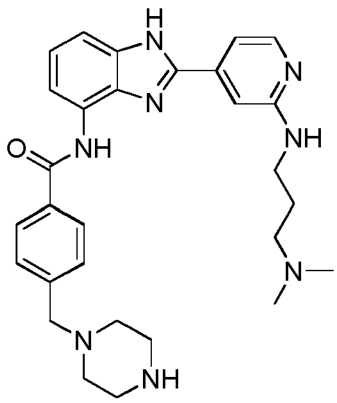

(XIV),

and

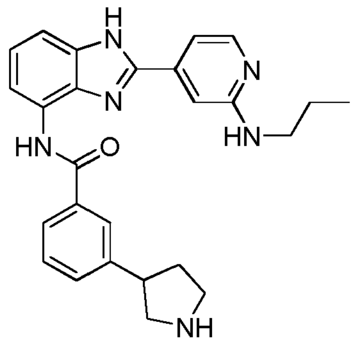

(XV).

In particular aspects, the invention provides sirtuin-modulating compounds of structural formula (XXXIII):

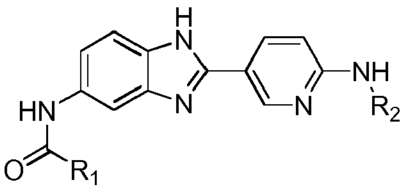

(XXXIII),

or a salt thereof, wherein:

$R^1$ is phenyl, optionally substituted with -($C_0$-$C_2$ alkyl)-piperazine; and $R^2$ is selected from -$C_1$-$C_3$ alkyl and -($C_1$-$C_3$)-$N(CH_3)_2$.

In specific embodiments, the invention provides compounds, or salts thereof, of structural formula (XXXIII), having the structural formulas (XVI) and (XVIII):

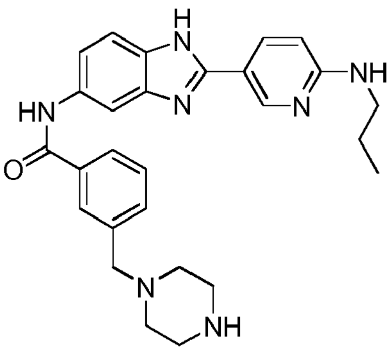

(XVI), and

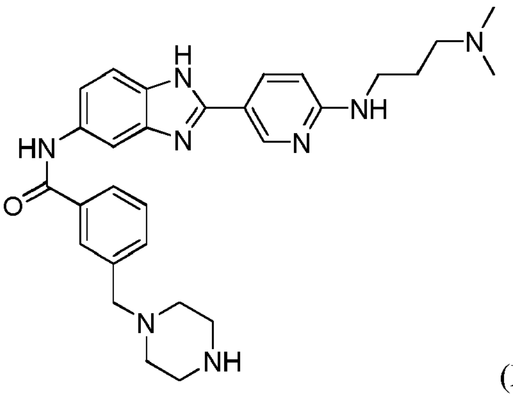

(XVIII).

In particular aspects the invention provides sirtuin-modulating compounds of structural formula (XXXIV):

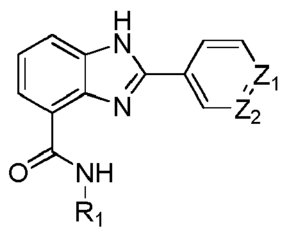

(XXXIV),

or a salt thereof, wherein: one of $Z^1$ and $Z^2$ is N, and the other is selected from $CR^2$, wherein $R^2$ is selected from -($C_0$-$C_2$ alkyl)-morpholine, -($C_0$-$C_2$ alkyl)-piperazine, and -$N(C_1$-$C_4)_2$; and $R^1$ is selected from phenyl and benzo[d]thiazole op-

tionally substituted with $-OCH_3$, wherein $R^1$ is further optionally substituted with one to two substituents independently selected from -C≡N, $C_1$-$C_2$ fluoro-substituted alkyl, and when $R^1$ is phenyl, $R^1$ is further optionally substituted with $-CH_2$-O-piperidine.

In specific embodiments, the invention provides compounds, or salts thereof, of structural formula (XXXIV), having the structural formulas (XVII), (XIX), (XX), and (XXI) :

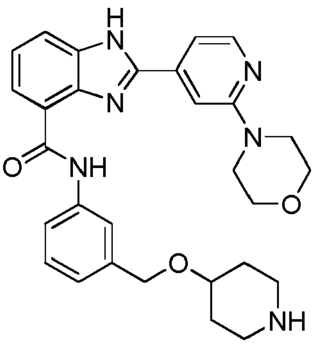 (XVII),

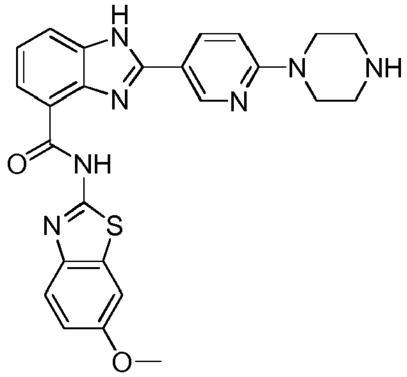 (XIX),

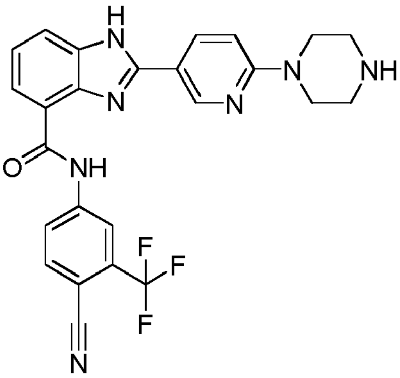 (XX), and

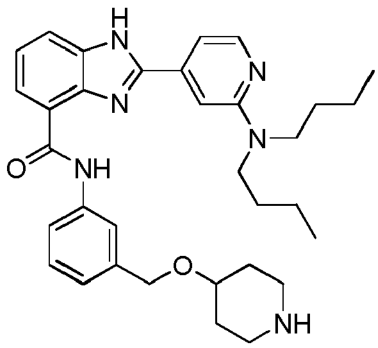 (XXI).

The invention further provides pharmaceutical compositions containing the sirtuin-modulating compounds of structural formulas (XXXI), (XXXII), (XXXIII), and (XXXIV), including compounds of structural formulas (XIII), (XIV), (XV), (XVI), (XVII), (XVIII), (XIX), (XX), and (XI).

In further aspects, the invention provides pharmaceutical compositions comprising a compound of any one of formulas (XXXI), (XXXII), (XXXIII), and (XXXIV), including compounds of structural formulas (XIII), (XIV), (XV), (XVI), (XVII), (XVIII), (XIX), (XX), and (XI), or a pharmaceutically acceptable salt thereof; and a pharmaceutically acceptable carrier. In certain embodiments, the pharmaceutical composition further includes an additional active agent.

In yet another aspect, the invention provides a method for treating a subject suffering from or susceptible to insulin resistance, a metabolic syndrome, diabetes, or complications thereof, or for increasing insulin sensitivity in a subject, by administering a pharmaceutical composition comprising a compound of any one of formulas (XXXI), (XXXII), (XXXIII), and (XXXIV), including compounds of structural formulas (XIII), (XIV), (XV), (XVI), (XVII), (XVIII), (XIX), (XX), and (XXI), to a subject in need thereof.

In another aspect, the invention provides methods for using sirtuin modulating compounds, or compositions comprising sirtuin-modulating compounds. In certain embodiments, sirtuin-modulating compounds that increase the level and/or activity of a sirtuin protein may be used for a variety of therapeutic applications including, for example, increasing the lifespan of a cell, and treating and/or preventing a wide variety of diseases and disorders including, for example, diseases or disorders related to aging or stress, diabetes, obesity, neurodegenerative diseases, chemotherapeutic induced neuropathy, neuropathy associated with an ischemic event, ocular diseases and/or disorders, cardiovascular disease, blood clotting disorders, inflammation, and/or flushing, etc. Sirtuin-modulating compounds that increase the level and/or activity of a sirtuin protein may also be used for treating a disease or disorder in a subject that would benefit from increased mitochondrial activity, for enhancing muscle performance, for increasing muscle ATP levels, or for treating or preventing muscle tissue damage associated with hypoxia or ischemia. In other embodiments, sirtuin-modulating compounds that decrease the level and/or activity of a sirtuin protein may be used for a variety of therapeutic applications including, for example, increasing cellular sensitivity to stress, increasing apoptosis, treatment of cancer,

stimulation of appetite, and/or stimulation of weight gain, etc. As described further below, the methods comprise administering to a subject in need thereof a pharmaceutically effective amount of a sirtuin-modulating compound. In certain aspects, the sirtuin-modulating compounds may be administered alone or in combination with other compounds, including other sirtuin-modulating compounds, or other therapeutic agents. In certain preferred embodiments, the invention provides methods for treating a subject suffering from or susceptible to insulin resistance, a metabolic syndrome, diabetes, or complications thereof, or for increasing insulin sensitivity in a subject, by administering to the subject a sirtuin-modulating compounds of Structural Formulas (XL), (XXXI), (XXXII), (XXXIII), and (XXXIV), including a compound of any one of formulas (I)-(XXI), or salts thereof.

## BRIEF DESCRIPTION OF THE FIGURES

FIGURE 1 shows the chemical structures of SRT1460, SRT1720, and SRT2183.

FIGURE 2A shows an "indirect" mechanism of enzyme activation for SIRT1 enzyme (E) on an acetylated substrate (S) in which activator X binds to substrate S, and activation results from the favorable kinetics of X:S turnover.

FIGURE 2B shows an allosteric mechanism of enzyme activation for SIRT1 enzyme (E) on an acetylated substrate (S) in which binding of substrate at the active site and activator at an allosteric exosite leads to the formation of (X:E:S)', and activation results when the $\gamma/\beta < 1$.

FIGURE 3 shows simulations of the dependence of relative initial velocity on activator concentration at the indicated values of $K_d$ assuming the 'indirect' activation mechanism of Figure 2A. Simulations were performed using eq (5) and the expression for free substrate concentration of eq (8), and the parameter assignments given in the text with $[S]_o$ set at 0.5 $\mu$M. The inset show the linear dependence of $K_{x,ap}$ on $K_d$ for the mechanism of Figure 2A.

FIGURE 4A shows a calorimetric study of the interaction between a SIRT1 activator (structure 4 in Figure 5) and TAMRA-peptide demonstrating lack of binding of the activator to TAMRA-peptide.

FIGURE 4B shows a calorimetric study of the interaction between a SIRT1 activator (structure 7 in Figure 5) and TAMRA-peptide demonstrating that the activator binds to TAMRA-peptide with a $K_d$ of 36 $\mu$M. Inset shows the activation titration curve, of relative velocity vs. $[3]_o$ from which curve an $EC_{50}$ of 0.5 $\mu$M is calculated ($EC_{1.5}$ = 0.1 $\mu$M).

FIGURE 5 shows the lack of dependence of $K_d$ for the binding of activator to TAMRA-peptide, with $EC_{1.5}$, for activation of SIRT1. The structures of specific compounds discussed in the text are shown. Open circles are for compounds with $K_d \geq 100$ $\mu$M. The gray bar highlights a series compounds with $K_d$ values that range from 2.5 $\mu$M to greater than 100 $\mu$M, but have the same $EC_{1.5}$ value of around 0.3 $\mu$M.

FIGURE 6 shows the dependence of relative velocity of an activator (structure 22 in Figure 5) for the SIRT1-catalyzed deacetylation of TAMRA-peptide. The two symbols correspond to two independent experiments, and the solid line was drawn using the best-fit parameters according to eq (1), $EC_{50} = 3.9 \pm 0.5$ $\mu$M, $RV_{max} = 6.8 \pm 0.3$.

FIGURE 7A shows the dependence of relative velocity on [SRT1460]o, at a TAMRA-peptide substrate concentration of 0.5 $\mu$M, using the substrate using data shown from Milne, et al. ((2007) Nature 450, 712-716). The solid line through the data was drawn using eqs (5) and (8) and best fit parameters $K_{m,x}$= 90 $\pm$ 8 nM, $\gamma$ = 0.20 + 0.01. In this fit, the following constraints were used: $K_d$ = 140 $\mu$M , $[S]_o$ = 0.05 $\mu$M, and $K_m$ = 14.5 $\mu$M.

FIGURE 7B shows each data set fit to Michaelis-Menten equation using the best fit parameters (Table 5) to draw the solid lines (Symbols: A, 37 $\mu$M; $\Delta$, 1 $\mu$M; ▼, 3 $\mu$M; O, 0 $\mu$M).

FIGURE 8A shows the binding of compound 11 (see Table 6) to SIRT1. ITC demonstrates that compound 11 binds to SIRT(183-664) with a $K_d$ of 0.32 $\mu$M.

FIGURE 8B shows the fluorescence of compound 11 (see Table 6) increases with increasing concentration of SIRT(183-664).

FIGURE 8C shows that compound 11 (see Table 6) binds to SIRT(183-664) with a $K_d$ of 0.27 $\mu$M using data from

FIGURE 8B to construct a titration curve.

FIGURE 9 shows the modulation of the catalytic activity of SIRT1 by activator compounds 22, 23, and 24 (structures indicated below). Closed symbols correspond to activation of the deacetylation of desTAMRA-peptide. Data points were fit to eq (1), and yielded: compound 22 $EC_{50}$ = 1.7 $\pm$ 0.1 $\mu$M, $RV_{max}$ = 2.6 + 0.1; compound 23 $EC_{50}$ = 2.2 $\pm$ 0.3 $\mu$M, $RV_{max}$ = 2.8 $\pm$ 0.2; 24 $EC_{50}$ = 1.5 $\pm$ 0.1 $\mu$M, $RV_{max}$ = 2.1 + 0.1. Open symbols correspond to inhibition of the deacetylation p53-20mer. Data points were fit to the equation: $RV = 1/(1+([X]_o/K_{i,app}))$, and yielded: compound 22 $K_{i,app}$ = 9 $\pm$ 4 V, 23 $K_{i,app}$ = 10 $\pm$ 1.0 $\mu$M, 24 $K_{i,app}$ = 9 $\pm$ 3.0 $\mu$M.

FIGURE 10 shows that compound 10 (FIGURE 9) and SRT1460 (FIGURE 1) activate the SIRT1-catalyzed deacetylation of Ac-Arg-His-Lys-$Lys^{Ac}$-X. Top Panel: X = $TrpNH_2$, $EC_{1.5}$ = 1.9 $\mu$M; X = AMC, $EC_{1.5}$ = 3.1 $\mu$M. Bottom Panel: X = $TrpNH_2$, $EC_{1.5}$ = 5.0 $\mu$M, $EC_{50}$ = 27 $\pm$ 8.0 $\mu$M, and $RV_{max}$ = 4.1 $\pm$ 0.1; X = $PheNH_2$, $EC_{1.5}$ = 26.0 $\mu$M. These titrations represent 3-5 independent experiments, and each point is the mean $\pm$ std. dev. from these experiments.

FIGURE 11 shows the structures and $EC_{1.5}$ values for two SIRT1 activators that differ only in the orientation of the core heterocycle.

FIGURE 12 is a model of the binding of Ac-Arg-His-Lys-$Lys^{Ac}$-Trp-$NH_2$ to the active site of SIRT1.

FIGURE 13 is a depiction of a SIRT3-based structural homology model of Ac-Arg-His-Lys-$Lys^{Ac}$-Trp-$NH_2$ docked into the active site of SIRT1. The indole of the Trp residue is loosely sandwiched between $Phe^{414}$ and $Arg^{446}$ of a SIRT1 homology model, forming a sort of molecular 'tweezers' for binding bulky groups at this position.

FIGURE 14 is a depiction of in vitro and in vivo models for SIRT1 activation mechanisms involving an activation cofactor requirement.

## DETAILED DESCRIPTION

### 1. Definitions

As used herein, the following terms and phrases shall have the meanings set forth below. Unless defined otherwise, all technical and scientific terms used herein have the same meaning as commonly understood to one of ordinary skill in the art.

The term "agent" is used herein to denote a chemical compound, a mixture of chemical compounds, a biological macromolecule (such as a nucleic acid, an antibody, a protein or portion thereof, e.g., a peptide), or an extract made from biological materials such as bacteria, plants, fungi, or animal (particularly mammalian) cells or tissues. The activity of such agents may render it suitable as a "therapeutic agent" which is a biologically, physiologically, or pharmacologically active substance (or substances) that acts locally or systemically in a subject.

The term "bioavailable," when referring to a compound is art-recognized and refers to a form of a compound that allows for it, or a portion of the amount of compound administered, to be absorbed by, incorporated to, or otherwise physiologically available to a subject or patient to whom it is administered.

"Biologically active portion of a sirtuin" refers to a portion of a sirtuin protein having a biological activity, such as the ability to deacetylate. Biologically active portions of a sirtuin may comprise the core domain of sirtuins. Biologically active portions of SIRT1 having GenBank Accession No. NP_036370 that encompass the $NAD^+$ binding domain and the substrate binding domain, for example, may include without limitation, amino acids 62-293 of GenBank Accession No. NP_036370, which are encoded by nucleotides 237 to 932 of GenBank Accession No. NM_012238. Therefore, this region is sometimes referred to as the core domain. Other biologically active portions of SIRT1, also sometimes referred to as core domains, include about amino acids 261 to 447 of GenBank Accession No. NP_036370, which are encoded by nucleotides 834 to 1394 of GenBank Accession No. NM_012238; about amino acids 242 to 493 of GenBank Accession No. NP_036370, which are encoded by nucleotides 777 to 1532 of GenBank Accession No. NM_012238; or about amino acids 254 to 495 of GenBank Accession No. NP_036370, which are encoded by nucleotides 813 to 1538 of GenBank Accession No. NM_012238.

The term "companion animals" refers to cats and dogs. As used herein, the term "dog(s)" denotes any member of the species Canis familiaris, of which there are a large number of different breeds. The term "cat(s)" refers to a feline animal including domestic cats and other members of the family Felidae, genus Felis.

"Diabetes" refers to high blood sugar or ketoacidosis, as well as chronic, general metabolic abnormalities arising from a prolonged high blood sugar status or a decrease in glucose tolerance. "Diabetes" encompasses both the type I and type II (Non Insulin Dependent Diabetes Mellitus or NIDDM) forms of the disease. The risk factors for diabetes

include the following factors: waistline of more than 40 inches for men or 35 inches for women, blood pressure of 130/85 mmHg or higher, triglycerides above 150 mg/dl, fasting blood glucose greater than 100 mg/dl or high-density lipoprotein of less than 40 mg/dl in men or 50 mg/dl in women.

The term "$ED_{50}$" is art-recognized. In certain embodiments, $ED_{50}$ means the dose of a drug which produces 50% of its maximum response or effect, or alternatively, the dose which produces a pre-determined response in 50% of test subjects or preparations. The term "$LD_{50}$" is art-recognized. In certain embodiments, $LD_{50}$ means the dose of a drug which is lethal in 50% of test subjects. The term "therapeutic index" is an art-recognized term which refers to the therapeutic index of a drug, defined as $LD_{50}/ED_{50}$.

The term "fluorescent-free activation substrate" shall mean an acetylated substrate of a sirtuin deacetylase (e.g., SIRT1), that does not include an art-recognized fluorescent labeling group such as TAMRA or AMC, but which may include natural amino acid residues, such as phenylalanine and tryptophan, which have fluorescent properties, and that further allows fluorescent-free activation of a sirtuin such as SIRT1 by a sirtuin activator compound (STAC). Such fluorescent-free activation substrates may include acetylated peptide, polypeptide, and protein substrates, including full-length SIRT1 protein substrates such as histones H1, H3, and H4, p53, p300, FOXOs 1, 3a, and 4, p65, HIVTat, PGC-1$\alpha$, PCAF, MyoD, PPAR$\gamma$, and Ku70, and peptide and polypeptide fragments thereof having such STAC-activation properties. Such fluorescent-free activation substrates may further include peptide, polypeptide and protein acetylated substrates having a non-fluorescent bulky group such as biotin that also allows for STAC activation of sirtuins. Such fluorescent-free activation substrates still further include acetylated peptide, polypeptide, and protein sirtuin substrates that may not otherwise be susceptible to STAC activation, in combination with an activation cofactor-bearing accessory protein or ligand that confers STAC activation properties on such substrates, the combination of substrate and cofactor thus providing the functional "fluorescent-free activation substrate." Suitable such cofactors include DBC1, HIC1, AROS, and CLOCK proteins, which can provide STAC-activatable properties on a sirtuin substrate that is not otherwise activated by a STAC.

The term "hyperinsulinemia" refers to a state in an individual in which the level of insulin in the blood is higher than normal.

The term "insulin resistance" refers to a state in which a normal amount of insulin produces a subnormal biologic response relative to the biological response in a subject that does not have insulin resistance.

An "insulin resistance disorder," as discussed herein, refers to any disease or condition that is caused by or contributed to by insulin resistance. Examples include: diabetes, obesity, metabolic syndrome, insulin-resistance syndromes, syndrome X, insulin resistance, high blood pressure, hypertension, high blood cholesterol, dyslipidemia, hyperlipidemia, dyslipidemia, atherosclerotic disease including stroke, coronary artery disease or myocardial infarction, hyperglycemia, hyperinsulinemia and/or hyperproinsulinemia, impaired glucose tolerance, delayed insulin release, diabetic complications, including coronary heart disease, angina pectoris, congestive heart failure, stroke, cognitive functions in dementia, retinopathy, peripheral neuropathy, nephropathy, glomerulonephritis, glomerulosclerosis, nephrotic syndrome, hypertensive nephrosclerosis some types of cancer (such as endometrial, breast, prostate, and colon), complications of pregnancy, poor female reproductive health (such as menstrual irregularities, infertility, irregular ovulation, polycystic ovarian syndrome (PCOS)), lipodystrophy, cholesterol related disorders, such as gallstones, cholescystitis and cholelithiasis, gout, obstructive sleep apnea and respiratory problems, osteoarthritis, and prevention and treatment of bone loss, e.g. osteoporosis.

The term "livestock animals" refers to domesticated quadrupeds, which includes those being raised for meat and various byproducts, e.g., a bovine animal including cattle and other members of the genus Bos, a porcine animal including domestic swine and other members of the genus Sus, an ovine animal including sheep and other members of the genus Ovis, domestic goats and other members of the genus Capra; domesticated quadrupeds being raised for specialized tasks such as use as a beast of burden, e.g., an equine animal including domestic horses and other members of the family Equidae, genus Equus.

The term "mammal" is known in the art, and exemplary mammals include humans, primates, livestock animals (including bovines, porcines, etc.), companion animals (e.g., canines, felines, etc.) and rodents (e.g., mice and rats).

"Obese" individuals or individuals suffering from obesity are generally individuals having a body mass index (BMI) of at least 25 or greater. Obesity may or may not be associated with insulin resistance.

The terms "parenteral administration" and "administered parenterally" are art-recognized and refer to modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intra-articulare, subcapsular, subarachnoid, intraspinal, and intrasternal injection and infusion.

A "patient", "subject", "individual" or "host" refers to either a human or a non-human animal.

The term "pharmaceutically acceptable carrier" is art-recognized and refers to a pharmaceutically-acceptable material, composition or vehicle, such as a liquid or solid filler, diluent, excipient, solvent or encapsulating material, involved in carrying or transporting any subject composition or component thereof. Each carrier must be "acceptable"

in the sense of being compatible with the subject composition and its components and not injurious to the patient. For example, a pharmaceutically acceptable carrier may have an $LD_{50}$ of at least 1 g/kg, 3 g/kg or even 5 g/kg. Alternatively, a pharmaceutically acceptable carrier may be a substance that is deemed by a national regulatory agency (e.g., the U.S. Food and Drug Administration) to be generally recognized as safe (a GRAS substance). Some examples of materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The term "prophylactic" or "therapeutic" treatment is art-recognized and refers to administration of a drug to a host. If it is administered prior to clinical manifestation of the unwanted condition (e.g., disease or other unwanted state of the host animal) then the treatment is prophylactic, i.e., it protects the host against developing the unwanted condition, whereas if administered after manifestation of the unwanted condition, the treatment is therapeutic (i.e., it is intended to diminish, ameliorate or maintain the existing unwanted condition or side effects therefrom).

The term "pyrogen-free", with reference to a composition, refers to a composition that does not contain a pyrogen in an amount that would lead to an adverse effect (e.g., irritation, fever, inflammation, diarrhea, respiratory distress, endotoxic shock, etc.) in a subject to which the composition has been administered. For example, the term is meant to encompass compositions that are free of, or substantially free of, an endotoxin such as, for example, a lipopolysaccharide (LPS).

"Replicative lifespan" of a cell refers to the number of daughter cells produced by an individual "mother cell." "Chronological aging" or "chronological lifespan," on the other hand, refers to the length of time a population of non-dividing cells remains viable when deprived of nutrients. "Increasing the lifespan of a cell" or "extending the lifespan of a cell," as applied to cells or organisms, refers to increasing the number of daughter cells produced by one cell; increasing the ability of cells or organisms to cope with stresses and combat damage, e.g., to DNA, proteins; and/or increasing the ability of cells or organisms to survive and exist in a living state for longer under a particular condition, e.g., stress (for example, heatshock, osmotic stress, high energy radiation, chemically-induced stress, DNA damage, inadequate salt level, inadequate nitrogen level, or inadequate nutrient level). Lifespan can be increased by at least about 20%, 30%, 40%, 50%, 60% or between 20% and 70%, 30% and 60%, 40% and 60% or more using methods described herein.

"Sirtuin-activating compound" refers to a compound that increases the level of a sirtuin protein and/or increases at least one activity of a sirtuin protein. In an exemplary embodiment, a sirtuin-activating compound may increase at least one biological activity of a sirtuin protein by at least about 10%, 25%, 50%, 75%, 100%, or more. Exemplary biological activities of sirtuin proteins include deacetylation, e.g., of histones and p53; extending lifespan; increasing genomic stability; silencing transcription; and controlling the segregation of oxidized proteins between mother and daughter cells.

"Sirtuin protein" refers to a member of the sirtuin deacetylase protein family, or preferably to the sir2 family, which include yeast Sir2 (GenBank Accession No. P53685), *C. elegans* Sir-2.1 (GenBank Accession No. NP_501912), and human SIRT1 (GenBank Accession No. NM_012238 and NP_036370 (or AF083106)) and SIRT2 (GenBank Accession No. NM_012237, NM_030593, NP_036369, NP_085096, and AF083107) proteins. Other family members include the four additional yeast Sir2-like genes termed *"HST* genes" (homologues of Sir two) HST1, HST2, HST3 and HST4, and the five other human homologues hSIRT3, hSIRT4, hSIRT5, hSIRT6 and hSIRT7 (Brachmann et al. (1995) Genes Dev. 9:2888 and Frye et al. (1999) BBRC 260:273). Preferred sirtuins are those that share more similarities with SIRT1, i.e., hSIRT1, and/or Sir2 than with SIRT2, such as those members having at least part of the N-terminal sequence present in SIRT1 and absent in SIRT2 such as SIRT3 has.

"SIRT1 protein" refers to a member of the sir2 family of sirtuin deacetylases. In one embodiment, a SIRT1 protein includes yeast Sir2 (GenBank Accession No. P53685), C. elegans Sir-2.1 (GenBank Accession No. NP_501912), human SIRT1 (GenBank Accession No. NM_012238 or NP_036370 (or AF083106)), and human SIRT2 (GenBank Accession No. NM_012237, NM_030593, NP_036369, NP_085096, or AF083107) proteins, and equivalents and fragments thereof. In another embodiment, a SIRT1 protein includes a polypeptide comprising a sequence consisting of, or consisting essentially of, the amino acid sequence set forth in GenBank Accession Nos. NP_036370, NP_501912, NP_085096, NP_036369, or P53685. SIRT1 proteins include polypeptides comprising all or a portion of the amino acid sequence set forth in GenBank Accession Nos. NP_036370, NP_501912, NP_085096, NP_036369, or P53685; the amino acid sequence set forth in GenBank Accession Nos. NP_036370, NP_501912, NP_085096, NP_036369, or P53685 with 1 to about 2, 3, 5, 7, 10, 15, 20, 30, 50, 75 or more conservative amino acid substitutions; an amino acid sequence that is at least 60%, 70%, 80%, 90%, 95%, 96%, 97%, 98%, or 99% identical to GenBank Accession Nos.

NP_036370, NP_501912, NP_085096, NP_036369, or P53685, and functional fragments thereof. Polypeptides of the invention also include homologs (e.g., orthologs and paralogs), variants, or fragments, of GenBank Accession Nos. NP_036370, NP_501912, NP_085096, NP_036369, or P53685.

"SIRT3 protein" refers to a member of the sirtuin deacetylase protein family and/or to a homolog of a SIRT1 protein. In one embodiment, a SIRT3 protein includes human SIRT3 (GenBank Accession No. AAH01042, NP_036371, or NP_001017524) and mouse SIRT3 (GenBank Accession No. NP_071878) proteins, and equivalents and fragments thereof. In another embodiment, a SIRT3 protein includes a polypeptide comprising a sequence consisting of, or consisting essentially of, the amino acid sequence set forth in GenBank Accession Nos. AAH01042, NP_036371, NP_001017524, or NP_071878. SIRT3 proteins include polypeptides comprising all or a portion of the amino acid sequence set forth in GenBank Accession AAH01042, NP_036371, NP_001017524, or NP_071878; the amino acid sequence set forth in GenBank Accession Nos. AAH01042, NP_036371, NP_001017524, or NP_071878 with 1 to about 2, 3, 5, 7, 10, 15, 20, 30, 50, 75 or more conservative amino acid substitutions; an amino acid sequence that is at least 60%, 70%, 80%, 90%, 95%, 96%, 97%, 98%, or 99% identical to GenBank Accession Nos. AAH01042, NP_036371, NP_001017524, or NP_071878, and functional fragments thereof. Polypeptides of the invention also include homo logs (e.g., orthologs and paralogs), variants, or fragments, of GenBank Accession Nos. AAH01042, NP_036371, NP_001017524, or NP_071878. In one embodiment, a SIRT3 protein includes a fragment of SIRT3 protein that is produced by cleavage with a mitochondrial matrix processing peptidase (MPP) and/or a mitochondrial intermediate peptidase (MIP).

The terms "systemic administration," "administered systemically," "peripheral administration" and "administered peripherally" are art-recognized and refer to the administration of a subject composition, therapeutic or other material other than directly into the central nervous system, such that it enters the patient's system and, thus, is subject to metabolism and other like processes.

The term "therapeutic agent" is art-recognized and refers to any chemical moiety that is a biologically, physiologically, or pharmacologically active substance that acts locally or systemically in a subject. The term also means any substance intended for use in the diagnosis, cure, mitigation, treatment or prevention of disease or in the enhancement of desirable physical or mental development and/or conditions in an animal or human.

The term "therapeutic effect" is art-recognized and refers to a local or systemic effect in animals, particularly mammals, and more particularly humans caused by a pharmacologically active substance. The phrase "therapeutically-effective amount" means that amount of such a substance that produces some desired local or systemic effect at a reasonable benefit/risk ratio applicable to any treatment. The therapeutically effective amount of such substance will vary depending upon the subject and disease condition being treated, the weight and age of the subject, the severity of the disease condition, the manner of administration and the like, which can readily be determined by one of ordinary skill in the art. For example, certain compositions described herein may be administered in a sufficient amount to produce a desired effect at a reasonable benefit/risk ratio applicable to such treatment.

"Treating" a condition or disease refers to curing as well as ameliorating at least one symptom of the condition or disease.

The term "vision impairment" refers to diminished vision, which is often only partially reversible or irreversible upon treatment (e.g., surgery). Particularly severe vision impairment is termed "blindness" or "vision loss", which refers to a complete loss of vision, vision worse than 20/200 that cannot be improved with corrective lenses, or a visual field of less than 20 degrees diameter (10 degrees radius).

## 2. Sirtuin Modulators

In one aspect, the invention provides novel sirtuin-modulating compounds for treating and/or preventing a wide variety of diseases and disorders including, for example, diseases or disorders related to aging or stress, diabetes, obesity, neurodegenerative diseases, ocular diseases and disorders, cardiovascular disease, blood clotting disorders, inflammation, cancer, and/or flushing, etc. Sirtuin-modulating compounds that increase the level and/or activity of a sirtuin protein may also be used for treating a disease or disorder in a subject that would benefit from increased mitochondrial activity, for enhancing muscle performance, for increasing muscle ATP levels, or for treating or preventing muscle tissue damage associated with hypoxia or ischemia. Other compounds disclosed herein may be suitable for use in a pharmaceutical composition and/or one or more methods disclosed herein.

In certain embodiments, sirtuin-modulating compounds of the invention are represented by Structural (XL), (XXXI), (XXXII), (XXXIII), and (XXXIV), including a compound of any of structural formulas (I), (II), (III), (IV), (V), (VI), (VII), (VIII), (IX), (X), (XI), (XII) (XIII), (XIV), (XV), (XVI), (XVII), (XVIII), (XIX), (XX), and (XXI).

The embodiments described below apply to compounds of any of Structural Formulas: (XL), (XXXI), (XXXII), (XXXIII), and (XXXIV), including a compound of any of structural formulas (I), (II), (III), (IV), (V), (VI), (VII), (VIII), (IX), (X), (XI), (XII) (XIII), (XIV), (XV), (XVI), (XVII), (XVIII), (XIX), (XX), and (XXI).

Compounds of the invention, including novel compounds of the invention, can also be used in the methods described herein.

The compounds and salts thereof described herein also include their corresponding hydrates (e.g., hemihydrate, monohydrate, dihydrate, trihydrate, tetrahydrate) and solvates. Suitable solvents for preparation of solvates and hydrates can generally be selected by a skilled artisan.

The compounds and salts thereof can be present in amorphous or crystalline (including co-crystalline and polymorph) forms.

Sirtuin-modulating compounds of the invention advantageously modulate the level and/or activity of a sirtuin protein, particularly the deacetylase activity of the sirtuin protein.

Separately or in addition to the above properties, certain sirtuin-modulating compounds of the invention do not substantially have one or more of the following activities: inhibition of PI3-kinase, inhibition of aldoreductase, inhibition of tyrosine kinase, transactivation of EGFR tyrosine kinase, coronary dilation, or spasmolytic activity, at concentrations of the compound that are effective for modulating the deacetylation activity of a sirtuin protein (e.g., such as a SIRT1 and/or a SIRT3 protein).

An alkyl group is a straight chained or branched hydrocarbon which is completely saturated. Typically, a straight chained or branched alkyl group has from 1 to about 20 carbon atoms, preferably from 1 to about 10. Examples of straight chained and branched alkyl groups include methyl, ethyl, n-propyl, iso-propyl, n-butyl, sec-butyl, tert-butyl, pentyl, hexyl, pentyl and octyl. A $C_1$-$C_4$ straight chained or branched alkyl group is also referred to as a "lower alkyl" group.

A cycloalkyl group is a cyclic hydrocarbon which is completely saturated.

Carbocyclic includes 5-7 membered monocyclic and 8-12 membered bicyclic rings wherein the monocyclic or bicyclic rings are selected from saturated, unsaturated and aromatic. In certain embodiments, where the carbocyclic is a bicyclic ring system, each ring may be characterized by a different degree of saturation. For example, an aromatic ring, e.g., phenyl, may be fused to a saturated or unsaturated ring, e.g., cyclohexane, cyclopentane, or cyclohexene. Any combination of saturated, unsaturated and aromatic bicyclic rings, as valence permits, are included in the definition of carbocyclic. Exemplary carbocycles include cyclopentyl, cyclohexyl, cyclohexenyl, adamantyl, phenyl and naphthyl.

Heterocyclic includes 4-8 membered monocyclic and 8-12 membered bicyclic rings comprising one or more heteroatoms selected from, for example, N, O, and S atoms. In certain embodiments, the heterocyclic group is selected from saturated, unsaturated or aromatic. In certain embodiments, in a bicyclic ring system, each ring may be characterised by a different degree of saturation. For example, an aromatic ring, e.g., phenyl or pyridine, may be fused to a saturated ring, e.g., morpholinyl, or tetrahydrofuran. Any combination of saturated, unsaturated and aromatic bicyclic rings, as valence permits, are included in the definition of heterocyclic. In a saturated heterocycle, atoms of the heterocycle are bound to one another by single bonds.

Monocyclic rings include 5-7 membered aryl or 4-8 membered heteroaryl, 5-7 membered cycloalkyl, and 4-8 membered non-aromatic heterocyclyl. Exemplary monocyclic groups include substituted or unsubstituted heterocycles such as thiazolyl, oxazolyl, oxazinyl, thiazinyl, dithianyl, dioxanyl, isoxazolyl, isothiozolyl, triazolyl, furanyl, tetrahydrofuranyl, dihydrofuranyl, pyranyl, tetrazolyl, pyrazolyl, pyrazinyl, pyridazinyl, imidazolyl, pyridinyl, pyrrolyl, dihydropyrrolyl, pyrrolidinyl, thiazinyl, oxazinyl, piperidinyl, piperazinyl, pyrimidinyl, morpholinyl, tetrahydrothiophenyl, thiophenyl, cyclohexyl, cyclopentyl, cyclopropyl, cyclobutyl, cycloheptanyl, azetidinyl, oxetanyl, thiiranyl, oxiranyl, aziridinyl, and thiomorpholinyl.

Aromatic (aryl) groups include carbocyclic aromatic groups such as phenyl, naphthyl, and anthracyl. Heteroaromatic (heteroaryl) groups include heteroaromatic groups such as imidazolyl, thienyl, furyl, pyridyl, pyrimidyl, pyranyl, pyrazolyl, pyrroyl, pyrazinyl, thiazolyl, oxazolyl, and tetrazolyl.

Aromatic groups also include fused polycyclic aromatic ring systems, in which a carbocyclic aromatic ring or heteroaryl ring is fused to one or more other aromatic or heteroaryl rings. Examples include benzothienyl, naphthyl, benzofuryl, indolyl, quinolinyl, benzothiazole, benzomorpholinyl, benzoxazole, benzimidazole, quinolinyl, isoquinolinyl and isoindolyl.

Fluoro-substituted includes from one fluoro substituent up to per-fluoro-substitution. Exemplary fluoro-substituted $C_1$-$C_2$ alkyl includes $-CFH_2$, $CF_2H$, $-CF_3$,$-CH_2CH_2F$, $-CH_2CHF_2$, $-CHFCH_3$, $-CF_2CHF_2$. Per-fluoro-substituted $C_1$-$C_2$ alkyl, for example, includes $CF_3$, and $-CF_2CF_3$.

Suitable substituents on moieties indicated as being substituted or unsubstituted are those which do not substantially interfere with the ability of the disclosed compounds to have one or more of the properties disclosed herein. A substituent substantially interferes with the properties of a compound when the magnitude of the property is reduced by more than about 50% in a compound with the substituent compared with a compound without the substituent.

Combinations of substituents and variables envisioned by this invention are only those that result in the formation of stable compounds. As used herein, the term "stable" refers to compounds that possess stability sufficient to allow manufacture and that maintain the integrity of the compound for a sufficient period of time to be useful for the purposes detailed herein.

The compounds disclosed herein also include partially and fully deuterated variants. In certain embodiments, one or more deuterium atoms are present for kinetic studies. One of ordinary skill in the art can select the sites at which such deuterium atoms are present.

Also included in the present invention are salts, particularly pharmaceutically acceptable salts, of the sirtuin-modulating compounds described herein. The compounds of the present invention that possess a sufficiently acidic, a sufficiently basic, or both functional groups, can react with any of a number of inorganic bases, and inorganic and organic acids, to form a salt. Alternatively, compounds that are inherently charged, such as those with a quaternary nitrogen, can form a salt with an appropriate counterion (e.g., a halide such as bromide, chloride, or fluoride, particularly bromide).

Acids commonly employed to form acid addition salts are inorganic acids such as hydrochloric acid, hydrobromic acid, hydroiodic acid, sulfuric acid, phosphoric acid, and the like, and organic acids such as p-toluenesulfonic acid, methanesulfonic acid, oxalic acid, p-bromophenyl-sulfonic acid, carbonic acid, succinic acid, citric acid, benzoic acid, acetic acid, and the like. Examples of such salts include the sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, gamma-hydroxybutyrate, glycolate, tartrate, methanesulfonate, propanesulfonate, naphthalene-1-sulfonate, naphthalene-2-sulfonate, mandelate, and the like.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, and the like.

According to another embodiment, the present invention provides methods of producing the above-defined sirtuin-modulating compounds. The compounds may be synthesized using conventional techniques. Advantageously, these compounds are conveniently synthesized from readily available starting materials.

Synthetic chemistry transformations and methodologies useful in synthesizing the sirtuin-modulating compounds described herein are known in the art and include, for example, those described in R. Larock, *Comprehensive Organic Transformations* (1989); T. W. Greene and P. G. M. Wuts, *Projective Groups in Organic Syntheses,* 2d. Ed. (1991); L. Fieser and M. Fieser, *Fieser and Fieser's Reagents for Organic Synthesis* (1994); and L. Paquette, ed., *Encyclopedia of Reagents for Organic Synthesis* (1995).

In an exemplary embodiment, a sirtuin-modulating compound may traverse the cytoplasmic membrane of a cell. For example, a compound may have a cell-permeability of at least about 20%, 50%, 75%, 80%, 90% or 95%.

Sirtuin-modulating compounds described herein may also have one or more of the following characteristics: the compound may be essentially non-toxic to a cell or subject; the sirtuin-modulating compound may be an organic molecule or a small molecule of 2000 amu or less, 1000 amu or less; a compound may have a half-life under normal atmospheric conditions of at least about 30 days, 60 days, 120 days, 6 months or 1 year; the compound may have a half-life in solution of at least about 30 days, 60 days, 120 days, 6 months or 1 year; a sirtuin-modulating compound may be more stable in solution than resveratrol by at least a factor of about 50%, 2 fold, 5 fold, 10 fold, 30 fold, 50 fold or 100 fold; a sirtuin-modulating compound may promote deacetylation of the DNA repair factor Ku70; a sirtuin-modulating compound may promote deacetylation of RelA/p65; a compound may increase general turnover rates and enhance the sensitivity of cells to TNF$\alpha$-induced apoptosis.

In certain embodiments, a sirtuin-modulating compound does not have any substantial ability to inhibit a histone deacetylase (HDACs) class I, a HDAC class II, or HDACs I and II, at concentrations (e.g., in vivo) effective for modulating the deacetylase activity of the sirtuin. For instance, in preferred embodiments the sirtuin-modulating compound is a sirtuin-activating compound and is chosen to have an $EC_{50}$ for activating sirtuin deacetylase activity that is at least 5 fold less than the $EC_{50}$ for inhibition of an HDAC I and/or HDAC II, and even more preferably at least 10 fold, 100 fold or even 1000 fold less. Methods for assaying HDAC I and/or HDAC II activity are well known in the art and kits to perform such assays may be purchased commercially. See e.g., BioVision, Inc. (Mountain View, CA; world wide web at biovision.com) and Thomas Scientific (Swedesboro, NJ; world wide web at thomassci.com).

In certain embodiments, a sirtuin-modulating compound does not have any substantial ability to modulate sirtuin homo logs. In one embodiment, an activator of a human sirtuin protein may not have any substantial ability to activate a sirtuin protein from lower eukaryotes, particularly yeast or human pathogens, at concentrations (e.g., in vivo) effective for activating the deacetylase activity of human sirtuin. For example, a sirtuin-activating compound may be chosen to have an $EC_{50}$ for activating a human sirtuin, such as SIRT1 and/or SIRT3, deacetylase activity that is at least 5 fold less than the $EC_{50}$ for activating a yeast sirtuin, such as Sir2 (such as Candida, S. cerevisiae, etc.), and even more preferably at least 10 fold, 100 fold or even 1000 fold less. In another embodiment, an inhibitor of a sirtuin protein from lower eukaryotes, particularly yeast or human pathogens, does not have any substantial ability to inhibit a sirtuin protein from humans at concentrations (e.g., in vivo) effective for inhibiting the deacetylase activity of a sirtuin protein from a lower eukaryote. For example, a sirtuin-inhibiting compound may be chosen to have an $IC_{50}$ for inhibiting a human sirtuin, such as SIRT1, SIRT2 and/or SIRT3, deacetylase activity that is at least 5 fold less than the $IC_{50}$ for inhibiting a yeast sirtuin, such as Sir2 (such as Candida, S. cerevisiae, etc.), and even more preferably at least 10 fold, 100 fold or even 1000 fold less.

In certain embodiments, a sirtuin-modulating compound may have the ability to modulate one or more sirtuin protein homologs, such as, for example, one or more of human SIRT1, SIRT2, SIRT3, SIRT4, SIRT5, SIRT6, or SIRT7. In one embodiment, a sirtuin-modulating compound has the ability to modulate both a SIRT1 and a SIRT3 protein. In another embodiment, a sirtuin-modulating compound has the ability to modulate both a SIRT1 and a SIRT 2 protein. In particular embodiments, a sirtuin modulating compound has the ability to both activate SIRT1 and inhibit SIRT2 proteins.

In other embodiments, a SIRT1 modulator does not have any substantial ability to modulate other sirtuin protein homologs, such as, for example, one or more of human SIRT2, SIRT3, SIRT4, SIRT5, SIRT6, or SIRT7, at concentrations (e.g., in vivo) effective for modulating the deacetylase activity of human SIRT1. For example, a sirtuin-modulating compound may be chosen to have an $ED_{50}$ for modulating human SIRT 1 deacetylase activity that is at least 5 fold less than the $ED_{50}$ for modulating one or more of human SIRT2, SIRT3, SIRT4, SIRT5, SIRT6, or SIRT7, and even more preferably at least 10 fold, 100 fold or even 1000 fold less. In one embodiment, a SIRT1 modulator does not have any substantial ability to modulate a SIRT3 protein.

In other embodiments, a SIRT2 modulator does not have any substantial ability to modulate other sirtuin protein homologs, such as, for example, one or more of human SIRT1, SIRT3, SIRT4, SIRT5, SIRT6, or SIRT7, at concentrations (e.g., *in vivo*) effective for modulating the deacetylase activity of human SIRT2. For example, a sirtuin-modulating compound may be chosen to have an $ED_{50}$ for modulating human SIRT2 deacetylase activity that is at least 5 fold less than the $ED_{50}$ for modulating one or more of human SIRT1, SIRT3, SIRT4, SIRT5, SIRT6, or SIRT7, and even more preferably at least 10 fold, 100 fold or even 1000 fold less. In one embodiment, a SIRT2 modulator does not have any substantial ability to modulate a SIRT1 protein. In certain particular embodiments, the SIRT2 modulator inhibits the SIRT2 protein.

In other embodiments, a SIRT3 modulator does not have any substantial ability to modulate other sirtuin protein homologs, such as, for example, one or more of human SIRT1, SIRT2, SIRT4, SIRT5, SIRT6, or SIRT7, at concentrations (e.g., in vivo) effective for modulating the deacetylase activity of human SIRT3. For example, a sirtuin-modulating compound may be chosen to have an $ED_{50}$ for modulating human SIRT3 deacetylase activity that is at least 5 fold less than the $ED_{50}$ for modulating one or more of human SIRT1, SIRT2, SIRT4, SIRT5, SIRT6, or SIRT7, and even more preferably at least 10 fold, 100 fold or even 1000 fold less. In one embodiment, a SIRT3 modulator does not have any substantial ability to modulate a SIRT1 protein.

In certain embodiments, a sirtuin-modulating compound may have a binding affinity for a sirtuin protein of about $10^{-9}$M, $10^{-10}$M, $10^{-11}$M, $10^{-12}$M or less. A sirtuin-modulating compound may reduce (activator) or increase (inhibitor) the apparent Km of a sirtuin protein for its substrate or $NAD^+$ (or other cofactor) by a factor of at least about 2, 3, 4, 5, 10, 20, 30, 50 or 100. In certain embodiments, Km values are determined using the mass spectrometry assay described herein. Preferred activating compounds reduce the Km of a sirtuin for its substrate or cofactor to a greater extent than caused by resveratrol at a similar concentration or reduce the Km of a sirtuin for its substrate or cofactor similar to that caused by resveratrol at a lower concentration. A sirtuin-modulating compound may increase the $V_{max}$ of a sirtuin protein by a factor of at least about 2, 3, 4, 5, 10, 20, 30, 50 or 100. A sirtuin-modulating compound may have an $ED_{50}$ for modulating the deacetylase activity of a SIRT1 and/or SIRT3 protein of less than about 1 nM, less than about 10 nM, less than about 100 nM, less than about 1 $\mu$M, less than about 10 $\mu$M, less than about 100 $\mu$M, or from about 1-10 nM, from about 10-100 nM, from about 0.1-1 $\mu$M, from about 1-10 $\mu$M or from about 10-100 $\mu$M. A sirtuin-modulating compound may modulate the deacetylase activity of a SIRT1 and/or SIRT3 protein by a factor of at least about 5, 10, 20, 30, 50, or 100, as measured in a cellular assay or in a cell based assay. A sirtuin-activating compound may cause at least about 10%, 30%, 50%, 80%, 2 fold, 5 fold, 10 fold, 50 fold or 100 fold greater induction of the deacetylase activity of a sirtuin protein relative to the same concentration of resveratrol. A sirtuin-modulating compound may have an $ED_{50}$ for modulating SIRT5 that is at least about 10 fold, 20 fold, 30 fold, 50 fold greater than that for modulating SIRT1 and/or SIRT3.

### 3. Exemplary Uses

In certain aspects, the invention provides methods for modulating the level and/or activity of a sirtuin protein and methods of use thereof.

In certain embodiments, the invention provides methods for increasing sirtuin-1 activity in a cell comprising the step of contacting the cell with a compound represented by any one of Structural Formulas (XL), (XXXI), (XXXII), (XXXIII), and (XXXIV), including a compound of any of structural formulas (I), (II), (III), (IV), (V), (VI), (VII), (VIII), (IX), (X), (XI), (XII) (XIII), (XIV), (XV), (XVI), (XVII), (XVIII), (XIX), (XX), and (XXI).

In certain embodiments, the invention provides methods for using sirtuin-modulating compounds wherein the sirtuin-modulating compounds activate a sirtuin protein, e.g., increase the level and/or activity of a sirtuin protein. Sirtuin-modulating compounds that increase the level and/or activity of a sirtuin protein may be useful for a variety of therapeutic applications including, for example, increasing the lifespan of a cell, and treating and/or preventing a wide variety of diseases and disorders including, for example, diseases or disorders related to aging or stress, diabetes, obesity, neurodegenerative diseases, cardiovascular disease, blood clotting disorders, inflammation, cancer, and/or flushing,

etc. The methods comprise administering to a subject in need thereof a pharmaceutically effective amount of a sirtuin-modulating compound, e.g., a sirtuin-activating compound.

While Applicants do not wish to be bound by theory, it is believed that activators of the instant invention may interact with a sirtuin at the same location within the sirtuin protein (e.g., active site or site affecting the $K_m$ or $V_{max}$ of the active site). It is believed that this is the reason why certain classes of sirtuin activators and inhibitors can have substantial structural similarity.

In certain embodiments, the sirtuin-modulating compounds described herein may be taken alone or in combination with other compounds. In one embodiment, a mixture of two or more sirtuin-modulating compounds may be administered to a subject in need thereof. In another embodiment, a sirtuin-modulating compound that increases the level and/or activity of a sirtuin protein may be administered with one or more of the following compounds: resveratrol, butein, fisetin, piceatannol, or quercetin. In an exemplary embodiment, a sirtuin-modulating compound that increases the level and/or activity of a sirtuin protein may be administered in combination with nicotinic acid. In another embodiment, a sirtuin-modulating compound that decreases the level and/or activity of a sirtuin protein may be administered with one or more of the following compounds: nicotinamide (NAM), suranim; NF023 (a G-protein antagonist); NF279 (a purinergic receptor antagonist); Trolox (6-hydroxy-2,5,7,8,tetramethylchroman-2-carboxylic acid); (-)-epigallocatechin (hydroxy on sites 3,5,7,3',4', 5'); (-)-epigallocatechin gallate (Hydroxy sites 5,7,3',4',5' and gallate ester on 3); cyanidin choloride (3,5,7,3',4'-pentahydroxyflavylium chloride); delphinidin chloride (3,5,7,3',4',5'-hexahydroxyflavylium chloride); myricetin (cannabiscetin; 3,5,7,3',4',5'-hexahydroxyflavone); 3,7,3',4',5'-pentahydroxyflavone; gossypetin (3,5,7,8,3',4'-hexahydroxyflavone), sirtinol; and splitomicin. In yet another embodiment, one or more sirtuin-modulating compounds may be administered with one or more therapeutic agents for the treatment or prevention of various diseases, including, for example, cancer, diabetes, neurodegenerative diseases, cardiovascular disease, blood clotting, inflammation, flushing, obesity, ageing, stress, etc. In various embodiments, combination therapies comprising a sirtuin-modulating compound may refer to (1) pharmaceutical compositions that comprise one or more sirtuin-modulating compounds in combination with one or more therapeutic agents (e.g., one or more therapeutic agents described herein); and (2) coadministration of one or more sirtuin-modulating compounds with one or more therapeutic agents wherein the sirtuin-modulating compound and therapeutic agent have not been formulated in the same compositions (but may be present within the same kit or package, such as a blister pack or other multi-chamber package; connected, separately sealed containers (e.g., foil pouches) that can be separated by the user; or a kit where the sirtuin modulating compound(s) and other therapeutic agent(s) are in separate vessels). When using separate formulations, the sirtuin-modulating compound may be administered at the same, intermittent, staggered, prior to, subsequent to, or combinations thereof, with the administration of another therapeutic agent.

In certain embodiments, methods for reducing, preventing or treating diseases or disorders using a sirtuin-modulating compound may also comprise increasing the protein level of a sirtuin, such as human SIRT1, SIRT2 and/or SIRT3, or homologs thereof. Increasing protein levels can be achieved by introducing into a cell one or more copies of a nucleic acid that encodes a sirtuin. For example, the level of a sirtuin can be increased in a mammalian cell by introducing into the mammalian cell a nucleic acid encoding the sirtuin, e.g., increasing the level of SIRT1 by introducing a nucleic acid encoding the amino acid sequence set forth in GenBank Accession No. NP_036370 and/or increasing the level of SIRT3 by introducing a nucleic acid encoding the amino acid sequence set forth in GenBank Accession No. AAH01042.

A nucleic acid that is introduced into a cell to increase the protein level of a sirtuin may encode a protein that is at least about 80%, 85%, 90%, 95%, 98%, or 99% identical to the sequence of a sirtuin, e.g., SIRT1 and/or SIRT3 protein. For example, the nucleic acid encoding the protein may be at least about 80%, 85%, 90%, 95%, 98%, or 99% identical to a nucleic acid encoding a SIRT1 (e.g. GenBank Accession No. NM_012238) and/or SIRT3 (e.g., GenBank Accession No. BC001042) protein. The nucleic acid may also be a nucleic acid that hybridizes, preferably under stringent hybridization conditions, to a nucleic acid encoding a wild-type sirtuin, e.g., SIRT1 and/or SIRT3 protein. Stringent hybridization conditions may include hybridization and a wash in 0.2 x SSC at 65 °C. When using a nucleic acid that encodes a protein that is different from a wild-type sirtuin protein, such as a protein that is a fragment of a wild-type sirtuin, the protein is preferably biologically active, e.g., is capable of deacetylation. It is only necessary to express in a cell a portion of the sirtuin that is biologically active. For example, a protein that differs from wild-type SIRT1 having GenBank Accession No. NP_036370, preferably contains the core structure thereof. The core structure sometimes refers to amino acids 62-293 of GenBank Accession No. NP_036370, which are encoded by nucleotides 237 to 932 of GenBank Accession No. NM_012238, which encompasses the NAD binding as well as the substrate binding domains. The core domain of SIRT1 may also refer to about amino acids 261 to 447 of GenBank Accession No. NP_036370, which are encoded by nucleotides 834 to 1394 of GenBank Accession No. NM_012238; to about amino acids 242 to 493 of GenBank Accession No. NP_036370, which are encoded by nucleotides 777 to 1532 of GenBank Accession No. NM_012238; or to about amino acids 254 to 495 of GenBank Accession No. NP_036370, which are encoded by nucleotides 813 to 1538 of GenBank Accession No. NM_012238. Whether a protein retains a biological function, e.g., deacetylation capabilities, can be determined according to methods known in the art.

In certain embodiments, methods for reducing, preventing or treating diseases or disorders using a sirtuin-modulating compound may also comprise decreasing the protein level of a sirtuin, such as human SIRT1, SIRT2 and/or SIRT3, or homologs thereof. Decreasing a sirtuin protein level can be achieved according to methods known in the art. For example, an siRNA, an antisense nucleic acid, or a ribozyme targeted to the sirtuin can be expressed in the cell. A dominant negative sirtuin mutant, e.g., a mutant that is not capable of deacetylating, may also be used. For example, mutant H363Y of SIRT1, described, e.g., in Luo et al. (2001) Cell 107:137 can be used. Alternatively, agents that inhibit transcription can be used.

Methods for modulating sirtuin protein levels also include methods for modulating the transcription of genes encoding sirtuins, methods for stabilizing/destabilizing the corresponding mRNAs, and other methods known in the art.

***Aging/Stress***

In one embodiment, the invention provides a method extending the lifespan of a cell, extending the proliferative capacity of a cell, slowing aging of a cell, promoting the survival of a cell, delaying cellular senescence in a cell, mimicking the effects of calorie restriction, increasing the resistance of a cell to stress, or preventing apoptosis of a cell, by contacting the cell with a sirtuin-modulating compound of the invention that increases the level and/or activity of a sirtuin protein. In an exemplary embodiment, the methods comprise contacting the cell with a sirtuin-activating compound.

The methods described herein may be used to increase the amount of time that cells, particularly primary cells (i.e., cells obtained from an organism, e.g., a human), may be kept alive in a cell culture. Embryonic stem (ES) cells and pluripotent cells, and cells differentiated therefrom, may also be treated with a sirtuin-modulating compound that increases the level and/or activity of a sirtuin protein to keep the cells, or progeny thereof, in culture for longer periods of time. Such cells can also be used for transplantation into a subject, e.g., after ex vivo modification.

In one embodiment, cells that are intended to be preserved for long periods of time may be treated with a sirtuin-modulating compound that increases the level and/or activity of a sirtuin protein. The cells may be in suspension (e.g., blood cells, serum, biological growth media, etc.) or in tissues or organs. For example, blood collected from an individual for purposes of transfusion may be treated with a sirtuin-modulating compound that increases the level and/or activity of a sirtuin protein to preserve the blood cells for longer periods of time. Additionally, blood to be used for forensic purposes may also be preserved using a sirtuin-modulating compound that increases the level and/or activity of a sirtuin protein. Other cells that may be treated to extend their lifespan or protect against apoptosis include cells for consumption, e.g., cells from non-human mammals (such as meat) or plant cells (such as vegetables).

Sirtuin-modulating compounds that increase the level and/or activity of a sirtuin protein may also be applied during developmental and growth phases in mammals, plants, insects or microorganisms, in order to, e.g., alter, retard or accelerate the developmental and/or growth process.

In another embodiment, sirtuin-modulating compounds that increase the level and/or activity of a sirtuin protein may be used to treat cells useful for transplantation or cell therapy, including, for example, solid tissue grafts, organ transplants, cell suspensions, stem cells, bone marrow cells, etc. The cells or tissue may be an autograft, an allograft, a syngraft or a xenograft. The cells or tissue may be treated with the sirtuin-modulating compound prior to administration/implantation, concurrently with administration/implantation, and/or post administration/implantation into a subject. The cells or tissue may be treated prior to removal of the cells from the donor individual, *ex vivo* after removal of the cells or tissue from the donor individual, or post implantation into the recipient. For example, the donor or recipient individual may be treated systemically with a sirtuin-modulating compound or may have a subset of cells/tissue treated locally with a sirtuin-modulating compound that increases the level and/or activity of a sirtuin protein. In certain embodiments, the cells or tissue (or donor/recipient individuals) may additionally be treated with another therapeutic agent useful for prolonging graft survival, such as, for example, an immunosuppressive agent, a cytokine, an angiogenic factor, etc.

In yet other embodiments, cells may be treated with a sirtuin-modulating compound that increases the level and/or activity of a sirtuin protein *in vivo,* e.g., to increase their lifespan or prevent apoptosis. For example, skin can be protected from aging (e.g., developing wrinkles, loss of elasticity, etc.) by treating skin or epithelial cells with a sirtuin-modulating compound that increases the level and/or activity of a sirtuin protein. In an exemplary embodiment, skin is contacted with a pharmaceutical or cosmetic composition comprising a sirtuin-modulating compound that increases the level and/or activity of a sirtuin protein. Exemplary skin afflictions or skin conditions that may be treated in accordance with the methods described herein include disorders or diseases associated with or caused by inflammation, sun damage or natural aging. For example, the compositions find utility in the prevention or treatment of contact dermatitis (including irritant contact dermatitis and allergic contact dermatitis), atopic dermatitis (also known as allergic eczema), actinic keratosis, keratinization disorders (including eczema), epidermolysis bullosa diseases (including penfigus), exfoliative dermatitis, seborrheic dermatitis, erythemas (including erythema multiforme and erythema nodosum), damage caused by the sun or other light sources, discoid lupus erythematosus, dermatomyositis, psoriasis, skin cancer and the effects of natural aging. In another embodiment, sirtuin-modulating compounds that increase the level and/or activity of a sirtuin

protein may be used for the treatment of wounds and/or burns to promote healing, including, for example, first-, second- or third-degree burns and/or a thermal, chemical or electrical burns. The formulations may be administered topically, to the skin or mucosal tissue.

Topical formulations comprising one or more sirtuin-modulating compounds that increase the level and/or activity of a sirtuin protein may also be used as preventive, e.g., chemopreventive, compositions. When used in a chemopreventive method, susceptible skin is treated prior to any visible condition in a particular individual.

Sirtuin-modulating compounds may be delivered locally or systemically to a subject. In one embodiment, a sirtuin-modulating compound is delivered locally to a tissue or organ of a subject by injection, topical formulation, etc.

In another embodiment, a sirtuin-modulating compound that increases the level and/or activity of a sirtuin protein may be used for treating or preventing a disease or condition induced or exacerbated by cellular senescence in a subject; methods for decreasing the rate of senescence of a subject, e.g., after onset of senescence; methods for extending the lifespan of a subject; methods for treating or preventing a disease or condition relating to lifespan; methods for treating or preventing a disease or condition relating to the proliferative capacity of cells; and methods for treating or preventing a disease or condition resulting from cell damage or death. In certain embodiments, the method does not act by decreasing the rate of occurrence of diseases that shorten the lifespan of a subject. In certain embodiments, a method does not act by reducing the lethality caused by a disease, such as cancer.

In yet another embodiment, a sirtuin-modulating compound that increases the level and/or activity of a sirtuin protein may be administered to a subject in order to generally increase the lifespan of its cells and to protect its cells against stress and/or against apoptosis. It is believed that treating a subject with a compound described herein is similar to subjecting the subject to hormesis, i.e., mild stress that is beneficial to organisms and may extend their lifespan.

Sirtuin-modulating compounds that increase the level and/or activity of a sirtuin protein may be administered to a subject to prevent aging and aging-related consequences or diseases, such as stroke, heart disease, heart failure, arthritis, high blood pressure, and Alzheimer's disease. Other conditions that can be treated include ocular disorders, e.g., associated with the aging of the eye, such as cataracts, glaucoma, and macular degeneration. Sirtuin-modulating compounds that increase the level and/or activity of a sirtuin protein can also be administered to subjects for treatment of diseases, e.g., chronic diseases, associated with cell death, in order to protect the cells from cell death. Exemplary diseases include those associated with neural cell death, neuronal dysfunction, or muscular cell death or dysfunction, such as Parkinson's disease, Alzheimer's disease, multiple sclerosis, amniotropic lateral sclerosis, and muscular dystrophy; AIDS; fulminant hepatitis; diseases linked to degeneration of the brain, such as Creutzfeld-Jakob disease, retinitis pigmentosa and cerebellar degeneration; myelodysplasis such as aplastic anemia; ischemic diseases such as myocardial infarction and stroke; hepatic diseases such as alcoholic hepatitis, hepatitis B and hepatitis C; joint-diseases such as osteoarthritis; atherosclerosis; alopecia; damage to the skin due to UV light; lichen planus; atrophy of the skin; cataract; and graft rejections. Cell death can also be caused by surgery, drug therapy, chemical exposure or radiation exposure.

Sirtuin-modulating compounds that increase the level and/or activity of a sirtuin protein can also be administered to a subject suffering from an acute disease, e.g., damage to an organ or tissue, e.g., a subject suffering from stroke or myocardial infarction or a subject suffering from a spinal cord injury. Sirtuin-modulating compounds that increase the level and/or activity of a sirtuin protein may also be used to repair an alcoholic's liver.

### *Cardiovascular Disease*

In another embodiment, the invention provides a method for treating and/or preventing a cardiovascular disease by administering to a subject in need thereof a sirtuin-modulating compound that increases the level and/or activity of a sirtuin protein.

Cardiovascular diseases that can be treated or prevented using the sirtuin-modulating compounds that increase the level and/or activity of a sirtuin protein include cardiomyopathy or myocarditis; such as idiopathic cardiomyopathy, metabolic cardiomyopathy, alcoholic cardiomyopathy, drug-induced cardiomyopathy, ischemic cardiomyopathy, and hypertensive cardiomyopathy. Also treatable or preventable using compounds and methods described herein are atheromatous disorders of the major blood vessels (macrovascular disease) such as the aorta, the coronary arteries, the carotid arteries, the cerebrovascular arteries, the renal arteries, the iliac arteries, the femoral arteries, and the popliteal arteries. Other vascular diseases that can be treated or prevented include those related to platelet aggregation, the retinal arterioles, the glomerular arterioles, the vasa nervorum, cardiac arterioles, and associated capillary beds of the eye, the kidney, the heart, and the central and peripheral nervous systems. The sirtuin-modulating compounds that increase the level and/or activity of a sirtuin protein may also be used for increasing HDL levels in plasma of an individual.

Yet other disorders that may be treated with sirtuin-modulating compounds that increase the level and/or activity of a sirtuin protein include restenosis, e.g., following coronary intervention, and disorders relating to an abnormal level of high density and low density cholesterol.

In one embodiment, a sirtuin-modulating compound that increases the level and/or activity of a sirtuin protein may be administered as part of a combination therapeutic with another cardiovascular agent. In one embodiment, a

sirtuin-modulating compound that increases the level and/or activity of a sirtuin protein may be administered as part of a combination therapeutic with an anti-arrhythmia agent. In another embodiment, a sirtuin-modulating compound that increases the level and/or activity of a sirtuin protein may be administered as part of a combination therapeutic with another cardiovascular agent.

### *Cell Death/Cancer*

Sirtuin-modulating compounds may be administered to subjects who have recently received or are likely to receive a dose of radiation or toxin. In one embodiment, the dose of radiation or toxin is received as part of a work-related or medical procedure, e.g., administered as a prophylactic measure. In another embodiment, the radiation or toxin exposure is received unintentionally. In such a case, the compound is preferably administered as soon as possible after the exposure to inhibit apoptosis and the subsequent development of acute radiation syndrome.

Sirtuin-modulating compounds may also be used for treating and/or preventing cancer. The terms "cancer," "tumor," and "neoplasia" are used interchangeably herein. As used herein, a cancer (tumor or neoplasia) is characterized by one or more of the following properties: cell growth is not regulated by the normal biochemical and physical influences in the environment; anaplasia (e.g., lack of normal coordinated cell differentiation); and in some instances, metastasis. Cancer diseases that may be treated and/or prevented by sirtuin-modulating compounds of the invention include, for example, anal carcinoma, bladder carcinoma, breast carcinoma, cervix carcinoma, chronic lymphocytic leukemia, chronic myelogenous leukemia, endometrial carcinoma, hairy cell leukemia, head and neck carcinoma, lung (small cell) carcinoma, multiple myeloma, non-Hodgkin's lymphoma, follicular lymphoma, ovarian carcinoma, brain tumors, colorectal carcinoma, hepatocellular carcinoma, Kaposi's sarcoma, lung (non-small cell carcinoma), melanoma, pancreatic carcinoma, prostate carcinoma, renal cell carcinoma, and soft tissue sarcoma. Additional cancer disorders can be found in, for example, Isselbacher et al. (1994) Harrison's Principles of Internal Medicine 1814-1877, herein incorporated by reference.

In certain embodiments, sirtuin-modulating compounds that increase the level and/or activity of a sirtuin protein (e.g., SIRT1) or decrease the level and/or activity of a sirtuin protein (e.g., SIRT2) may be used for treating and/or preventing cancer. Calorie restriction has been linked to a reduction in the incidence of age-related disorders including cancer. Accordingly, an increase or decrease in the level and/or activity of a sirtuin protein may be useful for treating and/or preventing the incidence of age-related disorders, such as, for example, cancer. In cancers associated with solid tumors, a modulating compound may be administered directly into the tumor. Cancer of blood cells, e.g., leukemia, can be treated by administering a modulating compound into the blood stream or into the bone marrow. Benign cell growth, e.g., warts, can also be treated. Other diseases that can be treated include autoimmune diseases, e.g., systemic lupus erythematosus, scleroderma, and arthritis, in which autoimmune cells should be removed. Viral infections such as herpes, HIV, adenovirus, and HTLV-1 associated malignant and benign disorders can also be treated by administration of sirtuin-modulating compound. Alternatively, cells can be obtained from a subject, treated ex vivo to remove certain undesirable cells, e.g., cancer cells, and administered back to the same or a different subject.

Chemotherapeutic agents may be co-administered with modulating compounds described herein as having anti-cancer activity, e.g., compounds that induce apoptosis, compounds that reduce lifespan or compounds that render cells sensitive to stress. Chemotherapeutic agents may be used by themselves with a sirtuin-modulating compound described herein as inducing cell death or reducing lifespan or increasing sensitivity to stress and/or in combination with other chemotherapeutics agents.

In addition to conventional chemotherapeutics, the sirtuin-modulating compounds described herein may also be used with antisense RNA, RNAi or other polynucleotides to inhibit the expression of the cellular components that contribute to unwanted cellular proliferation.

Combination therapies comprising sirtuin-modulating compounds and a conventional chemotherapeutic agent may be advantageous over combination therapies known in the art because the combination allows the conventional chemotherapeutic agent to exert greater effect at lower dosage. In a preferred embodiment, the effective dose ($ED_{50}$) for a chemotherapeutic agent, or combination of conventional chemotherapeutic agents, when used in combination with a sirtuin-modulating compound is at least 2 fold less than the $ED_{50}$ for the chemotherapeutic agent alone, and even more preferably at 5 fold, 10 fold or even 25 fold less. Conversely, the therapeutic index (TI) for such chemotherapeutic agent or combination of such chemotherapeutic agent when used in combination with a sirtuin-modulating compound described herein can be at least 2 fold greater than the TI for conventional chemotherapeutic regimen alone, and even more preferably at 5 fold, 10 fold or even 25 fold greater.

### *Neuronal Diseases/Disorders*

In certain aspects, sirtuin-modulating compounds that increase the level and/or activity of a sirtuin protein can be used to treat patients suffering from neurodegenerative diseases, and traumatic or mechanical injury to the central

nervous system (CNS), spinal cord or peripheral nervous system (PNS). Neurodegenerative disease typically involves reductions in the mass and volume of the human brain, which may be due to the atrophy and/or death of brain cells, which are far more profound than those in a healthy person that are attributable to aging. Neurodegenerative diseases can evolve gradually, after a long period of normal brain function, due to progressive degeneration (e.g., nerve cell dysfunction and death) of specific brain regions. Alternatively, neurodegenerative diseases can have a quick onset, such as those associated with trauma or toxins. The actual onset of brain degeneration may precede clinical expression by many years. Examples of neurodegenerative diseases include, but are not limited to, Alzheimer's disease (AD), Parkinson's disease (PD), Huntington's disease (HD), amyotrophic lateral sclerosis (ALS; Lou Gehrig's disease), diffuse Lewy body disease, chorea-acanthocytosis, primary lateral sclerosis, ocular diseases (ocular neuritis), chemotherapy-induced neuropathies (e.g., from vincristine, paclitaxel, bortezomib), diabetes-induced neuropathies and Friedreich's ataxia. Sirtuin-modulating compounds that increase the level and/or activity of a sirtuin protein can be used to treat these disorders and others as described below.

AD is a CNS disorder that results in memory loss, unusual behavior, personality changes, and a decline in thinking abilities. These losses are related to the death of specific types of brain cells and the breakdown of connections and their supporting network (e.g. glial cells) between them. The earliest symptoms include loss of recent memory, faulty judgment, and changes in personality. PD is a CNS disorder that results in uncontrolled body movements, rigidity, tremor, and dyskinesia, and is associated with the death of brain cells in an area of the brain that produces dopamine. ALS (motor neuron disease) is a CNS disorder that attacks the motor neurons, components of the CNS that connect the brain to the skeletal muscles.

HD is another neurodegenerative disease that causes uncontrolled movements, loss of intellectual faculties, and emotional disturbance. Tay-Sachs disease and Sandhoff disease are glycolipid storage diseases where GM2 ganglioside and related glycolipidssubstrates for β-hexosaminidase accumulate in the nervous system and trigger acute neurodegeneration.

It is well-known that apoptosis plays a role in AIDS pathogenesis in the immune system. However, HIV-1 also induces neurological disease, which can be treated with sirtuin-modulating compounds of the invention.

Neuronal loss is also a salient feature of prion diseases, such as Creutzfeldt-Jakob disease in human, BSE in cattle (mad cow disease), Scrapie Disease in sheep and goats, and feline spongiform encephalopathy (FSE) in cats. Sirtuin-modulating compounds that increase the level and/or activity of a sirtuin protein may be useful for treating or preventing neuronal loss due to these prior diseases.

In another embodiment, a sirtuin-modulating compound that increases the level and/or activity of a sirtuin protein may be used to treat or prevent any disease or disorder involving axonopathy. Distal axonopathy is a type of peripheral neuropathy that results from some metabolic or toxic derangement of peripheral nervous system (PNS) neurons. It is the most common response of nerves to metabolic or toxic disturbances, and as such may be caused by metabolic diseases such as diabetes, renal failure, deficiency syndromes such as malnutrition and alcoholism, or the effects of toxins or drugs. Those with distal axonopathies usually present with symmetrical glove-stocking sensori-motor disturbances. Deep tendon reflexes and autonomic nervous system (ANS) functions are also lost or diminished in affected areas.

Diabetic neuropathies are neuropathic disorders that are associated with diabetes mellitus. Relatively common conditions which may be associated with diabetic neuropathy include third nerve palsy; mononeuropathy; mononeuritis multiplex; diabetic amyotrophy; a painful polyneuropathy; autonomic neuropathy; and thoracoabdominal neuropathy.

Peripheral neuropathy is the medical term for damage to nerves of the peripheral nervous system, which may be caused either by diseases of the nerve or from the side-effects of systemic illness. Major causes of peripheral neuropathy include seizures, nutritional deficiencies, and HIV, though diabetes is the most likely cause.

In an exemplary embodiment, a sirtuin-modulating compound that increases the level and/or activity of a sirtuin protein may be used to treat or prevent multiple sclerosis (MS), including relapsing MS and monosymptomatic MS, and other demyelinating conditions, such as, for example, chromic inflammatory demyelinating polyneuropathy (CIDP), or symptoms associated therewith.

In yet another embodiment, a sirtuin-modulating compound that increases the level and/or activity of a sirtuin protein may be used to treat trauma to the nerves, including, trauma due to disease, injury (including surgical intervention), or environmental trauma (e.g., neurotoxins, alcoholism, etc.).

Sirtuin-modulating compounds that increase the level and/or activity of a sirtuin protein may also be useful to prevent, treat, and alleviate symptoms of various PNS disorders. The term "peripheral neuropathy" encompasses a wide range of disorders in which the nerves outside of the brain and spinal cord-peripheral nerves-have been damaged. Peripheral neuropathy may also be referred to as peripheral neuritis, or if many nerves are involved, the terms polyneuropathy or polyneuritis may be used.

PNS diseases treatable with sirtuin-modulating compounds that increase the level and/or activity of a sirtuin protein include: diabetes, leprosy, Charcot-Marie-Tooth disease, Guillain-Barré syndrome and Brachial Plexus Neuropathies (diseases of the cervical and first thoracic roots, nerve trunks, cords, and peripheral nerve components of the

brachial plexus.

In another embodiment, a sirtuin activating compound may be used to treat or prevent a polyglutamine disease. Exemplary polyglutamine diseases include Spinobulbar muscular atrophy (Kennedy disease), Huntington's Disease (HD), Dentatorubral-pallidoluysian atrophy (Haw River syndrome), Spinocerebellar ataxia type 1, Spinocerebellar ataxia type 2, Spinocerebellar ataxia type 3 (Machado-Joseph disease), Spinocerebellar ataxia type 6, Spinocerebellar ataxia type 7, and Spinocerebellar ataxia type 17.

In certain embodiments, the invention provides a method to treat a central nervous system cell to prevent damage in response to a decrease in blood flow to the cell. Typically the severity of damage that may be prevented will depend in large part on the degree of reduction in blood flow to the cell and the duration of the reduction. In one embodiment, apoptotic or necrotic cell death may be prevented. In still a further embodiment, ischemic-mediated damage, such as cytoxic edema or central nervous system tissue anoxemia, may be prevented. In each embodiment, the central nervous system cell may be a spinal cell or a brain cell.

Another aspect encompasses administrating a sirtuin activating compound to a subject to treat a central nervous system ischemic condition. A number of central nervous system ischemic conditions may be treated by the sirtuin activating compounds described herein. In one embodiment, the ischemic condition is a stroke that results in any type of ischemic central nervous system damage, such as apoptotic or necrotic cell death, cytoxic edema or central nervous system tissue anoxia. The stroke may impact any area of the brain or be caused by any etiology commonly known to result in the occurrence of a stroke. In one alternative of this embodiment, the stroke is a brain stem stroke. In another alternative of this embodiment, the stroke is a cerebellar stroke. In still another embodiment, the stroke is an embolic stroke. In yet another alternative, the stroke may be a hemorrhagic stroke. In a further embodiment, the stroke is a thrombotic stroke.

In yet another aspect, a sirtuin activating compound may be administered to reduce infarct size of the ischemic core following a central nervous system ischemic condition. Moreover, a sirtuin activating compound may also be beneficially administered to reduce the size of the ischemic penumbra or transitional zone following a central nervous system ischemic condition.

In one embodiment, a combination drug regimen may include drugs or compounds for the treatment or prevention of neurodegenerative disorders or secondary conditions associated with these conditions. Thus, a combination drug regimen may include one or more sirtuin activators and one or more anti-neurodegeneration agents.

### *Blood Coagulation Disorders*

In other aspects, sirtuin-modulating compounds that increase the level and/or activity of a sirtuin protein can be used to treat or prevent blood coagulation disorders (or hemostatic disorders). As used interchangeably herein, the terms "hemostasis", "blood coagulation," and "blood clotting" refer to the control of bleeding, including the physiological properties of vasoconstriction and coagulation. Blood coagulation assists in maintaining the integrity of mammalian circulation after injury, inflammation, disease, congenital defect, dysfunction or other disruption. Further, the formation of blood clots does not only limit bleeding in case of an injury (hemostasis), but may lead to serious organ damage and death in the context of atherosclerotic diseases by occlusion of an important artery or vein. Thrombosis is thus blood clot formation at the wrong time and place.

Accordingly, the present invention provides anticoagulation and antithrombotic treatments aiming at inhibiting the formation of blood clots in order to prevent or treat blood coagulation disorders, such as myocardial infarction, stroke, loss of a limb by peripheral artery disease or pulmonary embolism.

As used interchangeably herein, "modulating or modulation of hemostasis" and "regulating or regulation of hemostasis" includes the induction (e.g., stimulation or increase) of hemostasis, as well as the inhibition (e.g., reduction or decrease) of hemostasis.

In one aspect, the invention provides a method for reducing or inhibiting hemostasis in a subject by administering a sirtuin-modulating compound that increases the level and/or activity of a sirtuin protein. The compositions and methods disclosed herein are useful for the treatment or prevention of thrombotic disorders. As used herein, the term "thrombotic disorder" includes any disorder or condition characterized by excessive or unwanted coagulation or hemostatic activity, or a hypercoagulable state. Thrombotic disorders include diseases or disorders involving platelet adhesion and thrombus formation, and may manifest as an increased propensity to form thromboses, e.g., an increased number of thromboses, thrombosis at an early age, a familial tendency towards thrombosis, and thrombosis at unusual sites.

In another embodiment, a combination drug regimen may include drugs or compounds for the treatment or prevention of blood coagulation disorders or secondary conditions associated with these conditions. Thus, a combination drug regimen may include one or more sirtuin-modulating compounds that increase the level and/or activity of a sirtuin protein and one or more anti-coagulation or anti-thrombosis agents.

***Weight Control***

In another aspect, sirtuin-modulating compounds that increase the level and/or activity of a sirtuin protein may be used for treating or preventing weight gain or obesity in a subject. For example, sirtuin-modulating compounds that increase the level and/or activity of a sirtuin protein may be used, for example, to treat or prevent hereditary obesity, dietary obesity, hormone related obesity, obesity related to the administration of medication, to reduce the weight of a subject, or to reduce or prevent weight gain in a subject. A subject in need of such a treatment may be a subject who is obese, likely to become obese, overweight, or likely to become overweight. Subjects who are likely to become obese or overweight can be identified, for example, based on family history, genetics, diet, activity level, medication intake, or various combinations thereof.

In yet other embodiments, sirtuin-modulating compounds that increase the level and/or activity of a sirtuin protein may be administered to subjects suffering from a variety of other diseases and conditions that may be treated or prevented by promoting weight loss in the subject. Such diseases include, for example, high blood pressure, hypertension, high blood cholesterol, dyslipidemia, type 2 diabetes, insulin resistance, glucose intolerance, hyperinsulinemia, coronary heart disease, angina pectoris, congestive heart failure, stroke, gallstones, cholescystitis and cholelithiasis, gout, osteoarthritis, obstructive sleep apnea and respiratory problems, some types of cancer (such as endometrial, breast, prostate, and colon), complications of pregnancy, poor female reproductive health (such as menstrual irregularities, infertility, irregular ovulation), bladder control problems (such as stress incontinence); uric acid nephrolithiasis; psychological disorders (such as depression, eating disorders, distorted body image, and low self esteem). Finally, patients with AIDS can develop lipodystrophy or insulin resistance in response to combination therapies for AIDS.

In another embodiment, sirtuin-modulating compounds that increase the level and/or activity of a sirtuin protein may be used for inhibiting adipogenesis or fat cell differentiation, whether *in vitro* or *in vivo.* Such methods may be used for treating or preventing obesity.

In other embodiments, sirtuin-modulating compounds that increase the level and/or activity of a sirtuin protein may be used for reducing appetite and/or increasing satiety, thereby causing weight loss or avoidance of weight gain. A subject in need of such a treatment may be a subject who is overweight, obese or a subject likely to become overweight or obese. The method may comprise administering daily or, every other day, or once a week, a dose, e.g., in the form of a pill, to a subject. The dose may be an "appetite reducing dose."

In an exemplary embodiment, sirtuin-modulating compounds that increase the level and/or activity of a sirtuin protein may be administered as a combination therapy for treating or preventing weight gain or obesity. For example, one or more sirtuin-modulating compounds that increase the level and/or activity of a sirtuin protein may be administered in combination with one or more anti-obesity agents.

In another embodiment, sirtuin-modulating compounds that increase the level and/or activity of a sirtuin protein may be administered to reduce drug-induced weight gain. For example, a sirtuin-modulating compound that increases the level and/or activity of a sirtuin protein may be administered as a combination therapy with medications that may stimulate appetite or cause weight gain, in particular, weight gain due to factors other than water retention.

***Metabolic Disorders/Diabetes***

In another aspect, sirtuin-modulating compounds that increase the level and/or activity of a sirtuin protein may be used for treating or preventing a metabolic disorder, such as insulin-resistance, a pre-diabetic state, type II diabetes, and/or complications thereof. Administration of a sirtuin-modulating compounds that increases the level and/or activity of a sirtuin protein may increase insulin sensitivity and/or decrease insulin levels in a subject. A subject in need of such a treatment may be a subject who has insulin resistance or other precursor symptom of type II diabetes, who has type II diabetes, or who is likely to develop any of these conditions. For example, the subject may be a subject having insulin resistance, e.g., having high circulating levels of insulin and/or associated conditions, such as hyperlipidemia, dyslipogenesis, hypercholesterolemia, impaired glucose tolerance, high blood glucose sugar level, other manifestations of syndrome X, hypertension, atherosclerosis and lipodystrophy.

In an exemplary embodiment, sirtuin-modulating compounds that increase the level and/or activity of a sirtuin protein may be administered as a combination therapy for treating or preventing a metabolic disorder. For example, one or more sirtuin-modulating compounds that increase the level and/or activity of a sirtuin protein may be administered in combination with one or more anti-diabetic agents.

***Inflammatory Diseases***

In other aspects, sirtuin-modulating compounds that increase the level and/or activity of a sirtuin protein can be used to treat or prevent a disease or disorder associated with inflammation. Sirtuin-modulating compounds that increase the level and/or activity of a sirtuin protein may be administered prior to the onset of, at, or after the initiation

of inflammation. When used prophylactically, the compounds are preferably provided in advance of any inflammatory response or symptom. Administration of the compounds may prevent or attenuate inflammatory responses or symptoms.

In another embodiment, sirtuin-modulating compounds that increase the level and/or activity of a sirtuin protein may be used to treat or prevent allergies and respiratory conditions, including asthma, bronchitis, pulmonary fibrosis, allergic rhinitis, oxygen toxicity, emphysema, chronic bronchitis, acute respiratory distress syndrome, and any chronic obstructive pulmonary disease (COPD). The compounds may be used to treat chronic hepatitis infection, including hepatitis B and hepatitis C.

Additionally, sirtuin-modulating compounds that increase the level and/or activity of a sirtuin protein may be used to treat autoimmune diseases and/or inflammation associated with autoimmune diseases such as organ-tissue autoimmune diseases (e.g., Raynaud's syndrome), scleroderma, myasthenia gravis, transplant rejection, endotoxin shock, sepsis, psoriasis, eczema, dermatitis, multiple sclerosis, autoimmune thyroiditis, uveitis, systemic lupus erythematosis, Addison's disease, autoimmune polyglandular disease (also known as autoimmune polyglandular syndrome), and Grave's disease.

In certain embodiments, one or more sirtuin-modulating compounds that increase the level and/or activity of a sirtuin protein may be taken alone or in combination with other compounds useful for treating or preventing inflammation.

***Flushing***

In another aspect, sirtuin-modulating compounds that increase the level and/or activity of a sirtuin protein may be used for reducing the incidence or severity of flushing and/or hot flashes which are symptoms of a disorder. For instance, the subject method includes the use of sirtuin-modulating compounds that increase the level and/or activity of a sirtuin protein, alone or in combination with other agents, for reducing incidence or severity of flushing and/or hot flashes in cancer patients. In other embodiments, the method provides for the use of sirtuin-modulating compounds that increase the level and/or activity of a sirtuin protein to reduce the incidence or severity of flushing and/or hot flashes in menopausal and post-menopausal woman.

In another aspect, sirtuin-modulating compounds that increase the level and/or activity of a sirtuin protein may be used as a therapy for reducing the incidence or severity of flushing and/or hot flashes which are side-effects of another drug therapy, e.g., drug-induced flushing. In certain embodiments, a method for treating and/or preventing drug-induced flushing comprises administering to a patient in need thereof a formulation comprising at least one flushing inducing compound and at least one sirtuin-modulating compound that increases the level and/or activity of a sirtuin protein. In other embodiments, a method for treating drug induced flushing comprises separately administering one or more compounds that induce flushing and one or more sirtuin-modulating compounds, e.g., wherein the sirtuin-modulating compound and flushing inducing agent have not been formulated in the same compositions. When using separate formulations, the sirtuin-modulating compound may be administered (1) at the same as administration of the flushing inducing agent, (2) intermittently with the flushing inducing agent, (3) staggered relative to administration of the flushing inducing agent, (4) prior to administration of the flushing inducing agent, (5) subsequent to administration of the flushing inducing agent, and (6) various combination thereof. Exemplary flushing inducing agents include, for example, niacin, faloxifene, antidepressants, anti-psychotics, chemotherapeutics, calcium channel blockers, and antibiotics.

In one embodiment, sirtuin-modulating compounds that increase the level and/or activity of a sirtuin protein may be used to reduce flushing side effects of a vasodilator or an antilipemic agent (including anticholesteremic agents and lipotropic agents). In an exemplary embodiment, a sirtuin-modulating compound that increases the level and/or activity of a sirtuin protein may be used to reduce flushing associated with the administration of niacin.

In another embodiment, the invention provides a method for treating and/or preventing hyperlipidemia with reduced flushing side effects. In another representative embodiment, the method involves the use of sirtuin-modulating compounds that increase the level and/or activity of a sirtuin protein to reduce flushing side effects of raloxifene. In another representative embodiment, the method involves the use of sirtuin-modulating compounds that increase the level and/or activity of a sirtuin protein to reduce flushing side effects of antidepressants or anti-psychotic agent. For instance, sirtuin-modulating compounds that increase the level and/or activity of a sirtuin protein can be used in conjunction (administered separately or together) with a serotonin reuptake inhibitor, or a 5HT2 receptor antagonist.

In certain embodiments, sirtuin-modulating compounds that increase the level and/or activity of a sirtuin protein may be used as part of a treatment with a serotonin reuptake inhibitor (SRI) to reduce flushing. In still another representative embodiment, sirtuin-modulating compounds that increase the level and/or activity of a sirtuin protein may be used to reduce flushing side effects of chemotherapeutic agents, such as cyclophosphamide and tamoxifen.

In another embodiment, sirtuin-modulating compounds that increase the level and/or activity of a sirtuin protein may be used to reduce flushing side effects of calcium channel blockers, such as amlodipine.

In another embodiment, sirtuin-modulating compounds that increase the level and/or activity of a sirtuin protein may be used to reduce flushing side effects of antibiotics. For example, sirtuin-modulating compounds that increase the level and/or activity of a sirtuin protein can be used in combination with levofloxacin.

***Ocular Disorders***

One aspect of the present invention is a method for inhibiting, reducing or otherwise treating vision impairment by administering to a patient a therapeutic dosage of sirtuin modulator selected from a compound disclosed herein, or a pharmaceutically acceptable salt, prodrug or a metabolic derivative thereof.

In certain aspects of the invention, the vision impairment is caused by damage to the optic nerve or central nervous system. In particular embodiments, optic nerve damage is caused by high intraocular pressure, such as that created by glaucoma. In other particular embodiments, optic nerve damage is caused by swelling of the nerve, which is often associated with an infection or an immune (e.g., autoimmune) response such as in optic neuritis.

In certain aspects of the invention, the vision impairment is caused by retinal damage. In particular embodiments, retinal damage is caused by disturbances in blood flow to the eye (e.g., arteriosclerosis, vasculitis). In particular embodiments, retinal damage is caused by disrupton of the macula (e.g., exudative or non-exudative macular degeneration).

Exemplary retinal diseases include Exudative Age Related Macular Degeneration, Nonexudative Age Related Macular Degeneration, Retinal Electronic Prosthesis and RPE Transplantation Age Related Macular Degeneration, Acute Multifocal Placoid Pigment Epitheliopathy, Acute Retinal Necrosis, Best Disease, Branch Retinal Artery Occlusion, Branch Retinal Vein Occlusion, Cancer Associated and Related Autoimmune Retinopathies, Central Retinal Artery Occlusion, Central Retinal Vein Occlusion, Central Serous Chorioretinopathy, Eales Disease, Epimacular Membrane, Lattice Degeneration, Macroaneurysm, Diabetic Macular Edema, Irvine-Gass Macular Edema, Macular Hole, Subretinal Neovascular Membranes, Diffuse Unilateral Subacute Neuroretinitis, Nonpseudophakic Cystoid Macular Edema, Presumed Ocular Histoplasmosis Syndrome, Exudative Retinal Detachment, Postoperative Retinal Detachment, Proliferative Retinal Detachment, Rhegmatogenous Retinal Detachment, Tractional Retinal Detachment, Retinitis Pigmentosa, CMV Retinitis, Retinoblastoma, Retinopathy of Prematurity, Birdshot Retinopathy, Background Diabetic Retinopathy, Proliferative Diabetic Retinopathy, Hemoglobinopathies Retinopathy, Purtscher Retinopathy, Valsalva Retinopathy, Juvenile Retinoschisis, Senile Retinoschisis, Terson Syndrome and White Dot Syndromes.

Other exemplary diseases include ocular bacterial infections (e.g. conjunctivitis, keratitis, tuberculosis, syphilis, gonorrhea), viral infections (e.g. Ocular Herpes Simplex Virus, Varicella Zoster Virus, Cytomegalovirus retinitis, Human Immunodeficiency Virus (HIV)) as well as progressive outer retinal necrosis secondary to HIV or other HIV-associated and other immunodeficiency-associated ocular diseases. In addition, ocular diseases include fungal infections (e.g. Candida choroiditis, histoplasmosis), protozoal infections (e.g. toxoplasmosis) and others such as ocular toxocariasis and sarcoidosis.

One aspect of the invention is a method for inhibiting, reducing or treating vision impairment in a subject undergoing treatment with a chemotherapeutic drug (e.g., a neurotoxic drug, a drug that raises intraocular pressure such as a steroid), by administering to the subject in need of such treatment a therapeutic dosage of a sirtuin modulator disclosed herein.

Another aspect of the invention is a method for inhibiting, reducing or treating vision impairment in a subject undergoing surgery, including ocular or other surgeries performed in the prone position such as spinal cord surgery, by administering to the subject in need of such treatment a therapeutic dosage of a sirtuin modulator disclosed herein. Ocular surgeries include cataract, iridotomy and lens replacements.

Another aspect of the invention is the treatment, including inhibition and prophylactic treatment, of age related ocular diseases include cataracts, dry eye, age-related macular degeneration (AMD), retinal damage and the like, by administering to the subject in need of such treatment a therapeutic dosage of a sirtuin modulator disclosed herein.

Another aspect of the invention is the prevention or treatment of damage to the eye caused by stress, chemical insult or radiation, by administering to the subject in need of such treatment a therapeutic dosage of a sirtuin modulator disclosed herein. Radiation or electromagnetic damage to the eye can include that caused by CRT's or exposure to sunlight or UV.

In one embodiment, a combination drug regimen may include drugs or compounds for the treatment or prevention of ocular disorders or secondary conditions associated with these conditions. Thus, a combination drug regimen may include one or more sirtuin activators and one or more therapeutic agents for the treatment of an ocular disorder.

In one embodiment, a sirtuin modulator can be administered in conjunction with a therapy for reducing intraocular pressure. In another embodiment, a sirtuin modulator can be administered in conjunction with a therapy for treating and/or preventing glaucoma. In yet another embodiment, a sirtuin modulator can be administered in conjunction with a therapy for treating and/or preventing optic neuritis. In one embodiment, a sirtuin modulator can be administered in conjunction with a therapy for treating and/or preventing CMV Retinopathy. In another embodiment, a sirtuin modulator can be administered in conjunction with a therapy for treating and/or preventing multiple sclerosis.

***Mitochondrial-Associated Diseases and Disorders***

In certain embodiments, the invention provides methods for treating diseases or disorders that would benefit

from increased mitochondrial activity. The methods involve administering to a subject in need thereof a therapeutically effective amount of a sirtuin activating compound. Increased mitochondrial activity refers to increasing activity of the mitochondria while maintaining the overall numbers of mitochondria (e.g., mitochondrial mass), increasing the numbers of mitochondria thereby increasing mitochondrial activity (e.g., by stimulating mitochondrial biogenesis), or combinations thereof. In certain embodiments, diseases and disorders that would benefit from increased mitochondrial activity include diseases or disorders associated with mitochondrial dysfunction.

In certain embodiments, methods for treating diseases or disorders that would benefit from increased mitochondrial activity may comprise identifying a subject suffering from a mitochondrial dysfunction. Methods for diagnosing a mitochondrial dysfunction may involve molecular genetic, pathologic and/or biochemical analyses. Diseases and disorders associated with mitochondrial dysfunction include diseases and disorders in which deficits in mitochondrial respiratory chain activity contribute to the development of pathophysiology of such diseases or disorders in a mammal. Diseases or disorders that would benefit from increased mitochondrial activity generally include for example, diseases in which free radical mediated oxidative injury leads to tissue degeneration, diseases in which cells inappropriately undergo apoptosis, and diseases in which cells fail to undergo apoptosis.

In certain embodiments, the invention provides methods for treating a disease or disorder that would benefit from increased mitochondrial activity that involves administering to a subject in need thereof one or more sirtuin activating compounds in combination with another therapeutic agent such as, for example, an agent useful for treating mitochondrial dysfunction or an agent useful for reducing a symptom associated with a disease or disorder involving mitochondrial dysfunction.

In exemplary embodiments, the invention provides methods for treating diseases or disorders that would benefit from increased mitochondrial activity by administering to a subject a therapeutically effective amount of a sirtuin activating compound. Exemplary diseases or disorders include, for example, neuromuscular disorders (e.g., Friedreich's Ataxia, muscular dystrophy, multiple sclerosis, etc.), disorders of neuronal instability (e.g., seizure disorders, migrane, etc.), developmental delay, neurodegenerative disorders (e.g., Alzheimer's Disease, Parkinson's Disease, amyotrophic lateral sclerosis, etc.), ischemia, renal tubular acidosis, age-related neurodegeneration and cognitive decline, chemotherapy fatigue, age-related or chemotherapy-induced menopause or irregularities of menstrual cycling or ovulation, mitochondrial myopathies, mitochondrial damage (e.g., calcium accumulation, excitotoxicity, nitric oxide exposure, hypoxia, etc.), and mitochondrial deregulation.

Muscular dystrophy refers to a family of diseases involving deterioration of neuromuscular structure and function, often resulting in atrophy of skeletal muscle and myocardial dysfunction, such as Duchenne muscular dystrophy. In certain embodiments, sirtuin activating compounds may be used for reducing the rate of decline in muscular functional capacities and for improving muscular functional status in patients with muscular dystrophy.

In certain embodiments, sirtuin modulating compounds may be useful for treatment mitochondrial myopathies. Mitochondrial myopathies range from mild, slowly progressive weakness of the extraocular muscles to severe, fatal infantile myopathies and multisystem encephalomyopathies. Some syndromes have been defined, with some overlap between them. Established syndromes affecting muscle include progressive external ophthalmoplegia, the Kearns-Sayre syndrome (with ophthalmoplegia, pigmentary retinopathy, cardiac conduction defects, cerebellar ataxia, and sensorineural deafness), the MELAS syndrome (mitochondrial encephalomyopathy, lactic acidosis, and stroke-like episodes), the MERFF syndrome (myoclonic epilepsy and ragged red fibers), limb-girdle distribution weakness, and infantile myopathy (benign or severe and fatal).

In certain embodiments, sirtuin activating compounds may be useful for treating patients suffering from toxic damage to mitochondria, such as, toxic damage due to calcium accumulation, excitotoxicity, nitric oxide exposure, drug induced toxic damage, or hypoxia.

In certain embodiments, sirtuin activating compounds may be useful for treating diseases or disorders associated with mitochondrial deregulation.

### *Muscle Performance*

In other embodiments, the invention provides methods for enhancing muscle performance by administering a therapeutically effective amount of a sirtuin activating compound. For example, sirtuin activating compounds may be useful for improving physical endurance (e.g., ability to perform a physical task such as exercise, physical labor, sports activities, etc.), inhibiting or retarding physical fatigues, enhancing blood oxygen levels, enhancing energy in healthy individuals, enhance working capacity and endurance, reducing muscle fatigue, reducing stress, enhancing cardiac and cardiovascular function, improving sexual ability, increasing muscle ATP levels, and/or reducing lactic acid in blood. In certain embodiments, the methods involve administering an amount of a sirtuin activating compound that increase mitochondrial activity, increase mitochondrial biogenesis, and/or increase mitochondrial mass.

Sports performance refers to the ability of the athlete's muscles to perform when participating in sports activities. Enhanced sports performance, strength, speed and endurance are measured by an increase in muscular contraction

strength, increase in amplitude of muscle contraction, shortening of muscle reaction time between stimulation and contraction. Athlete refers to an individual who participates in sports at any level and who seeks to achieve an improved level of strength, speed and endurance in their performance, such as, for example, body builders, bicyclists, long distance runners, short distance runners, etc. Enhanced sports performance in manifested by the ability to overcome muscle fatigue, ability to maintain activity for longer periods of time, and have a more effective workout.

In the arena of athlete muscle performance, it is desirable to create conditions that permit competition or training at higher levels of resistance for a prolonged period of time.

It is contemplated that the methods of the present invention will also be effective in the treatment of muscle related pathological conditions, including acute sarcopenia, for example, muscle atrophy and/or cachexia associated with burns, bed rest, limb immobilization, or major thoracic, abdominal, and/or orthopedic surgery.

In certain embodiments, the invention provides novel dietary compositions comprising sirtuin modulators, a method for their preparation, and a method of using the compositions for improvement of sports performance. Accordingly, provided are therapeutic compositions, foods and beverages that have actions of improving physical endurance and/or inhibiting physical fatigues for those people involved in broadly-defined exercises including sports requiring endurance and labors requiring repeated muscle exertions. Such dietary compositions may additional comprise electrolytes, caffeine, vitamins, carbohydrates, etc.

***Other Uses***

Sirtuin-modulating compounds that increase the level and/or activity of a sirtuin protein may be used for treating or preventing viral infections (such as infections by influenza, herpes or papilloma virus) or as antifungal agents. In certain embodiments, sirtuin-modulating compounds that increase the level and/or activity of a sirtuin protein may be administered as part of a combination drug therapy with another therapeutic agent for the treatment of viral diseases. In another embodiment, sirtuin-modulating compounds that increase the level and/or activity of a sirtuin protein may be administered as part of a combination drug therapy with another anti-fungal agent.

Subjects that may be treated as described herein include eukaryotes, such as mammals, e.g., humans, ovines, bovines, equines, porcines, canines, felines, non-human primate, mice, and rats. Cells that may be treated include eukaryotic cells, e.g., from a subject described above, or plant cells, yeast cells and prokaryotic cells, e.g., bacterial cells. For example, modulating compounds may be administered to farm animals to improve their ability to withstand farming conditions longer.

Sirtuin-modulating compounds that increase the level and/or activity of a sirtuin protein may also be used to increase lifespan, stress resistance, and resistance to apoptosis in plants. In one embodiment, a compound is applied to plants, e.g., on a periodic basis, or to fungi. In another embodiment, plants are genetically modified to produce a compound. In another embodiment, plants and fruits are treated with a compound prior to picking and shipping to increase resistance to damage during shipping. Plant seeds may also be contacted with compounds described herein, e.g., to preserve them.

In other embodiments, sirtuin-modulating compounds that increase the level and/or activity of a sirtuin protein may be used for modulating lifespan in yeast cells. Situations in which it may be desirable to extend the lifespan of yeast cells include any process in which yeast is used, e.g., the making of beer, yogurt, and bakery items, e.g., bread. Use of yeast having an extended lifespan can result in using less yeast or in having the yeast be active for longer periods of time. Yeast or other mammalian cells used for recombinantly producing proteins may also be treated as described herein.

Sirtuin-modulating compounds that increase the level and/or activity of a sirtuin protein may also be used to increase lifespan, stress resistance and resistance to apoptosis in insects. In this embodiment, compounds would be applied to useful insects, e.g., bees and other insects that are involved in pollination of plants. In a specific embodiment, a compound would be applied to bees involved in the production of honey. Generally, the methods described herein may be applied to any organism, e.g., eukaryote, that may have commercial importance. For example, they can be applied to fish (aquaculture) and birds (e.g., chicken and fowl).

Higher doses of sirtuin-modulating compounds that increase the level and/or activity of a sirtuin protein may also be used as a pesticide by interfering with the regulation of silenced genes and the regulation of apoptosis during development. In this embodiment, a compound may be applied to plants using a method known in the art that ensures the compound is bio-available to insect larvae, and not to plants.

At least in view of the link between reproduction and longevity, sirtuin-modulating compounds that increase the level and/or activity of a sirtuin protein can be applied to affect the reproduction of organisms such as insects, animals and microorganisms.

**4. Assays**

Yet other methods contemplated herein include screening methods for identifying compounds or agents that

modulate sirtuins. An agent may be a nucleic acid, such as an aptamer. Assays may be conducted in a cell based or cell free format. For example, an assay may comprise incubating (or contacting) a sirtuin with a test agent under conditions in which a sirtuin can be modulated by an agent known to modulate the sirtuin, and monitoring or determining the level of modulation of the sirtuin in the presence of the test agent relative to the absence of the test agent. The level of modulation of a sirtuin can be determined by determining its ability to deacetylate a substrate. Exemplary substrates are acetylated peptides which can be obtained from BIOMOL (Plymouth Meeting, PA). Preferred substrates include peptides of p53, such as those comprising an acetylated K382. A particularly preferred substrate is the Fluor de Lys-SIRT1 (BIOMOL), i.e., the acetylated peptide Arg-His-Lys-Lys (SEQ ID NO:2). Other substrates are peptides from human histones H3 and H4 or an acetylated amino acid. Substrates may be fluorogenic. The sirtuin may be SIRT1, Sir2, SIRT3, or a portion thereof. For example, recombinant SIRT1 can be obtained from BIOMOL. The reaction may be conducted for about 30 minutes and stopped, e.g., with nicotinamide. The HDAC fluorescent activity assay/drug discovery kit (AK-500, BIOMOL Research Laboratories) may be used to determine the level of acetylation. Similar assays are described in Bitterman et al. ((2002) J. Biol. Chem. 277:45099). The level of modulation of the sirtuin in an assay may be compared to the level of modulation of the sirtuin in the presence of one or more (separately or simultaneously) compounds described herein, which may serve as positive or negative controls. Sirtuins for use in the assays may be full length sirtuin proteins or portions thereof. Since it has been shown herein that activating compounds appear to interact with the N-terminus of SIRT1, proteins for use in the assays include N-terminal portions of sirtuins, e.g., about amino acids 1-176 or 1-255 of SIRT1; about amino acids 1-174 or 1-252 of Sir2.

In one embodiment, a screening assay comprises (i) contacting a sirtuin with a test agent and an acetylated substrate under conditions appropriate for the sirtuin to deacetylate the substrate in the absence of the test agent; and (ii) determining the level of acetylation of the substrate, wherein a lower level of acetylation of the substrate in the presence of the test agent relative to the absence of the test agent indicates that the test agent stimulates deacetylation by the sirtuin, whereas a higher level of acetylation of the substrate in the presence of the test agent relative to the absence of the test agent indicates that the test agent inhibits deacetylation by the sirtuin.

Methods for identifying an agent that modulates, e.g., stimulates, sirtuins *in vivo* may comprise (i) contacting a cell with a test agent and a substrate that is capable of entering a cell in the presence of an inhibitor of class I and class II HDACs under conditions appropriate for the sirtuin to deacetylate the substrate in the absence of the test agent; and (ii) determining the level of acetylation of the substrate, wherein a lower level of acetylation of the substrate in the presence of the test agent relative to the absence of the test agent indicates that the test agent stimulates deacetylation by the sirtuin, whereas a higher level of acetylation of the substrate in the presence of the test agent relative to the absence of the test agent indicates that the test agent inhibits deacetylation by the sirtuin. A preferred substrate is an acetylated peptide, which is also preferably fluorogenic, as further described herein. The method may further comprise lysing the cells to determine the level of acetylation of the substrate. Substrates may be added to cells at a concentration ranging from about 1$\mu$M to about 10 mM, preferably from about 10 $\mu$M to 1 mM, even more preferably from about 100 $\mu$M to 1 mM, such as about 200 $\mu$M. A preferred substrate is an acetylated lysine, e.g., $\varepsilon$-acetyl lysine (Fluor de Lys, FdL) or Fluor de Lys-SIRT1. A preferred inhibitor of class I and class II HDACs is trichostatin A (TSA), which may be used at concentrations ranging from about 0.01 to 100 $\mu$M, preferably from about 0.1 to 10 $\mu$M, such as 1 $\mu$M. Incubation of cells with the test compound and the substrate may be conducted for about 10 minutes to 5 hours, preferably for about 1-3 hours. Since TSA inhibits all class I and class II HDACs, and that certain substrates, e.g., Fluor de Lys, is a poor substrate for SIRT2 and even less a substrate for SIRT3-7, such an assay may be used to identify modulators of SIRT1 *in vivo.*

### 5. Pharmaceutical Compositions

The sirtuin-modulating compounds described herein may be formulated in a conventional manner using one or more physiologically acceptable carriers or excipients. For example, sirtuin-modulating compounds and their physiologically acceptable salts and solvates may be formulated for administration by, for example, injection (e.g. SubQ, IM, IP), inhalation or insufflation (either through the mouth or the nose) or oral, buccal, sublingual, transdermal, nasal, parenteral or rectal administration. In one embodiment, a sirtuin-modulating compound may be administered locally, at the site where the target cells are present, i.e., in a specific tissue, organ, or fluid (e.g., blood, cerebrospinal fluid, etc.).

Sirtuin-modulating compounds can be formulated for a variety of modes of administration, including systemic and topical or localized administration. Techniques and formulations generally may be found in Remington's Pharmaceutical Sciences, Meade Publishing Co., Easton, PA. For parenteral administration, injection is preferred, including intramuscular, intravenous, intraperitoneal, and subcutaneous. For injection, the compounds can be formulated in liquid solutions, preferably in physiologically compatible buffers such as Hank's solution or Ringer's solution. In addition, the compounds may be formulated in solid form and redissolved or suspended immediately prior to use. Lyophilized forms are also included.

For oral administration, the pharmaceutical compositions may take the form of, for example, tablets, lozenges,

or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., ationd oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate. Preparations for oral administration may be suitably formulated to give controlled release of the active compound.

For administration by inhalation (e.g., pulmonary delivery), sirtuin-modulating compounds may be conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g., gelatin, for use in an inhaler or insufflator may be formulated containing a powder mix of the compound and a suitable powder base such as lactose or starch.

Sirtuin-modulating compounds may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

Sirtuin-modulating compounds may also be formulated in rectal compositions such as suppositories or retention enemas, e.g., containing conventional suppository bases such as cocoa butter or other glycerides.

In addition to the formulations described previously, sirtuin-modulating compounds may also be formulated as a depot preparation. Such long acting formulations may be administered by implantation (for example subcutaneously or intramuscularly) or by intramuscular injection. Thus, for example, sirtuin-modulating compounds may be formulated with suitable polymeric or hydrophobic materials (for example as an emulsion in an acceptable oil) or ion exchange resins, or as sparingly soluble derivatives, for example, as a sparingly soluble salt. Controlled release formula also includes patches.

In certain embodiments, the compounds described herein can be formulated for delivery to the central nervous system (CNS) (reviewed in Begley, Pharmacology & Therapeutics 104: 29-45 (2004)). Conventional approaches for drug delivery to the CNS include: neurosurgical strategies (e.g., intracerebral injection or intracerebroventricular infusion); molecular manipulation of the agent (e.g., production of a chimeric fusion protein that comprises a transport peptide that has an affinity for an endothelial cell surface molecule in combination with an agent that is itself incapable of crossing the BBB) in an attempt to exploit one of the endogenous transport pathways of the BBB; pharmacological strategies designed to increase the lipid solubility of an agent (e.g., conjugation of watersoluble agents to lipid or cholesterol carriers); and the transitory disruption of the integrity of the BBB by hyperosmotic disruption (resulting from the infusion of a mannitol solution into the carotid artery or the use of a biologically active agent such as an angiotensin peptide).

Liposomes are a further drug delivery system which is easily injectable. Accordingly, in the method of invention the active compounds can also be administered in the form of a liposome delivery system. Liposomes are well-known by a person skilled in the art. Liposomes can be formed from a variety of phospholipids, such as cholesterol, stearylamine of phosphatidylcholines. Liposomes being usable for the method of invention encompass all types of liposomes including, but not limited to, small unilamellar vesicles, large unilamellar vesicles and multilamellar vesicles.

Another way to produce a formulation, particularly a solution, of a sirtuin modulator such as resveratrol or a derivative thereof, is through the use of cyclodextrin. By cyclodextrin is meant $\alpha$-, $\beta$-, or $\gamma$-cyclodextrin. Cyclodextrins are described in detail in Pitha et al., U.S. Pat. No. 4,727,064, which is incorporated herein by reference. Cyclodextrins are cyclic oligomers of glucose; these compounds form inclusion complexes with any drug whose molecule can fit into the lipophile-seeking cavities of the cyclodextrin molecule.

Rapidly disintegrating or dissolving dosage forms are useful for the rapid absorption, particularly buccal and sublingual absorption, of pharmaceutically active agents. Fast melt dosage forms are beneficial to patients, such as aged and pediatric patients, who have difficulty in swallowing typical solid dosage forms, such as caplets and tablets. Additionally, fast melt dosage forms circumvent drawbacks associated with, for example, chewable dosage forms, wherein the length of time an active agent remains in a patient's mouth plays an important role in determining the amount of taste masking and the extent to which a patient may object to throat grittiness of the active agent.

Pharmaceutical compositions (including cosmetic preparations) may comprise from about 0.00001 to 100%

such as from 0.001 to 10% or from 0.1% to 5% by weight of one or more sirtuin-modulating compounds described herein.

In one embodiment, a sirtuin-modulating compound described herein, is incorporated into a topical formulation containing a topical carrier that is generally suited to topical drug administration and comprising any such material known in the art. The topical carrier may be selected so as to provide the composition in the desired form, e.g., as an ointment, lotion, cream, microemulsion, gel, oil, solution, or the like, and may be comprised of a material of either naturally occurring or synthetic origin. It is preferable that the selected carrier not adversely affect the active agent or other components of the topical formulation. Examples of suitable topical carriers for use herein include water, alcohols and other nontoxic organic solvents, glycerin, mineral oil, silicone, petroleum jelly, lanolin, fatty acids, vegetable oils, parabens, waxes, and the like.

Formulations may be colorless, odorless ointments, lotions, creams, microemulsions and gels.

Sirtuin-modulating compounds may be incorporated into ointments, which generally are semisolid preparations which are typically based on petrolatum or other petroleum derivatives. The specific ointment base to be used, as will be appreciated by those skilled in the art, is one that will provide for optimum drug delivery, and, preferably, will provide for other desired characteristics as well, e.g., emolliency or the like. As with other carriers or vehicles, an ointment base should be inert, stable, nonirritating and nonsensitizing.

Sirtuin-modulating compounds may be incorporated into lotions, which generally are preparations to be applied to the skin surface without friction, and are typically liquid or semiliquid preparations in which solid particles, including the active agent, are present in a water or alcohol base. Lotions are usually suspensions of solids, and may comprise a liquid oily emulsion of the oil-in-water type.

Sirtuin-modulating compounds may be incorporated into creams, which generally are viscous liquid or semisolid emulsions, either oil-in-water or water-in-oil. Cream bases are water-washable, and contain an oil phase, an emulsifier and an aqueous phase. The oil phase is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation, as explained in Remington's, *supra*, is generally a nonionic, anionic, cationic or amphoteric surfactant.

Sirtuin-modulating compounds may be incorporated into microemulsions, which generally are thermodynamically stable, isotropically clear dispersions of two immiscible liquids, such as oil and water, stabilized by an interfacial film of surfactant molecules (Encyclopedia of Pharmaceutical Technology (New York: Marcel Dekker, 1992), volume 9).

Sirtuin-modulating compounds may be incorporated into gel formulations, which generally are semisolid systems consisting of either suspensions made up of small inorganic particles (two-phase systems) or large organic molecules distributed substantially uniformly throughout a carrier liquid (single phase gels). Although gels commonly employ aqueous carrier liquid, alcohols and oils can be used as the carrier liquid as well.

Other active agents may also be included in formulations, e.g., other antiinflammatory agents, analgesics, antimicrobial agents, antifungal agents, antibiotics, vitamins, antioxidants, and sunblock agents commonly found in sunscreen formulations including, but not limited to, anthranilates, benzophenones (particularly benzophenone-3), camphor derivatives, cinnamates (e.g., octyl methoxycinnamate), dibenzoyl methanes (e.g., butyl methoxydibenzoyl methane), p-aminobenzoic acid (PABA) and derivatives thereof, and salicylates (e.g., octyl salicylate).

In certain topical formulations, the active agent is present in an amount in the range of approximately 0.25 wt. % to 75 wt. % of the formulation, preferably in the range of approximately 0.25 wt. % to 30 wt. % of the formulation, more preferably in the range of approximately 0.5 wt. % to 15 wt. % of the formulation, and most preferably in the range of approximately 1.0 wt. % to 10 wt. % of the formulation.

Conditions of the eye can be treated or prevented by, e.g., systemic, topical, intraocular injection of a sirtuin-modulating compound, or by insertion of a sustained release device that releases a sirtuin-modulating compound. A sirtuin-modulating compound that increases the level and/or activity of a sirtuin protein may be delivered in a pharmaceutically acceptable ophthalmic vehicle, such that the compound is maintained in contact with the ocular surface for a sufficient time period to allow the compound to penetrate the corneal and internal regions of the eye, as for example the anterior chamber, posterior chamber, vitreous body, aqueous humor, vitreous humor, cornea, iris/ciliary, lens, choroid/retina and sclera. The pharmaceutically-acceptable ophthalmic vehicle may, for example, be an ointment, vegetable oil or an encapsulating material. Alternatively, the compounds of the invention may be injected directly into the vitreous and aqueous humour. In a further alternative, the compounds may be administered systemically, such as by intravenous infusion or injection, for treatment of the eye.

Sirtuin-modulating compounds described herein may be stored in oxygen free environment. For example, resveratrol or analog thereof can be prepared in an airtight capsule for oral administration, such as Capsugel from Pfizer, Inc.

Cells, e.g., treated *ex vivo* with a sirtuin-modulating compound, can be administered according to methods for administering a graft to a subject, which may be accompanied, e.g., by administration of an immunosuppressant drug, e.g., cyclosporin A. For general principles in medicinal formulation, the reader is referred to Cell Therapy: Stem Cell Transplantation, Gene Therapy, and Cellular Immunotherapy, by G. Morstyn & W. Sheridan eds, Cambridge University

Press, 1996; and Hematopoietic Stem Cell Therapy, E. D. Ball, J. Lister & P. Law, Churchill Livingstone, 2000.

Toxicity and therapeutic efficacy of sirtuin-modulating compounds can be determined by standard pharmaceutical procedures in cell cultures or experimental animals. The $LD_{50}$ is the dose lethal to 50% of the population. The $ED_{50}$ is the dose therapeutically effective in 50% of the population. The dose ratio between toxic and therapeutic effects ($LD_{50}/ED_{50}$) is the therapeutic index. Sirtuin-modulating compounds that exhibit large therapeutic indexes are preferred. While sirtuin-modulating compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds may lie within a range of circulating concentrations that include the $ED_{50}$ with little or no toxicity. The dosage may vary within this range depending upon the dosage form employed and the route of administration utilized. For any compound, the therapeutically effective dose can be estimated initially from cell culture assays. A dose may be formulated in animal models to achieve a circulating plasma concentration range that includes the $IC_{50}$ (i.e., the concentration of the test compound that achieves a half-maximal inhibition of symptoms) as determined in cell culture. Such information can be used to more accurately determine useful doses in humans. Levels in plasma may be measured, for example, by high performance liquid chromatography.

**6. Kits**

Also provided herein are kits, e.g., kits for therapeutic purposes or kits for modulating the lifespan of cells or modulating apoptosis. A kit may comprise one or more sirtuin-modulating compounds, e.g., in premeasured doses. A kit may optionally comprise devices for contacting cells with the compounds and instructions for use. Devices include syringes, stents and other devices for introducing a sirtuin-modulating compound into a subject (e.g., the blood vessel of a subject) or applying it to the skin of a subject.

In yet another embodiment, the invention provides a composition of matter comprising a sirtruin modulator of this invention and another therapeutic agent (the same ones used in combination therapies and combination compositions) in separate dosage forms, but associated with one another. The term "associated with one another" as used herein means that the separate dosage forms are packaged together or otherwise attached to one another such that it is readily apparent that the separate dosage forms are intended to be sold and administered as part of the same regimen. The agent and the sirtruin modulator are preferably packaged together in a blister pack or other multi-chamber package, or as connected, separately sealed containers (such as foil pouches or the like) that can be separated by the user (e.g., by tearing on score lines between the two containers).

In still another embodiment, the invention provides a kit comprising in separate vessels, a) a sirtruin modulator of this invention; and b) another another therapeutic agent such as those described elsewhere in the specification.

The practice of the present methods will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Patent No: 4,683,195; Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986).

**Examples**

The invention now being generally described, it will be more readily understood by reference to the following examples which are included merely for purposes of illustration of certain aspects and embodiments of the present invention, and are not intended to limit the invention in any way.

**Example 1** ***Materials and Methods***

All peptides were prepared by BioPeptide (San Diego, CA) and shown to be at least 95% pure by analytical HPLC analysis. Bovine glutamate dehydrogenase (GDH), $\alpha$-ketoglutarate, NADH and $NAD^+$ were from Sigma Chemical

Company. Full-length, human SIRT1 and the truncated version used for biophysical studies SIRT1(183-664) were prepared as described previously, and shown to have equivalent kinetic properties (Milne et al. (2007) Nature 450, 712-716). The nicotinamidase PNC1 was prepared as described by Denu and colleagues (Smith et al. (2009) Anal Biochem 394, 101-109).

**Example 2** ***Structures and Synthesis of STACs***

Structures of compounds 1 - 20, which are used in the correlation of Figure 5, appear in the compound table, while structures of 21 - 26 appear in Table 1 and Figures 9 and 11. Experimental procedures and characterization data for 10, 11, and 12 can be found herein. SRT1460, SRT1720, SRT2183 were prepared according to literature procedures (Milne et al. (2007) Nature 450, 712-716), as were 3, 23, and 24 (Vu et al. (2009) J Med Chem). Compound 25 was prepared according to the procedures described for structurally similar compounds (Vu et al. (2009) J Med Chem). Compounds 3 (Nunes et al. (2007). Int. Pat. Number WO 2007/019346), 4 (Bemis et. al. (2008). Int. Pat. Number WO 2008/156866), 26, 13, and 14 (Vu et al. (2010). Int. Pat. Number WO 2010/003048) were prepared according to the procedures described in the relevant patents. The synthesis of 7 is described herein.

**Example 3** ***Isothermal Titration Calorimetry Experiments***

ITC experiments were performed using either a VP-ITC system or iTC200 system (MicroCal) at 26 °C in a buffer composed of 137 mM NaCl, 2.7 mM KCl, 1 mM MgCl2, 2 mM TCEP, 5% (v/v) glycerol and 50 mM HEPES-NaOH, pH 7.3. SIRT1(183-664) was purified and dialyzed against the buffer, centrifuged, and degassed before the experiment. The peptide and compounds were dissolved in the final dialysis buffer and their pH values were adjusted to match that of the protein solution. In a typical binding experiment to characterize the STAC/TAMRA-peptide interaction performed on the VP-ITC system, 1 mM TAMRA-peptide was injected in 35 aliquots of 8 μL (except the first injection, which was 2 μL) into a 1.47 ml sample cell containing 100 μM STAC. To characterize this interaction using the iTC200 system, 1 mM TAMRA-peptide was injected in 20 aliquots of 2 μL (except the first injection, which was 0.5 μL) into a 0.202 mL sample cell containing 100 μM STAC. For STACs with poor solubility, the concentrations were adjusted accordingly. To characterize the interaction of SRT1460 with the TAMRA-peptide, 5 mM TAMRA-peptide and 0.5 μM SRT1460 were used to obtain better quality data.

To characterize the interaction of a STAC with SIRT1(183-664) on the VP-ITC system, 100 μM enzyme was injected in 35 aliquots of 8 μL (except the first injection, which was 2 μL) into a 1.47 mL sample cell containing 10 μM STAC. In a typical binding experiment on the iTC200 system, 100 μM enzyme was injected in 20 aliquots of 2 μL (except the first injection, which was 0.5 μL) into a 0.202 mL sample cell containing 100 μM STAC.

In all cases, data was corrected for the heat of dilution and fitted using a nonlinear least-squares routine using a single-site binding model with Origin for ITC v7.0383 (Microcal) with the stoichiometry (n), the enthalpy of the reaction (ΔH) and the association constant ($K_a$) calculated.

Results

In the course of our studies of SIRT1 activation, we found that several STACs bind to TAMRA-peptide to form an activator:substrate complex. To explore the mechanistic significance of this complex, Kd values for the binding of STACs to TAMRA-peptide were determined, using ITC, for 20 activators that ranged in potency from $EC_{1.5}$ = 0.05 to ≥ 100 μM (see Table 6 for the structures of these compounds). Figure 4 contains representative ITC titrations. Shown in Figure 4A is the titration for 4, which exhibits no detectable binding to TAMRA-peptide. Under these experimental conditions, if this compound binds to TAMRA-peptide, it does so with a $K_d$ value that can be conservatively estimated to be greater than 100 μM. Figure 4B is the titration for 7, and indicates binding to the TAMRA-peptide with a $K_d$ of 36 μM. This Kd value is 72-fold higher than the $K_x$ value of 0.5 μM (see inset of Figure 4B for the activation titration curve for 7). Such a discrepancy between $K_d$ and $K_x$ suggests that activation by 7 does not depend on its binding to substrate.

The mechanism of Figure 2A predicts that $EC_{1.5}$ values should be dependent on $K_d$ values, as indicated by the simulations of Figure 3. To investigate this, we plotted $EC_{1.5}$ as a function of $K_d$ (Figure 5). As seen in this plot, there is no correlation between activation efficacy and the affinity of STACs for the TAMRA-peptide. Highlighted in gray, is a series of compounds with $K_d$ values that range from 2.5 to greater than 100 μM, but have the same $EC_{1.5}$ value of around 0.3 μM. This clearly indicates that the ability of STACs to activate the SIRT1-catalyzed deacetylation of the TAMRA-peptide is unrelated to the affinity of STACs for this substrate.

One can speculate that perhaps for a certain structural class of STAC, a correlation might exist between $EC_{1.5}$ and $K_d$. Such a correlation would define a line connecting 10 and 20 in Figure 5. Examination of the structures of the specific compounds on this line reveals that they represent all three structural classes comprising the compounds of Figure 5. Thus, there is no correlation between $EC_{1.5}$ and $K_d$ even if we try to parse a potential correlation according to

STAC structural class.

Binding of activators to SIRT1 is inconsistent with substrate enhancement, but consistent with enzyme allostery. Among the approximately 20 compounds tested (see Table 6), the interaction of SIRT1(183-664) with three STACs could be detected using ITC. We found that 4, 13, and 14 bind with $K_x$ values equal to 3.0, 0.32, and 0.43 $\mu$M, respectively (see Table 1 for summary and structures). As representative, the experimental data for 14 is shown in Figure 8A. For this compound, we were also able to perform a fluorescence binding experiment. We observed that this compound's emission fluorescence at 450 nm decreased with increasing concentrations of SIRT1 (Figure 8B). Quantification of these data allowed calculation of a $K_x$ value of 0.27 $\mu$M (Figure 8C), in agreement with the ITC measurement.

Table 1 Dissociation Constants for SRT:SIRT1 Complexes[a]

| | SIRT1 Activator | | |
|---|---|---|---|
| | **4** 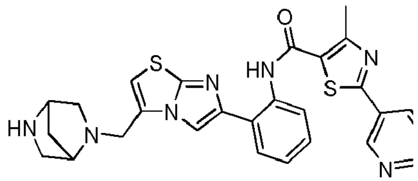 | **13** 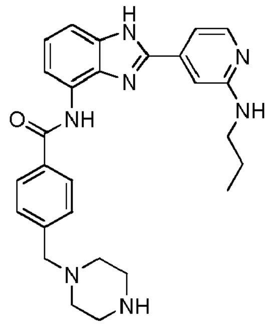 | **14** 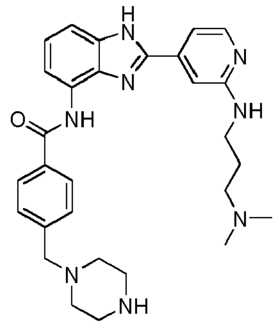 |
| $K_x$ (μM), ITC | 3.0 | 0.43 | 0.32 |
| $K_x$ (μM), Fluorescence | - | - | 0.27 |

(a) See herein for description of reaction conditions and description of experimental protocol.

Note that the mechanism of Figure 2A excludes the possibility of the formation of a complex between SIRT1 and activator. In contrast, the allosteric mechanism of Figure 2B includes, but does not require, the formation of such a complex. Only 3 out of the 20 STACs tested can bind to SIRT1 is thus consistent with this mechanism.

Table 2 summarizes $K_d$ values for the binding of STACs to Ac-Arg-His-Lys-Lys$^{Ac}$-X. Note that except for SRT1460, the solubility of the compounds required that these ITC experiments be done in solutions containing 10% DMSO. Given that the interactions of these STACs with Ac-Arg-His-Lys-Lys$^{Ac}$-Phe-$NH_2$ and Ac-Arg-His-Lys-Lys$^{Ac}$-Trp-$NH_2$ are with the phenyl and indole moieties of the C-terminal amino acid and thus, are likely driven by hydrophobic effects, these $K_d$ values may larger than those in solutions of 1% DMSO, the DMSO concentration at which activation assays are run.

Table 2 $K_d$ Values for the Binding STACs to Ac-Arg-His-Lys-Lys$^{Ac}$-X$^a$

| | $K_d$ ($\mu$M) | | | |
|---|---|---|---|---|
| | SRT1460 | SRT1720 | SRT2183 | 10 |
| AMC | > 500 | > 500 | > 50 | > 500 |
| $NH_2$ | > 500 | > 500 | > 50 | > 500 |
| Phe$NH_2$ | > 500 | > 500 | > 50 | 190 |
| Trp$NH_2$ | > 500 | 150 | > 50 | 25 |

(a) $K_d$ determinations for SRT1720, SRT2183, and 10 were done in buffer containing 10% DMSO. For SRT1460, no cosolvent was necessary.

For the amide and AMC derivative there is no detectable interaction with any of the four STACs. Significantly, 10 activates the deacetylation of the AMC derivative. Combined with the absence of binding of 10 to this substrate, activation by substrate enhancement can be ruled out.

For X = Phe$NH_2$, there is no binding to SRT1460, yet there is activation of the substrate's deacetylation. In contrast, while 10 does bind to this substrate, it does not activate its deacetylation. Finally for X = Trp$NH_2$, SRT1720 binds but does not activate, while 10 both binds and activates.

As with activation of the deacetylation of TAMRA-peptide, there is no correlation between the ability of a STAC to activate and its affinity for substrate.

**Example 4** ***Fluorescence Binding Experiments for the Interaction of 14 with SIRT1***

SIRT1(183-664) was dialyzed against a buffer composed of 137 mM NaCl, 2.7 mM KCl, 1 mM MgCl2, 2 mM TCEP, 5% (v/v) glycerol and 50 mM HEPES-NaOH, pH 7.3. 14 was dissolved in the final dialysis buffer and its pH values were adjusted to match that of the protein solution. The fluorescence emission spectra of 14 in the absence or presence of different concentrations of enzyme was monitored on the SpectraMax M5 with excitation at 315 nm. The fluorescence intensity change at 450 nm was plotted against the concentration of SIRT1(183-664) and fitted using a standard binding equation.

**Example 5** ***Screening of Sirtris Compound Collection Using desTAMRA-Peptide***

Our collection of approximately 5,000 SIRT1 activators was screened using the BioTrove mass spectrometry system, as previously described (Milne et al. (2007) Nature 450, 712-716). In this screen, $[SIRT1]_o$ = 5 nM, $[desTAMRA\text{-}peptide]_o$ = 1 $\mu$M = $K_m$/10, and $[NAD^+]_o$ = 30 $\mu$M = $K_m$/10. Compounds were present at 10 $\mu$M, and the assays were conducted in a pH 7.4 buffer containing 150 mM Tris, 5 mM DTT, 1% DMSO, and 0.025% BSA.

Results

Sirtris' initial activator lead structures were identified in a high-throughput screening campaign, which used a fluorescence-polarization assay, with TAMRA-peptide as substrate (Milne et al. (2007) Nature 450, 712-716). This 20mer is centered around Lys$^{382}$ of p53, a natural substrate for SIRT1. The modifications of the sequence of TAMRA-peptide relative to p53 were made specifically for purposes of the assay (Milne et al. (2007) Nature 450, 712-716; Marcotte et al. (2004) Anal Biochem 332, 90-99). Characterization of the initial screening hits, and all subsequent studies, have been conducted using TAMRA-peptide and a mass spectroscopy-based assay (Milne et al. (2007) Nature 450, 712-716).

As the structure-activity relationship around these initial activator lead compounds evolved, it became of interest to see if these compounds could activate the deacetylation of the TAMRA-peptide analog that lacked the TAMRA moiety.

Of the several STACs that we initially tested, including those reported by Milne et al. ((2007) Nature 450, 712-716), none were able to activate the deacetylation of the desTAMRA-peptide. These results are consistent with those recently reported by Pacholec et al. ((2010) J. Biol. Chem. 285, 8340-8351), and indicate that activation of SIRT1 by STACs is strongly dependent on structural features of the substrate. However, while Pacholec et al. ((2010) J. Biol. Chem. 285, 8340-8351) and others (Kaeberlein et al. (2005) J. Biol. Chem. 280, 17038-17045; Beher et. al (2009) Chem Biol Drug Des) have interpreted the dependence of activation on substrate structure as reflecting a substrate-associated artifact, and an 'indirect' mechanism for SIRT1 activation, we believe these results reveal important features of SIRT1's allosteric regulation.

Among the several STACs tested, none activated the deacetylation of the desTAMRA-peptide. To test the generality of this finding, we screened our collection of over 5,000 STACs for their ability to activate the deacetylation of this peptide. The result of this screen was the identification of three structurally-related STACs: 10, 11, and 12. Activation titration curves for the three compounds are show in Figure 6, where it can be seen that all three activate the SIRT1-catalyzed deacetylation of desTAMRA-peptide with EC50 and RVmax values of around 2 $\mu$M and 2.3, respectively. These results demonstrate that a peptide lacking the TAMRA moiety can still support activation by STACs.

While the desTAMRA-peptide still supports activation, there remains a biotin moiety on the N-terminal side of the $Lys^{Ac}$. This begs the question as to whether the biotin alone can support activation. To answer this question, we examined the effect of 10, 11, and 12 on the des(biotin,TAMRA)-peptide, and found that none activate the deacetylation of this substrate , suggesting that a chemical moiety having some measure of steric bulk needs to be present on peptide substrates for their deacetylation to be activated by STACs.

**Example 6** ***Kinetic Studies of Interaction of STACs with SIRT1***

To determine the interaction of STACs with SIRT1 two kinetic methods were employed. One, which was used for TAMRA-peptide, desTAMRA-peptide, des(biotin,TAMRA)-peptide, and p53-20mer, used a previously described mass spectroscopic method for detection of peptide reaction products (Milne et al. (2007) Nature 450, 712-716). The other, used with peptides of general structure Ac-Arg-His-Lys-$Lys^{Ac}$-X, used a continuous, enzyme-coupled method reported previously (Smith et al. (2009) Anal Biochem 394, 101-109), in which the SRT1 product nicotinamide is first converted into nicotinic acid and ammonia by the action of PNC1, and the ammonia then used by GDH to convert $\alpha$-ketoglutarate into glutamate. This reaction occurs with oxidation of NADH to $NAD^+$, which is accompanied by a change in absorbance at 340 nm ($\Delta\varepsilon_{340}$ =-6,200 $M^{-1}$ $cm^{-1}$).

The kinetic experiments used to characterize activators were run on a Perkin Elmer Lambda 25 spectrophotometer equipped with a water-jacketed, eight-cell changer maintained at 25°C. In a typical kinetic experiment, all components of the reaction solution except SIRT1 were added to a 1 mL cuvette and allowed to reach thermal equilibrium. Final concentrations of the coupling system components were: 1 $\mu$M PNC1, 0.23 mM NADH, 3.4 mM $\alpha$-ketoglutarate, and 20 U/mL bovine GDH. This solution also contained peptide substrate, $NAD^{.0.}$, and activator, at concentrations indicated in the text. The final DMSO concentration was kept constant at 1%, and the buffer used in these experiments was 50 mM HEPES, 150 mM NaCl, pH 7.5. While the system was reaching thermal equilibrium, the absorbance was monitored to provide an accurate estimate of the background rate, which was subtracted from the reaction rate after the addition of SIRT1.

Results

Table 3 summarizes steady-state kinetic parameters for the substrates used in this study. The parameters reported in this table were all determined using the PNC1-GDH-coupled assay. Kinetic parameters for the four 20mers were also estimated using the mass spectroscopy-based assay first described by Milne et al. ((2007) Nature 450, 712-716) and agree with those reported in Table 3.

Table 3 Summary of Steady-State Kinetic Parameters for SIRT1 Substrates[a,b,c]

| Substrate | | $k_c$ ($sec^{-1}$) | Km ($\mu$M) | $k_c/K_m$ ($sec^{-1}$ $mM^{-1}$) |
|---|---|---|---|---|
| TAMRA-peptide EEK$^B$GQSTSSH**K**$^{Ac}$JSTEGK$^T$EE | | 0.036 | 8.3 | 4.3 |
| desTAMRA-peptide EEK$^B$GQSTSSH**K**$^{Ac}$JSTEGKEE | | 0.038 | 33 | 1.2 |
| des(Biotin,TAMRA)-peptide EEKGQSTSSH**K**$^{Ac}$JSTEGKEE | | 0.044 | 40 | 1.1 |
| p53-20mer | SKKGQSTSRHK**K**$^{Ac}$LJKTEGP | 0.36 | 8.5 | 42 |

(continued)

| Substrate | $k_c$ ($sec^{-1}$) | Km ($\mu M$) | $k_c/K_m$ ($sec^{-1}$ $mM^{-1}$) |
|---|---|---|---|
| RHK**K**$^{Ac}$-AMC | 0.091 | 110 | 0.8 |
| RHK**K**$^{Ac}$ | 0.14 | 23 | 6.0 |
| RHK**K**$^{Ac}$A | 0.15 | 13 | 12 |
| RHK**K**$^{Ac}$F | 0.16 | 7.1 | 23 |
| RHK**K**$^{Ac}$W | 0.19 | 4.0 | 48 |

(a) $K^B$ is biotinylated lysine, $K^T$ is lysine labeled with a TAMRA group, and J is norleucine. All peptides are blocked at the C-terminus with an acetyl and at the N-terminus with an amide.
(b) Experiments were conducted at 25°C, in a pH 7.5 buffer containing 50 mM HEPES and 150 mM NaCl. $[NAD^+]_o$ = 2 mM and $[SIRT1]_o$ varied from 50 - 1,000 nM, depending on reactivity of the substrate.
(c) Each parameter estimate is based on 2 or 3 independent experiments. Standard deviations (n=3) or deviation from the mean (n=2) is less than 15% in all cases.

Motivated by the above studies that document a strong influence of substrate structure on how STACs interact with SIRT1, investigated how STACs effect the deacetylation of Ac-Arg-His-Lys-Lys$^{Ac}$-X, where X is $NH_2$, AMC, Phe$NH_2$, and Trp$NH_2$. Ac-Arg-His-Lys-Lys$^{Ac}$-AMC (a.k.a. Fluor-de-Lys®, Biomol) is the substrate used in the assay that identified and characterized resveratrol (Howitz et al. (2003) Nature 425, 191-196), and is widely used in screens for activators and inhibitors of SIRT1.

Table 4 summarizes the effect of single concentrations of 22 and the three STACs published in Milne et al. ((2007) Nature 450, 712-716) on the deacetylation of the five substrates. Similar to what we observed with the four 20mer's, the effect that a particular STAC has on deacetylation is dependent on the structure of the substrate. For example, while SRT1460 activates the deacetylation of Ac-Arg-His-Lys-Lys$^{Ac}$-Phe-$NH_2$ and Ac-Arg-His-Lys-Lys$^{Ac}$-Trp-$NH_2$, it inhibits deacetylation of Ac-Arg-His-Lys-Lys$^{Ac}$-AMC, and while 22 activates deacetylation of Ac-Arg-His-Lys-Lys$^{Ac}$-AMC and Ac-Arg-His-Lys-Lys$^{Ac}$-Trp-$NH_2$, it inhibits deacetylation of Ac-Arg-His-Lys-Lys$^{Ac}$-$NH_2$.

Table 4 Summary of the Effect of STACs on the SIRT1-Catalyzed Deacetylation of Ac-Arg-His-Lys-Lys$^{Ac}$-X[a]

| | Relative Velocity | | | |
|---|---|---|---|---|
| | SRT1460 | SRT1720 | SRT2183 | 10 |
| AMC | 0.6 | 1.0 | 1.1 | 1.9 |
| $NH_2$ | 0.8 | 1.1 | 1.2 | 0.6 |
| Ala-$NH_2$ | 1.1 | 0.9 | 0.8 | 0.3 |
| Phe-$NH_2$ | 1.4 | 0.9 | 1.0 | 1.1 |
| Trp-$NH_2$ | 1.6 | 1.0 | 1.0 | 2.8 |

(a) $[SRT]_o$ = 5 $\mu$M, $[peptide]_o \leq K_m$, $[NAD^+]$ = 70 $\mu$M. Each entry represents the average of 2-3 independent experiments; std. dev. ≤ 15%.

Several of these observations were followed-up with full titration curves. In Figure 10A, we see that 22 activates the deacetylation of Ac-Arg-His-Lys-Lys$^{Ac}$-AMC and Ac-Arg-His-Lys-Lys$^{Ac}$-Trp-$NH_2$, in a dose dependent manner. Likewise in Figure 10B, SRT1460 dose-dependently activates the deacetylation of Ac-Arg-His-Lys-Lys$^{Ac}$-Phe-$NH_2$ and Ac-Arg-His-Lys-Lys$^{Ac}$-Trp-$NH_2$. These result are highly significant, demonstrating that STACs can accelerate the deacetylation of peptide substrates comprising only natural amino acids.

**Example 7** ***General Treatment of Activation Data***

Activator titrations were plotted as relative velocity (i.e., the ratio of $v_x$, velocity in the presence of activator X, to $v_o$, velocity in the absence of activator) vs. $[X]_o$, initial concentration of activator, and fit by nonlinear least-squares to

the mechanism-independent expression for enzyme activation of eq (1):

$$\frac{v_x}{v_o} = 1 + \frac{RV_{max} - 1}{1 + \frac{EC_{50}}{[X]_o}} \qquad (1)$$

where $RV_{max}$ is the maximum relative velocity (i.e., $v_x/v_o$ at infinite $[X]_o$) and $EC_{50}$ is the activator concentration at which $v_x/v_o = (RV_{max}-1)/2$. $EC_{50}$ is a function of the kinetic mechanisms of both catalysis and activation, and the concentrations of substrates.

For activators whose insolubility and potency prohibited achieving high enough concentrations for accurate estimates of $RV_{max}$ and $EC_{50}$, activator potency can be expressed as $EC_{1.5}$, which is the concentration of activator needed to achieve 1.5-fold activation. $EC_{1.5}$ can be shown to equal:

$$EC_{1.5} = \frac{EC_{50}}{2RV_{max} - 3} \qquad (2)$$

and thus is a reflection of activator efficacy, with a dependence on both $RV_{max}$ and $EC_{50}$. For a series of activators in which $RV_{max}$ is roughly the same, the ratio $EC_{1.5}/EC_{50}$ will be similar for all members of the series, and thus, either $EC_{1.5}$ $EC_{50}$ can be used as the measure of activator potency.

The reciprocal of $EC_{1.5}$ is a direct measure of activator efficacy and equals:

$$(EC_{1.5})^{-1} = 2\left(\frac{RV_{max} - 1.5}{EC_{50}}\right) \qquad (3)$$

It can be seen that $(EC_{1.5})^{-1}$ is analogous to $V_{max}/K_m$ of standard steady-state enzyme kinetics.

**Example 8** ***Kinetic Model for Enzyme Activation by Formation of an Activator: Substrate Complex***

The rate law for the mechanism of Figure 2A can be derived starting with the expression of eq (4), which was derived using the rapid equilibrium assumption.

$$\frac{v_x}{[E]_o} = \frac{k_c[S]}{K_m\left(1 + \frac{[XS]}{K_{m,x}}\right) + [S]} + \frac{\gamma k_c[XS]}{K_{m,x}\left(1 + \frac{[S]}{K_m}\right) + [XS]} \qquad (4)$$

Note that the substrate concentration term corresponds to free substrate, rather than total substrate. This is so because in this mechanism X:S can account for a significant portion of total substrate, depending on $[S]_o$, $[X]_o$, and $K_d$.

To express the rate law in terms of total and free substrate, eq (4) can be recast as show in eq (5).

$$\frac{v_x}{[E]_o} = \frac{k_c[S]}{K_m\left(1 + \frac{[S]_o - [S]}{K_{m,x}}\right) + [S]} + \frac{\gamma k_c([S]_o - [S])}{K_{m,x}\left(1 + \frac{[S]}{K_m}\right) + [S]_o - [S]} \qquad (5)$$

This derivation will be complete if we can express [S] in terms of $[S]_o$, $[X]_o$, and $K_d$. This starts with the definition of $K_d$, given in eq (6):

$$K_d = \frac{[X][S]}{[XS]} = \frac{\{[X]_o - [S]_o + [S]\}[S]}{[S]_o - [S]} \quad (6)$$

Eq (6) can of course be expressed as the quadratic equation:

$$[S]^2 + (K_d + [X]_o - [S]_o)[S] + K_d[S]_o = 0 \quad (7)$$

which has the solution:

$$[S] = \frac{-(K_d + [X]_o - [S]_o) + \sqrt{(K_d + [X]_o - [S]_o)^2 + 4K_d[S]_o}}{2} \quad (8)$$

Results

The central feature of the 'indirect' mechanism of SIRT1 activation is X:S, the complex of activator and substrate (see Figure 2A). Examination of this mechanism indicates that activation is driven by formation X:S, meaning that X:S complexes with lower $K_d$ values should result in more potent activation. Thus, we should anticipate, for the mechanism of Figure 2A, a positive correlation between $EC_{50}$ and $K_d$. To test this, we simulated activator titration curves using the expression for free substrate of eq (8) and a modified form eq (5), in which both sides of the equation are divided by the control velocity, $v_o/[E]_o = k_e[S]_o/(K_m+[S]_o)$, to afford an expression for relative velocity, $v_x/v_o$.

These curves are shown in Figure 3 and were generated with the following parameter assignments: $K_m$ was assigned a value 5 μM, the $K_m$ for the TAMRA-peptide; γ was assigned a value of unity, since activation by STACs has been observed primarily to be a result of $K_m$ lowering, with little or no change in $k_c$; and $K_{m,x}$ was assigned a value of 0.25 μM, a value 20-fold less than $K_m$, to again give weight to the experimental observation that SIRT1-activation by STACs results primarily from a lowering $K_m$. $[S]_o = K_m/10 = 0.5$ μM, the concentration used in the work of both Milne et al. ((2007) Nature 450, 712-716) and Pacholec et al. (2010) J. Biol. Chem. 285, 8340-8351). Finally, $K_d$ was varied from 3 to 300 μM, which encompasses the range of values that have been measured for the interaction of STACs with TAMRA-peptide. As suspected for this mechanism, the simulations indicate that $EC_{50}$ correlates positively with $K_d$.

**Example 9** ***Kinetic Model for Enzyme Activation Through an Allosteric Mechanism***

The rate law for the mechanism of Figure 2B can be derived using the rapid equilibrium assumption and is given in eq (9).

$$\frac{v_x}{v_o} = \frac{1}{1 + \frac{[X]_o}{\beta K_{x,obs}}} + \frac{\gamma_{obs}}{1 + \frac{\beta K_{x,obs}}{[X]_o}} \quad (9)$$

where

$$K_{x,obs} = K_x \left( \frac{1 + \frac{K_s}{[S]_o}}{1 + \frac{\beta K_s}{[S]_o}} \right) \quad (10)$$

$$\gamma_{obs} = \gamma\left(\frac{1+\dfrac{K_s}{[S]_o}}{1+\dfrac{\beta K_s}{[S]_o}}\right) \quad (11)$$

Results

To determine if susbtrate enhancement can account for the activation of the SIRT1 catalyzed deacetylation of TAMRA peptide by 10 or SRT1460, the veracity of the indirect mechanism of Figure 2A,was assessed. Compounds 10 and SRT1460 were chosen, because they represent the two extremes of substrate affinity of the STACs of this study, with $K_d$ values of 2.5 and 140 μM, respectively.

Figure 6 shows the titration curve for the activation of SIRT1 by 10. When the data set was fit to eq (5), with $[S]_o$ set to the experimental value of 0.5 μM and $K_m$ constrained to 5 μM, the best fit estimates for the remaining parameters were calculated to be: $K_d$ = 53 ± 8 μM, $K_{m,x}$ = 0.035 ± 0.011 μM, and γ = 0.69 ± 0.08. What is immediately obvious is that the best-fit value of $K_d$ is 20-fold higher than the experimentally measured $K_d$ value of 2.5 μM. When $K_d$ was constrained to 2.5 μM, the nonlinear least squares fit did not converge, indicating that there are no values of $K_{m,x}$ and γ that can explain data for a STAC with $K_d$ = 2.5 μM.

The other extreme of $K_d$ for the STACs of this study occurs with SRT1460, which has a $K_d$ value of 140 μM. To determine if the mechanism of Figure 2A could accommodate such an activator, we fit the activation titration curve reported in Milne et al. ((2007) Nature 450, 712-716) to the expression of eq (5). This curve is reproduced in Figure 7A. In contrast to the titration curve for 10, these data could be fit to the rate law for the mechanism of Figure 2A. In this fitting, the following constraints were used: $K_d$ = 140 μM, the experimentally determined value; $[S]_o$ = 0.5 μM, the concentration used in the experiment; and $K_m$ = 14.5 μM, the experimentally determined value (i.e., control from Figure 7B). With these constraints, we found that $K_{m,x}$ = 90 nM and γ = 0.20.

To see if the mechanism of Figure 2A is actually at work for SRT1460, as the above analysis might suggest, we examined the data from another type of activation experiment in which initial velocities were determined as a function of substrate concentration, at several fixed concentrations of SRT1460. If the mechanism of Figure 2A obtains for SRT1460, parameter estimates from the two types of experiment must be the same. The data set we examined is also from Milne et al. ((2007) Nature 450, 712-716) and is shown in Figure 7B. A global fit of this data to eq (5), yields: $K_{m,x}$ = 1.0 ± 0.1 μM and γ = 1.53 ± 0.07. In this fit, $K_d$ was assigned the experimentally determined value of 140 μM, and $K_m$ and $V_{max}$ were assigned the values from the curve with [SRT1460] = 0 (see Table 5).

Table 5 Summary of Steady-State Kinetic Parameters for the SRT1460-Activated Deacetylation of TAMRA-Peptide[a]

| [SRT1460] (μM) | $V_{max,app}$ (units) | $K_{m,app}$ (μM) | $(V_{max}/K_m)_{app}$ (unit/μM) |
|---|---|---|---|
| 0 | 10 | 14 | 0.7 |
| 3 | 11 | 9.1 | 1.2 |
| 11 | 12 | 5.6 | 2.1 |
| 37 | 14 | 4.7 | 3.0 |

(a) Each data set of Figure 7B was fit to the Michaelis-Menten equation.

Note the very large discrepancy between the activation parameters $K_{m,x}$ and γ, for the two types of activation experiment. $K_{m,x}$ = 0.090 μM and γ = 0.20, from analysis of the activator titration curve, and $K_{m,x}$ = 1.0 and γ = 1.53, from analysis of the Michaelis-Menten plots at fixed concentrations of activator. Thus, the mechanism of Figure 2A cannot explain activation by SRT1460 in a manner that leads to internally consistent results.

Enzyme allostery can account for the activation of the SIRT1-Catalyzed deacetylation of TAMRA-Peptide by SRT1460 When the rate law for the allosteric mechanism of activation, is used to fit the data of Figure 7B the following best-fit parameters are obtained: $K_x$ = 8.7 ± 0.8 μM, β = 0.26 ± 0.02, and γ = 1.48 ± 0.04. The lines drawn using eq (9) and these parameters show an excellent fit to the data. These lines superimpose on those drawn from the individual fits and the parameter estimates of Table 5.

To examine the possibility that activation by STACs may be dependent on the peptide sequence, and might occur only for peptides having sequences corresponding to natural substrates, we tested 10, 11, and 12 for their ability

to activate the SIRT1-catalyzed deacetylation of p53-20mer. Significantly, these three compounds inhibit this reaction (see Figure 9). These results are consistent with the allosteric mechanism of Figure 2B, where the ratio $\gamma/\beta$ determines if an exosite ligand behaves as an activator or an inhibitor; i.e., activation is seen when $\gamma/\beta > 1$, while inhibition is seen when $\gamma/\beta < 1$. Note that the mechanism of Figure 2A cannot account for inhibition, since, in it, there is no provision for formation of a complex of enzyme and modulator. It should be noted that while inhibition by substrate depletion (i.e., X:S is not a substrate, but simply acts as a substrate 'sink') is a formal possibility, it is ruled out because STACs do not bind to peptides without TAMRA, as pointed out by Pacholec et al. ((2010) J. Biol. Chem. 285, 8340-8351), or some other 'bulky' group.

**Example 10 *Structural Model of SIRT1***

The SIRT1 homology model was created with ACS2 peptide-bound SIRT3 structure (PDB id. 3GLR) as (Jin et al. (2009) J. Biol. Chem. 284, 24394-24405) the template using Swiss-Pdb Viewer 4.0.1 (Guex and Peitsch (1997) Electrophoresis 18, 2714-2723). The Ac-Arg-His-Lys-Lys$^{Ac}$-Trp-$NH_2$ peptide was modeled based on the p53 peptide bound in the *A. Fulgidus* SIR2 structure (PDB id. 1MA3) (Avalos et al. (2002) Mol Cell 10, 523-535) and the ACS2 peptide bound in the SIRT3 structure (PDB id. 3GLR) (Jin et al. (2009) J. Biol. Chem. 284, 24394-24405).

**Example 11 Preparation of 2-butyl-6-(piperidin-4-ylamino)-N-(2-(6-((piperidin-4-yloxy)methyl)thiazolo[5,4-b]pyridin-2-yl)phenyl)pyrimidine-4-carboxamide (X):**

Step 1) Synthesis of 2-butyl-6-hydroxypyrimidine-4-carboxylic acid:

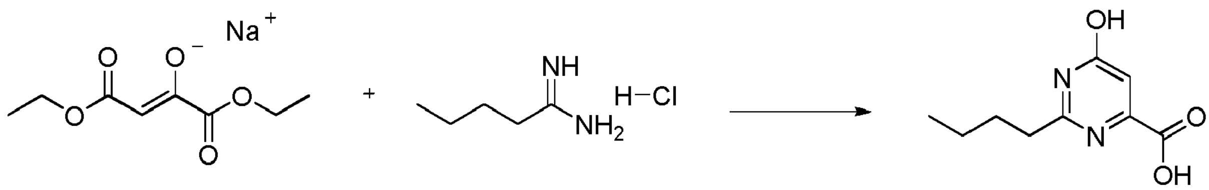

To 21.0 g (100 mmol) of diethyl oxaloacetate sodium salt was added 80 mL of water. To the stirred suspension was added 16 mL (100 mmol) of 6.25 M $NaOH_{(aq.)}$ over 1 min at ambient temperature. The mixture was stirred for 10 min, until only traces of undissolved oxaloacetate ester remained, giving an orange solution. To this was added a solution of 13.9 g (100 mmol) of n-pentanamidine hydrochloride in 20 mL of water. The reaction was monitored with a pH meter, and additional 6.25M NaOH was added as necessary to keep the pH between 10 and 11. After stirring at ambient temperature for 22h, the mixture was cooled with an ice bath, then 12M HCl was added until pH = 2, giving a white precipitate. This was filtered, washed with 50 mL of water, then dried on the filter for 1 h. The white solid was suspended in 50 mL of heptanes and distilled at 1 bar with a Dean-Stark trap until no more water collected in the trap. The suspension was cooled, filtered, and dried on the filter to give 8.13 g (41 %) of 2-butyl-6-hydroxypyrimidine-4-carboxylic acid. MS (ESI) calcd for $C_9H_{12}N_2O_3$: 196; found: 197 [M+H].

Step 2) Synthesis of 2-butyl-6-chloropyrimidine-4-carbonyl chloride:

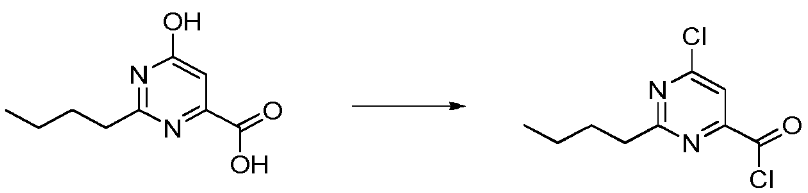

To 5.0 g (25.48 mmol) of 2-butyl-6-hydroxypyrimidine-4-carboxylic acid was added 35 mL of phosphorus oxychloride. The reaction was stirred at 105 °C for 1 h, then concentrated *in vacuo.* The dark residue was suspended in 50 mL of heptanes, then concentrated *in vacuo* to remove most of the remaining phosphorus oxychloride. Next, the residue was suspended in 100 mL of heptanes, and extracted with water (3 x 25 mL), then brine (1 x 25 mL). The organic layer was dried over $MgSO_4$, filtered, and concentrated *in vacuo* to afford 5.1 g (86%) of 2-butyl-6-chloropyrimidine-4-carbonyl chloride. MS (ESI) calcd for $C_9H_{10}Cl_2N_2O$: 233; found: 234 [M+H].

Step 3) Synthesis of 6-(bromomethyl)-2-(2-nitrophenyl)thiazolo[5,4-b]pyridine:

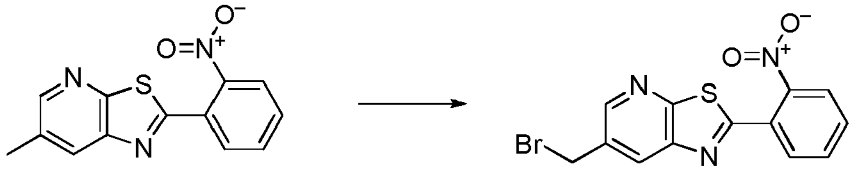

Prepared according to previously published procedure, WO2007/019346.

Step 4) Synthesis of *tert*-butyl 4-((2-(2-nitrophenyl)thiazolo[5,4-b]pyridin-6-yl)methoxy)piperidine-1-carboxylate:

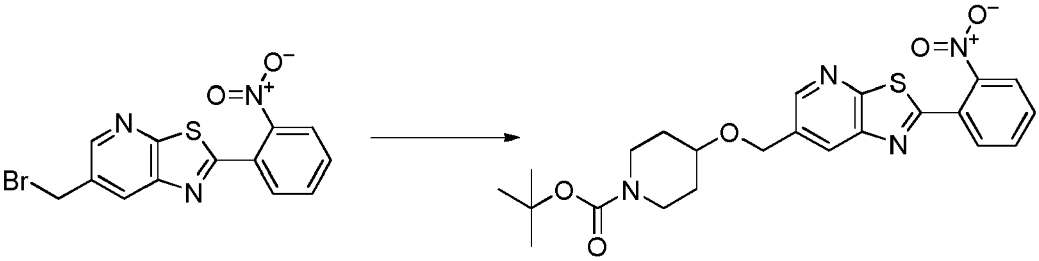

To a mixture of 2.73 g (13.56 g) of *tert*-butyl 4-hydroxypiperidine-1-carboxylate, 4.75 g (13.56 mmol) of 6-bromomethyl-2-(2-nitrophenyl)thiazolo[5,4-b]pyridine, 230 mg (0.68 mmol) of tetrabutylammonium bisulfate, and 5.42 g (135.6 mmol) of sodium hydroxide was added 15 mL of toluene and 5.4 mL of water. The reaction was stirred at ambient temperature, at a rate sufficient to mix the two layers well. After 15 h, the reaction was diluted with 100 mL of 1 M $HCl_{(aq.)}$, then extracted with ethyl acetate (3 x 25 mL). The combined ethyl acetate layers were back extracted with water (1 x 25 mL), and brine (1 x 25 mL), dried over $MgSO_4$, filtered, and concentrated to an orange oil. This was purified via medium pressure silica gel chromatography (240 g pre-packed column), eluting with an isocratic mixture of 50% ethyl acetate: heptanes. *tert*-Butyl 4-hydroxypiperidine-1-carboxylate and the product co-eluted. The product containing fractions were pooled and concentrated, then the crude product was taken up in 20 mL of hot ethyl acetate, and diluted with an equal volume of heptanes. *tert*-butyl 4-((2-(2-nitrophenyl)thiazolo[5,4-b]pyridin-6-yl)methoxy)piperidine-1-carboxylate crystallized to give a yellow solid, 3.64 g. A second crop of 509 mg was as pure as the first crop. Total 4.15 g (65%). MS (ESI) calcd for $C_{21}H_{26}N_4O_5S$: 470; found: 471 [M+H].

*This general procedure can be used to synthesize a variety of 2-nitrophenyl)thiazolo[5,4-b]pyridine derivative via the selection of the appropriate hydroxyl components.*

Step 5) Synthesis of *tert*-butyl 4-((2-(2-aminophenyl)thiazolo[5,4-b]pyridin-6-yl)methoxy)piperidine-1-carboxylate:

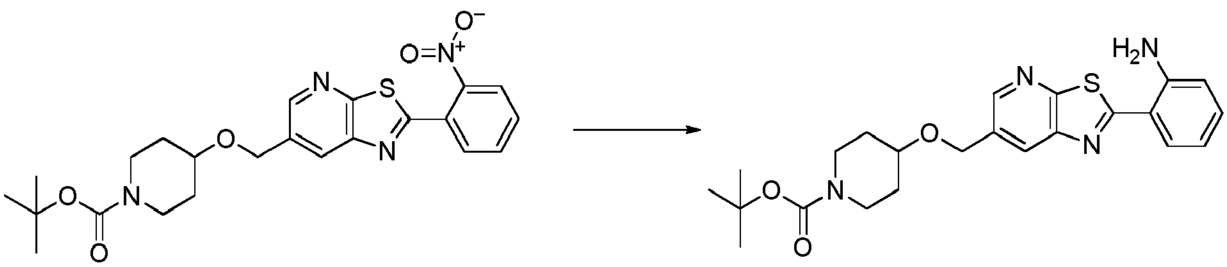

To 3.64 g (7.74 mmol) of *tert*-butyl 4-((2-(2-nitrophenyl)thiazolo[5,4-b]pyridin-6-yl)methoxy)piperidine-1-carboxylate was added 2.13 g (38.7 mmol) of iron powder, 497 mg (9.23 mmol) of ammonium chloride, then 20 mL of isopropanol and 4 mL of water. The reaction was heated at reflux for 1.75 h, then 4 mL of 4M $NaOH_{(aq.)}$ was added. The mixture was heated at reflux for 5 min, then filtered. The solids were stirred with 50 mL of $CH_2Cl_2$ for 10 min, then the mixture was filtered. The aqueous isopropanol mixture was concentrated in vacuo to remove most of the alcohol, then the remaining solution was diluted with 15 mL of water. The aqueous suspension was shaken with the $CH_2Cl_2$ filtrate then filtered through a paper filter. The $CH_2Cl_2$ layer was then extracted with water (1 x 15 mL), 2M $NaOH_{(aq.)}$ (1x15 mL), and brine (1x15 mL), then concentrated *in vacuo* to a foam. This was taken up in a mixture of 30 mL of ethyl acetate and 30 mL of pentane, then extracted with 2M NaOH (3 x 15 mL), and brine (1 x 15 mL), dried over $MgSO_4$, filtered, and concentrated to a yellow solid. The crude product was suspended in 15 mL of $CH_2Cl_2$, then loaded onto a 120 g

silica gel MPLC column, and eluted with a 40-50% pentane: ethyl acetate gradient. Following concentration of the product containing fractions, recrystallization from 65 mL of ethyl acetate afforded 795 mg (23%) of tert-butyl 4-((2-(2-aminophenyl)thiazolo[5,4-b]pyridin-6-yl)methoxy)piperidine-1-carboxylate. MS (ESI) calcd for $C_{23}H_{28}N_4O_3S$: 440; found: 441[M+H].

Step 6) Synthesis of *tert*-butyl 4-((2-(2-(2-butyl-6-chloropyrimidine-4-carboxamido)phenyl)thiazolo[5,4-b]pyridin-6-yl)methoxy)piperidine-1-carboxylate:

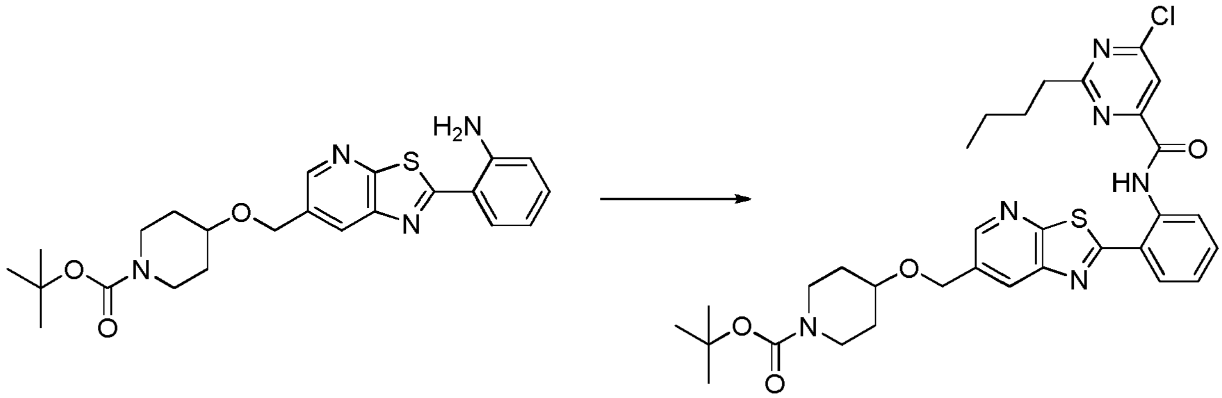

To a solution of 795 mg (1.80 mmol) of *tert*-butyl 4-((2-(2-aminophenyl)thiazolo[5,4-b]pyridin-6-yl)methoxy)piperidine-1-carboxylate in 8 mL of $CH_2Cl_2$ was added 0.45 mL (3.2 mmol) of triethylamine, then a solution of 505 mg (2.17 mmol) of 2-butyl-6-chloropyrimidine-4-carbonyl chloride in 2 mL of $CH_2Cl_2$. After 1.25 h, the reaction was diluted with 30 mL of methanol to give a crystalline precipitate. The precipitate was filtered, washed with 15 mL of methanol, and dried on the filter to give tert-butyl 4-((2-(2-(2-butyl-6-chloropyrimidine-4-carboxamido)phenyl)thiazolo[5,4-b]pyridin-6-yl)methoxy)piperidine-1-carboxylate 1.04 g (91%). MS (ESI) calcd for $C_{23}H_{28}N_4O_3S$: 440; found: 441 [M+H].

*This general procedure was used to prepare 3-(2-(methylamino)ethylamino)-N-(2-(6-((piperidin-4-yloxy)methyl)thiazolo[5,4-b]pyridin-2-yl)phenyl)benzamide and 3,4-dimethoxy-N-(2-(6-((piperidin-4-yloxy)methyl)thiazolo[5,4-b]pyridin-2-yl)phenyl)benzamide by coupling with the appropriate acid chloride. N-(2-(6-((dimethylamino)methyl)thiazolo[5,4-b]pyridin-2-yl)phenyl)quinoxaline-2-carboxamide was similarity prepared via this route by coupling with dimethyl amine in step 4.*

Step 7) Synthesis of tert-butyl 4-((2-(2-(6-(1-(tert-butoxycarbonyl)piperidin-4-ylamino)-2-butylpyrimidine-4-carboxamido)phenyl)thiazolo[5,4-b]pyridin-6-yl)methoxy)piperidine-1-carboxylate:

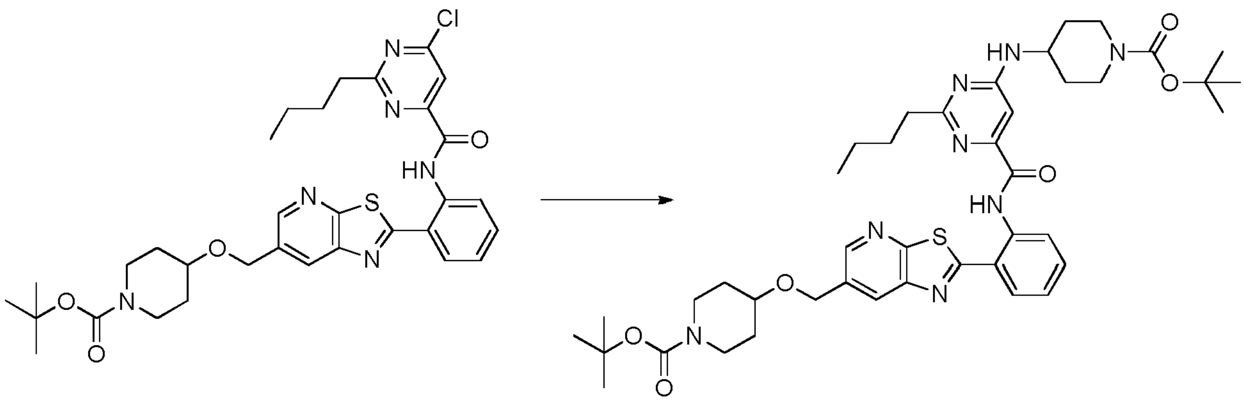

To a mixture of 709 mg (1.11 mmol) of *tert*-butyl 4-((2-(2-(2-butyl-6-chloropyrimidine-4-carboxamido)phenyl)thiazolo[5,4-b]pyridin-6-yl)methoxy)piperidine-1-carboxylate and 267 mg (1.33 mmol) of *tert*-butyl 4-aminopiperidine-1-carboxylate was added 7 mL of DMSO and 0.40 mL (2.24 mmol) of *N,N*-diisopropyl-*N*-ethylamine. The mixture was heated at 100 °C under $N_2$ for 4 h. The reaction was monitored by $^1H$ NMR, taking an aliquot of the reaction and dissolving in $CDCl_3$, then observing the resonances in the region downfield of 6 ppm. After the starting material had been consumed, the reaction was diluted with 35 mL of water to give a granular precipitate. This was filtered and washed with 25 mL of water to give a light tan solid. The crude solid was recrystallized from 30 mL of isopropanol, then the product was washed with 60 mL of cold isopropanol to afford 674 mg (76%) of tert-butyl 4-((2-(2-(6-(1-(tert-butoxycarbonyl)piperidin-4-ylamino)-2-butylpyrimidine-4-carboxamido)phenyl)thiazolo[5,4-b]pyridin-6-yl)methoxy)piperidine-1-carboxylate. MS (ESI)

calcd for $C_{42}H_{56}N_8O_6S$: 801; found: 802 [M+H].

Step 8) Synthesis of 2-butyl-6-(piperidin-4-ylamino)-N-(2-(6-((piperidin-4-yloxy)methyl)thiazolo[5,4-b]pyridin-2-yl)phenyl)pyrimidine-4-carboxamide (**X**):

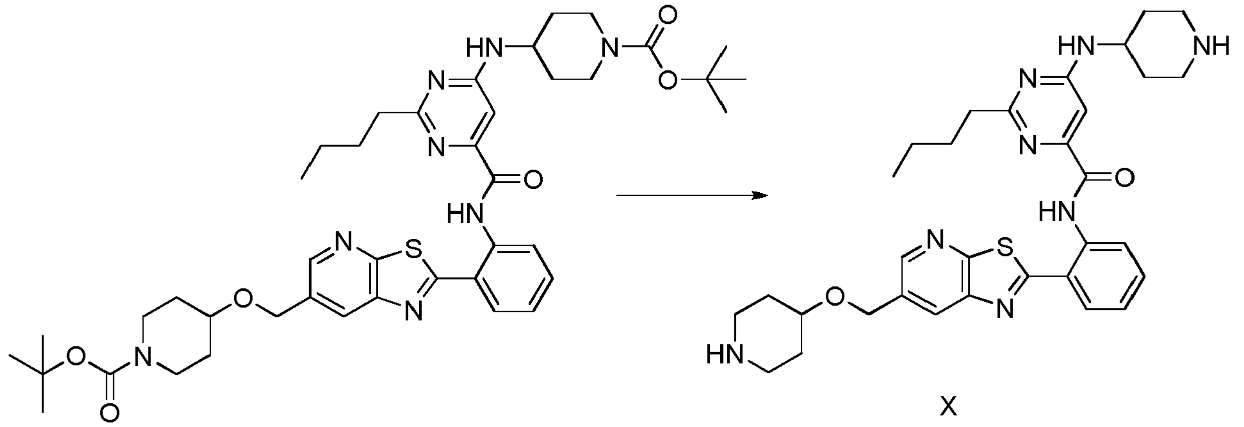

X

To 600 mg (0.75 mmol) of *tert*-butyl 4-((2-(2-(6-(1-(tert-butoxycarbonyl)piperidin-4-ylamino)-2-butylpyrimidine-4-carboxamido)phenyl)thiazolo[5,4-b]pyridin-6-yl)methoxy)piperidine-1-carboxylate was added 5 mL of trifluoroacetic acid, slowly to control the vigorous evolution of gas. The solution was stirred for 10 min at ambient temperature, the solvent was removed in vacuo at 50 °C. The residue was diluted with 12 mL of saturated $NaHCO_{3(aq.)}$, giving an oily suspension with pH = 8. This was stirred and heated at 80 °C for 60 min, then cooled to ambient temperature, affording a pale yellow precipitate. The precipitate was filtered, then suspended in water and filtered again (3 x 20 mL). The wet solid was dried by suction on the filter for 18 h to give 364 mg (81%) of 2-butyl-6-(piperidin-4-ylamino)-N-(2-(6-((piperidin-4-yloxy)methyl)thiazolo[5,4-b]pyridin-2-yl)phenyl)pyrimidine-4-carboxamide. MS (ESI) calcd for $C_{32}H_{40}N_8O_2S$: 600; found: 601[M+H].

**Example 12 Preparation of 2-isobutyl-6-(piperidin-4-ylamino)-N-(2-(6-((piperidin-4-yloxy)methyl)thiazolo [5,4-b] pyridin-2-yl)phenyl)pyrimidine-4-carboxamide (XI):**

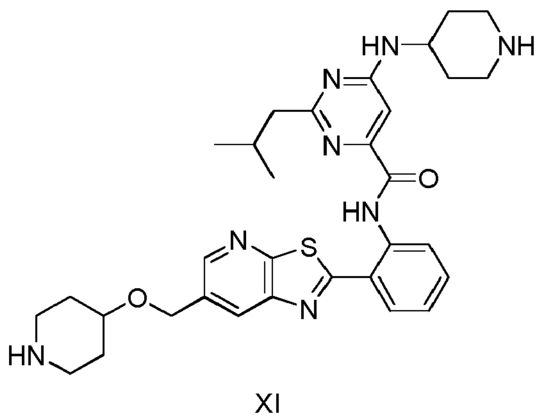

XI

Prepared via a similar sequence as for the 2-butylpyrimidine analogue, by substituting 6-chloro-2-isobutylpyrimidine-4-carbonyl chloride in step 6. The acid chloride was prepared using the same two step procedure described for 2-butyl-6-chloropyrimidine-4-carbonyl chloride, starting with isovalerylamidine hydrochloride. The final product was purified via reversed phase HPLC, followed by treatment of the product containing fractions with aqueous HCl prior to concentration to afford the 2HCl salt of 2-isobutyl-6-(piperidin-4-ylamino)-N-(2-(6-((piperidin-4-yloxy)methyl)thiazolo[5,4-b]pyridin-2-yl)phenyl)pyrimidine-4-carboxamide. MS (ESI) calcd for $C_{32}H_{40}N_8O_2S$: 600; found: 601[M+H].

**Example 13 Preparation of 2-butyl-6-(2-(methylamino)ethylamino)-N-(2-(6-((piperidin-4-yloxy)methyl)thiazolo[5,4-b]pyridin-2-yl)phenyl)pyrimidine-4-carboxamide (XII):**

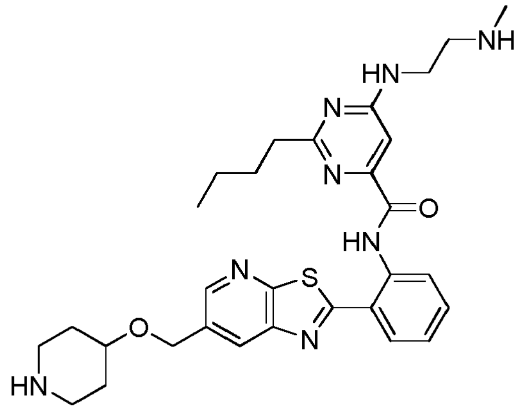

XII

Prepared via a similar sequence as for 2-butyl-6-(piperidin-4-ylamino)-N-(2-(6-((piperidin-4-yloxy)methyl)thiazolo[5,4-b]pyridin-2-yl)phenyl)pyrimidine-4-carboxamide, substituting (N-Boc-N-methyl)ethylenediamine in step 7. The final product was purified via reversed phase HPLC, followed by treatment of the product containing fractions with aqueous HCl prior to concentration to afford 2-butyl-6-(2-(methylamino)ethylamino)-N-(2-(6-((piperidin-4-yloxy)methyl)thiazolo[5,4-b]pyridin-2-yl)phenyl)pyrimidine-4-carboxamide as the 2HCl salt. MS (ESI) calcd for $C_{30}H_{38}N_8O_2S$: 575; found: 576[M+H].

**Example 14 Preparation of N-(2-(6-(((R)-3-fluoropyrrolidin-1-yl)methyl)thiazolo[5,4-b] pyridin-2-yl)phenyl)-3-(quinuclidin-3-yloxy)benzamide (V):**

Step 1) Synthesis of 3-(quinuclidin-3-yloxy)benzoic acid:

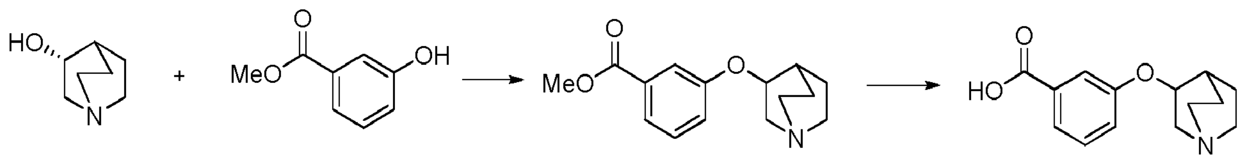

(*R*)-quinuclidin-3-ol as the HCl salt (1 g, 6.11 mmol) was dissolved in 5 mL of $H_2O$ and neutralized with $NaHCO_3$ (0.51 g, 6.11 mmol) and the mixture was lyophilized. The residue was extracted with EtOH and the EtOH layer concentrated to afford (R)-quinuclidin-3-ol as the free base which was subsequently taken up in 30 mL of THF along with DIAD (2.4 mL, 12.22 mmol), $PPh_3$ (1.6 g, 12.22 mmol) and methyl 3-hydroxybenzoate (0.93 g, 7.33 mmol). Reaction mixture was stirred at rt for 24 hours. It was then concentrated and partitioned between $CH_2Cl_2$ and 2N HCl. The aqueous layer was separated and washed (1 x $CH_2Cl_2$). The aqueous layer was neutralized with $NaHCO_3$ and extracted with $CH_2Cl_2$. The organic layer was dried over $Na_2SO_4$ and concentrated to afford methyl 3-(quinuclidin-3-yloxy)benzoate (0.85g, 53.2%) as crude material. The crude material was subjected to standard base hydrolysis conditions in ethanol with aqueous LiOH solution and brought to reflux. Upon completion, the reaction was cooled to rt and neutralized with 5N HCl to afford 3-(quinuclidin-3-yloxy)benzoic acid.

Step 2) Synthesis of 3-(quinuclidin-3-yloxy)benzoyl chloride:

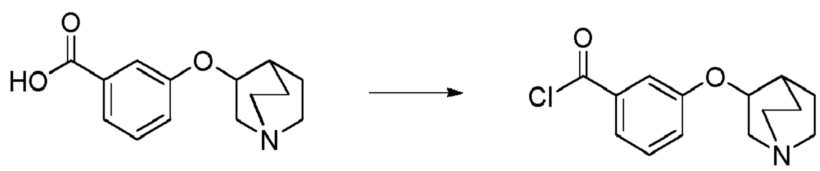

3-(quinuclidin-3-yloxy)benzoic acid (1 equiv.) was suspended in CH2Cl2 and to this solution was added oxalyl chloride (2.35 equiv.) and 2 drops of DMF. After 1 hour at rt, the solvents were removed in vacuo and the resulting residue placed under high vacuo for 30 min to afford 3-(quinuclidin-3-yloxy)benzoyl chloride.

Step 3) Synthesis of (R)-6-((3-fluoropyrrolidin-1-yl)methyl)-2-(2-nitrophenyl)thiazolo[5,4-b]pyridine:

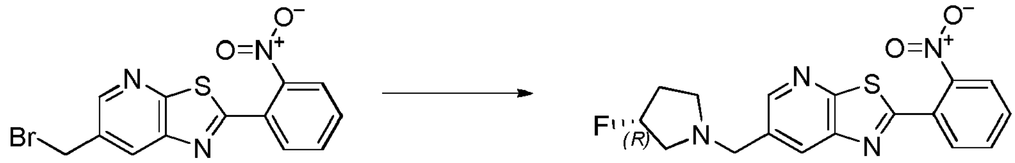

Prepared via a similar procedure previously published WO2007/019346. 6-(bromomethyl)-2-(2-nitrophenyl)thiazolo[5,4-b]pyridine (1 equiv.) and (R)-3-fluoropyrrolidine (1 equiv.) were taken up in acetonitrile with $Et_3N$ (1 equiv.) and heated at 50 °C for 4 h. The reaction was cooled to rt and stirred for an additional 60h. Purification by silica gel chromatography (pet ether:EtOAc: $Et_3N$) afforded (R)-6-((3-fluoropyrrolidin-1-yl)methyl)-2-(2-nitrophenyl)thiazolo[5,4-b]pyridine. MS (ESI) calcd for $C_{17}H_{15}FN_4$O2S: 358; found: 359[M+H].

Step 4) Synthesis of N-(2-(6-(((R)-3-fluoropyrrolidin-1-yl)methyl)thiazolo[5,4-b]pyridin-2-yl)phenyl)-3-(quinuclidin-3-yloxy)benzamide (V):

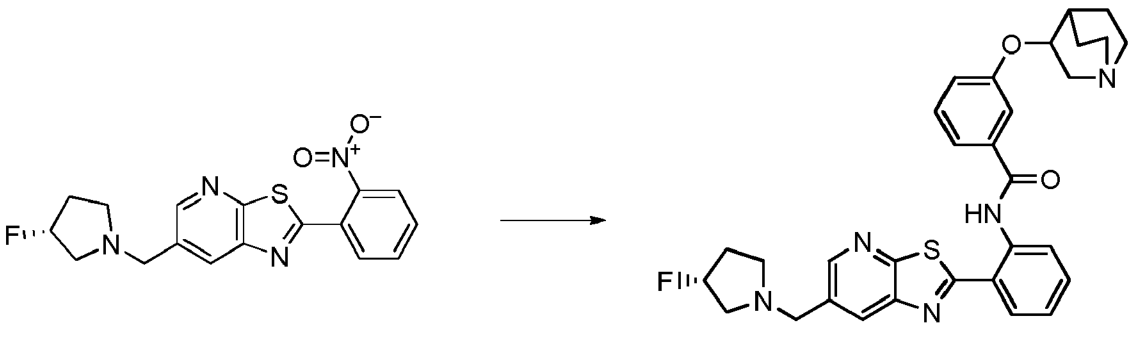

V

Prepared by a similar procedure previously published WO2007/019346. (*R*)-6-((3-fluoropyrrolidin-1-yl)methyl)-2-(2-nitrophenyl)thiazolo[5,4-b]pyridine was subjected to standard iron reduction conditions as described above to afford (R)-2-(6-((3-fluoropyrrolidin-1-yl)methyl)thiazolo[5,4-b]pyridin-2-yl)aniline. The aniline (1 equiv.) was taken up in $CH_2Cl_2$ and triethylamine (1.8 equiv.) was added. Then a solution of 3-(quinuclidin-3-yloxy)benzoyl chloride (0.8 equiv.) in $CH_2Cl_2$ was added. After 1.25 h, the reaction was diluted with 30 mL of methanol to give a crystalline precipitate. The precipitate was filtered, washed with methanol, and dried on the filter to give N-(2-(6-(((*R*)-3-fluoropyrrolidin-1-yl)methyl)thiazolo[5,4-b]pyridin-2-yl)phenyl)-3-(quinuclidin-3-yloxy)benzamide. MS (ESI) calcd for $C_{31}H_{32}FN_5O_2S$: 558; found: 559[M+H].

**Example 15 Preparation of N-(2-(3-(piperazin-1-ylmethyl)imidazo[2,1-b]thiazol-6-yl)phenyl)biphenyl-3-carboxamide (I):**

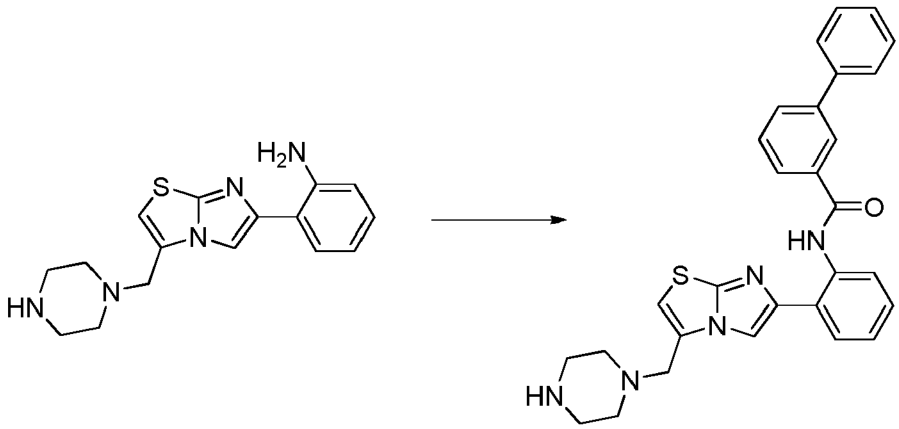

I

Prepared via a similar sequence as previously reported by Perni et al. (J. Med. Chem., 2009, 52, 1275-1283); WO2007/019346.

*This general procedure was used to prepare N-(2-(3-(piperazin-1-ylmethyl)imidazo[2,1-b]thiazol-6-yl)phenyl)oxazolo[4,5-b]pyridine-2-carboxamide and N-(2-(3-(2,5-diazabicyclo[2.2.1]heptan-2-ylmethyl)imidazo[2,1-b]thiazol-6-yl)phenyl)-4-methyl-2-(pyridin-3-yl)thiazole-5-carboxamide by selecting the appropriate acid chloride and amine components.*

**Example 16 Preparation of N-(4-cyano-3-(trifluoromethyl)phenyl)-2-(6-(piperazin-1-yl)pyridin-3-yl)-1H-benzo[d]imidazole-4-carboxamide (XX):**

Step 1) Synthesis of 2-(6-chloropyridin-3-yl)-1H-benzo[d]imidazole-4-carboxylic acid:

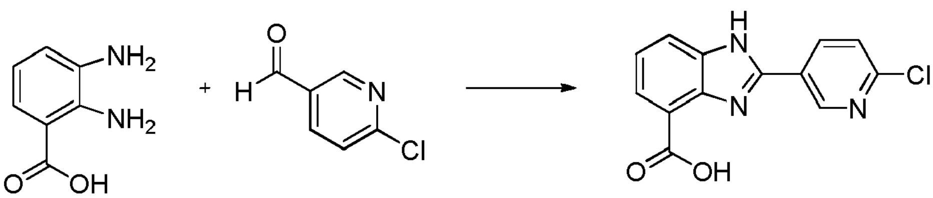

Prepared via a previously published procedure WO2010/003048.

*This general procedure was used to prepare the analogues 2-(2-chloropyridin-4-yl)-1H-benzo[d]imidazole-4-carboxylic acid.*

Step 2) Synthesis of 2-(6-chloropyridin-3-yl)-N-(4-cyano-3-(trifluoromethyl)phenyl)-1H-benzo[d]imidazole-4-carboxamide:

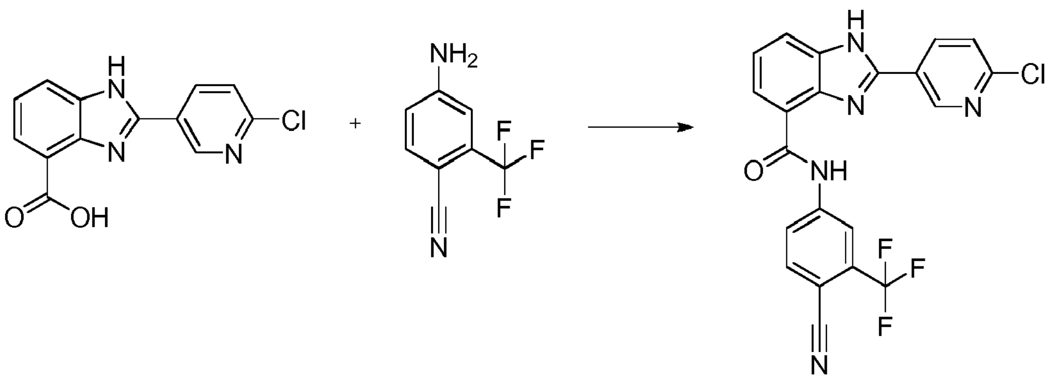

Prepared by a similar route to one previously published WO2010/003048. 2-(6-chloropyridin-3-yl)-1H-benzo[d]imidazole-4-carboxylic acid (250 mg, 0.91 mol) was taken up in DMF (5 mL) along with 4-amino-2-(trifluoromethyl)benzonitrile (170.57 mg, 0.91 mmol)and HATU (695.06 mg, 1.83 mmol). DIEA (0.32 mL, 1.83 mmol) was added

and the reaction was stirred at rt overnight. The reaction mixture was diluted with water and the resulting solids were collected via vacuum filtration. Purification by silica gel chromatography (pentanes/EtOAc) afforded 2-(6-chloropyridin-3-yl)-N-(4-cyano-3-(trifluoromethyl)phenyl)-1H-benzo[d]imidazole-4-carboxamide. MS (ESI) calcd for $C_{21}H_{11}ClF_3N_5O$: 442 found: 443[M+H].

*This general amide coupling can be used to make a variety of 1H-benzo[d]imidazole-4-carboxamide derivatives by selecting the appropriate acid and amine components.*

Step 3) Synthesis of tert-butyl 4-(5-(4-(4-cyano-3-(trifluoromethyl)phenylcarbamoyl)-1H-benzo[d]imidazol-2-yl)pyridin-2-yl)piperazine-1-carboxylate:

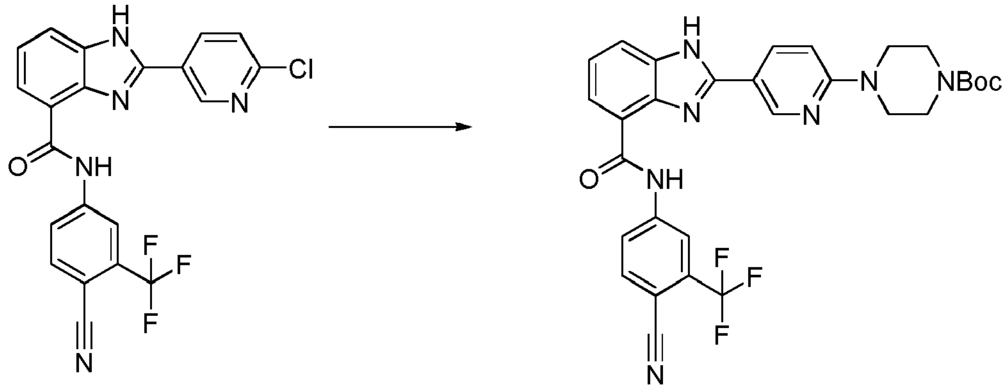

Prepared by a previously published route WO2010/003048. A mixture containing 2-(6-chloropyridin-3-yl)-N-(4-cyano-3-(trifluoromethyl)phenyl)-1H-benzo[d]imidazole-4-carboxamide (1 equiv.) and *tert*-butylpiperazine-1-carboxylate (1 equiv.) in DMSO was stirred at 140 °C in a microwave reactor for 25 min. The reaction was cooled to rt and diluted with water. The resulting solids were collected by filtration, washed with water and dried under vacuum to afford *tert*-butyl 4-(5-(4-(4-cyano-3-(trifluoromethyl)phenylcarbamoyl)-1H-benzo[d]imidazol-2-yl)pyridin-2-yl)piperazine-1-carboxylate. MS (ESI) calcd for $C_{30}H_{28}F_3N_7O_3$: 592; found: 593[M+H].

Step 4) Synthesis of N-(4-cyano-3-(trifluoromethyl)phenyl)-2-(6-(piperazin-1-yl)pyridin-3-yl)-1H-benzo[d]imidazole-4-carboxamide **(XX)**

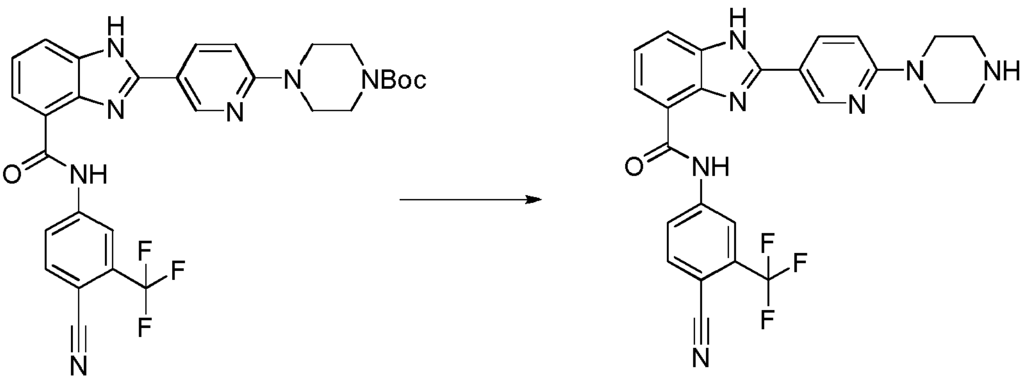

XX

Prepared by a previously published route WO2010/003048. A mixture containing *tert*-butyl 4-(5-(4-(4-cyano-3-(trifluoromethyl)phenylcarbamoyl)-1H-benzo[d]imidazol-2-yl)pyridin-2-yl)piperazine-1-carboxylate (0.04 mmol) in methanolic HCL (3N, 2 mL) was stirred at rt for 18 hours. The precipitated solids were collected by filtration, washed with methanol and dried to afford *N*-(4-cyano-3-(trifluoromethyl)phenyl)-2-(6-(piperazin-1-yl)pyridin-3-yl)-1H-benzo[d]imidazole-4-carboxamide. MS (ESI) calcd for $C_{25}H_{20}F_3N_7O$: 491; found: 492[M+H].

*The general procedure above was used in the preparation of prepare N- (6-methoxybenzo[d]thiazol-2-yl)-2-(6-(piperazin-1-yl)pyridin-3-yl)-1H-benzo[d]imidazole-4-carboxamide and 2-(2-morpholinopyridin-4-yl)-N-(3-((piperidin-4-yloxy)methyl)phenyl)-1H-benzo[d]imidazole-4-carboxamide.*

**Example 17 Preparation of 2-(2-morpholinopyridin-4-yl)-N-(3-((piperidin-4-yloxy)methyl)phenyl)-1H-benzo[d]imidazole-4-carboxamide (XVII):**

Step 1) Synthesis of *tert*-butyl 4-(3-aminobenzyloxy)piperidine-1-carboxylate:

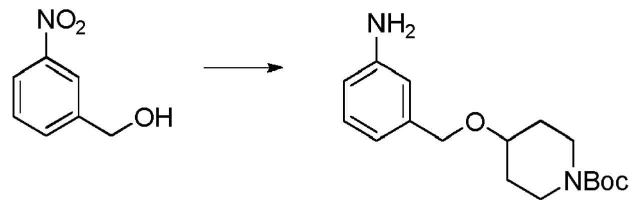

A mixture of (3-nitrophenyl)methanol (1 g, 6.55 mmol) and pyridine (19.59 mmol) were dissolved in $CH_2Cl_2$ and cooled to 0 °C. 4-toluenesulfonyl chloride (1.57 g, 7.18 mmol) was added dropwise at 0 °C and after completion of addition the reaction was allowed to warm to rt. Upon completion the reaction was concentrated, diluted in $CH_2Cl_2$, quenched and dried under high vacuo and carried forward without any further purification.

To a stirred solution of 1-Boc-4-hydroxypiperdine (1.5 g, 6.55 mmol) in $CH_2Cl_2$ was added NaH (0.19 g, 7.73 mmol) in portions at 0 °C, then allowed to stir for an additional 10 min at which point 3-nitrobenzyl 4-methylbenzenesulfonate was added. Followed by standard iron reduction and deprotection sequence, previously described above and previously published in WO2010/003048. MS (ESI) calcd for $C_{17}H_{26}N_2O_3$: 306; found: 307[M+H].

Step 2) Synthesis of *tert*-butyl 4-(3-(2-(2-chloropyridin-4-yl)-1H-benzo[d]imidazole-4-carboxamido)benzyloxy)piperidine-1-carboxylate:

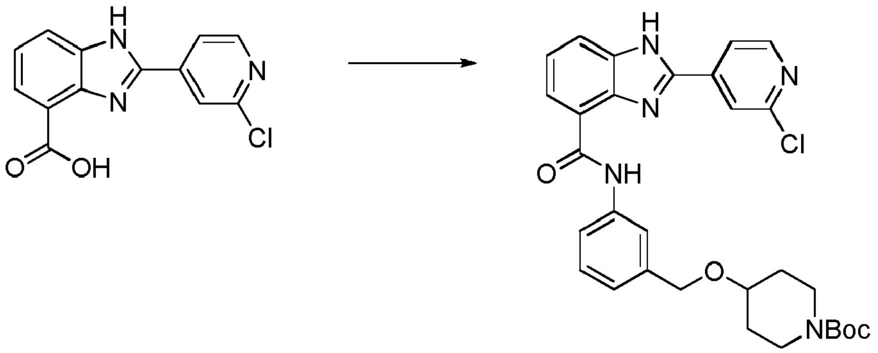

Prepared by a similar route previously published WO2010/003048, see general amide procedure above. MS (ESI) calcd for $C_{30}H_{12}CN_5O_4$: 561; found: 562[M+H].

Step 3) Synthesis of 2-(2-morpholinopyridin-4-yl)-N-(3-((piperidin-4-yloxy)methyl)phenyl)-1H-benzo[d]imidazole-4-carboxamide **(XVII):**

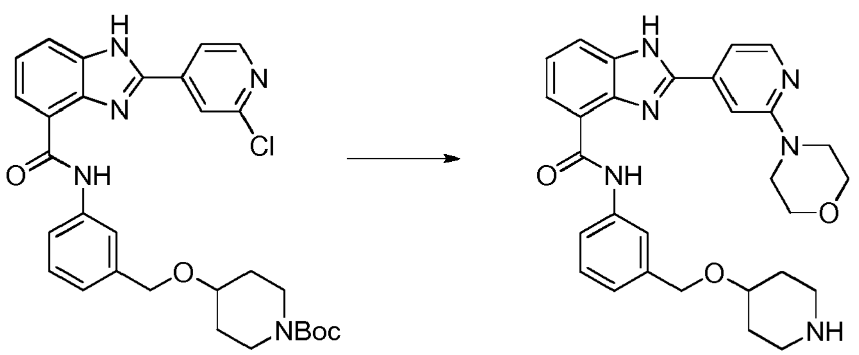

XVII

Prepared by a similar route previously published WO2010/003048, see general procedures described above. MS (ESI) calcd for $C_{29}H_{32}N_6O_3$: 512; found: 513[M+H].

**Example 18 Synthesis of N-(2-(2-(3-(dimethylamino)propylamino)pyridin-4-yl)-1H-benzo[d]imidazol-4-yl)-4-(piperazin-1-ylmethyl)benzamide trihydrochloride (XIV):**

Step 1) Preparation of 2-(2-chloropyridin-4-yl)-1H-benzo[d]imidazole-4-carboxylic acid:

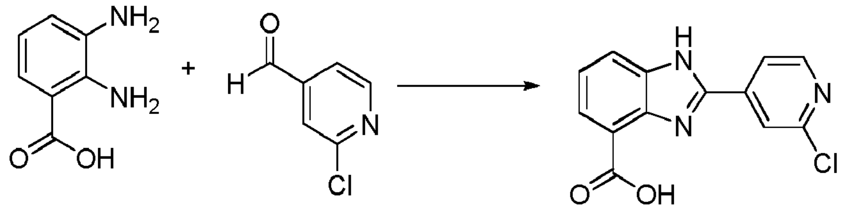

2,3-Diaminobenzoic acid (1.07 g, 7.06 mmol), 2-chloroisonicotinaldehyde (1.0 g, 7.06 mmol), and $Na_2S_2O_5$ (1.75 g, 9.18 mmol) were taken up in DMF (40 mL) and stirred at 100 °C for 18 h. The reaction mixture was cooled to room temperature and diluted with $H_2O$ (100 mL). The resulting mixture was stirred for 1h at room temperature and filtered. The collected solids were washed with water, and dried to afford 2-(2-chloropyridin-4-yl)-1H-benzo[d]imidazole-4-carboxylic acid as a solid (1.14 g, 59% yield). MS (ESI) calcd for $C_{13}H_8ClN_3O_2$: 273; found: 274[M+H].

Step 2) Preparation of benzyl 2-(2-chloropyridin-4-yl)-1H-benzo[d]imidazol-4-ylcarbamate:

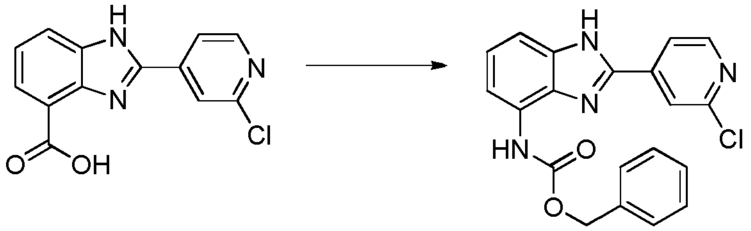

To a solution of 2-(2-chloropyridin-4-yl)-1H-benzo[d]imidazole-4-carboxylic acid (1.14 g, 4.17 mmol) and triethylamine (0.64 mL, 4.58 mmol) in toluene (15 mL) was added DPPA (0.9 mL, 4.17 mmol) dropwise. The mixture was stirred at room temperature for 18 hrs, then heated to reflux for 2h. Benzyl alcohol (0.65 mL, 6.25 mmol) was added and the resulting mixture was heated to reflux for 18h, concentrated to dryness and purified on silica gel column chromatography (9 to 17% Ethyl acetate in Petroleum ether) to obtain benzyl 2-(2-chloropyridin-4-yl)-1H-benzo[d]imidazol-4-ylcarbamate as a yellow solid (0.69 g, 44% yield). MS (ESI) calcd for $C_{20}H_{15}CN_4O_2$: 378; found: 379[M+H].

Step 3) Preparation of 2-(2-chloropyridin-4-yl)-1H-benzo[d]imidazol-4-amine:

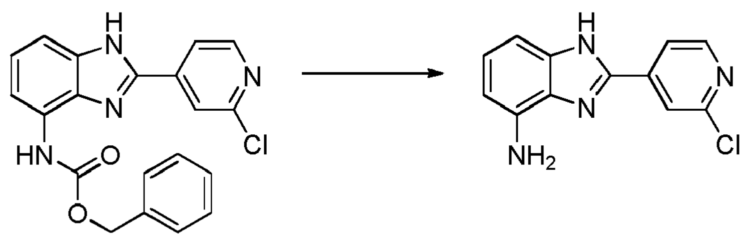

A mixture of benzyl 2-(2-chloropyridin-4-yl)-1H-benzo[d]imidazol-4-ylcarbamate (0.69 g, 1.82 mmol), HBr (8 mL, 33 wt% in acetic acid) and Acetic acid (5 mL) was stirred at room temperature for 0.5 h, and the solid was collected by filtration. The solids were stirred with aqueous $NaHCO_3$ (sat) for 0.5 h, collected by filtration, and dried to obtain 2-(2-chloropyridin-4-yl)-1H-benzo[d]imidazol-4-amine as a yellow solid (0.4 g, 90% yield). MS (ESI) calcd for $C_{12}H_9ClN_4$: 244; found: 245[M+H].

Step 4) Preparation of *tert*-butyl 4-(4-(2-(2-chloropyridin-4-yl)-1H-benzo[d]imidazol-4-ylcarbamoyl)benzyl)piperazine-1-carboxylate:

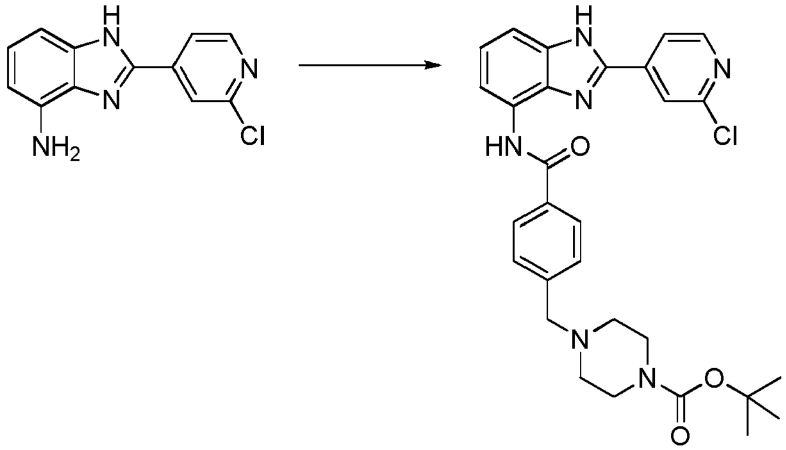

To a solution of 2-(2-chloropyridin-4-yl)-1H-benzo[d]imidazol-4-amine (250 mg, 1.02 mmol), 4-((4-(tert-butoxycarbonyl)piperazin-1-yl)methyl)benzoic acid (360 mg, 1.12 mmol), and HATU (583 mg, 1.53 mmol) in DMF (10 mL) was added N,N'-diisopropylethylamine (0.5 mL, 3.06 mmol). The mixture was stirred 18h at room temperature, diluted with water (40 mL), and extracted with $CH_2Cl_2$ (10 mL x 3). The combined organics layers were washed with brine, dried and concentrated. The residue was purified by silica gel column chromatography (17% to 50% Ethyl acetate in petroleum ether) to obtain *tert*-butyl 4-(4-(2-(2-chloropyridin-4-yl)-1H-benzo[d]imidazol-4-ylcarbamoyl)benzyl)piperazine-1-carboxylate as a yellow solid (270 mg, 48% yield). MS (ESI) calcd for $C_{29}H_{31}ClN_6O_3$: 546; found: 547[M+H].

Step 5) Preparation of *tert*-butyl 4-(4-(2-(2-(3-(dimethylamino)propylamino)pyridin-4-yl)-1H-benzo[d]imidazol-4-ylcarbamoyl)benzyl)piperazine-1-carboxylate:

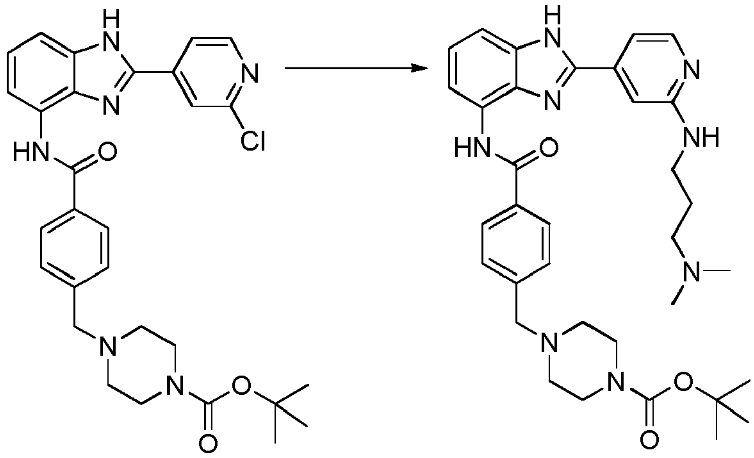

A solution of tert-butyl 4-(4-(2-(2-chloropyridin-4-yl)-1H-benzo[d]imidazol-4-ylcarbamoyl)benzyl)piperazine-1-carboxylate (100 mg, 0.18 mmol) N,N-dimethylpropane-1,3-diamine (0.5 mL) in pyridine (2 mL) was microwave heated (160°C x 2h). The solution was concentrated and purified by prep-TLC to obtain *tert*-butyl 4-(4-(2-(2-(3-(dimethylamino)propylamino)pyridin-4-yl)-1H-benzo[d]imidazol-4-ylcarbamoyl)benzyl)piperazine-1-carboxylate as a yellow solid (40 mg, 36% yield). MS (ESI) calcd for $C_{34}H_{44}N_8O_3$: 612; found: 613[M+H].

Step 6) Preparation of *N*-(2-(2-(3-(dimethylamino)propylamino)pyridin-4-yl)-1H-benzo[d]imidazol-4-yl)-4-(piperazin-1-ylmethyl)benzamide trihydrochloride **(XIV):**

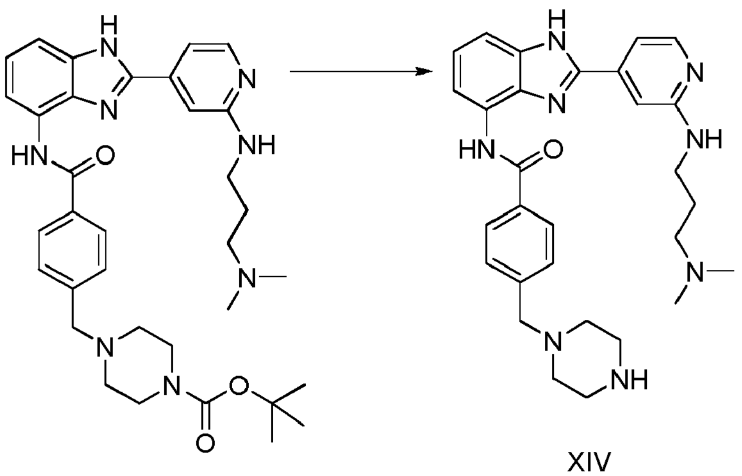

XIV

A solution of tert-butyl 4-(4-(2-(2-(3-(dimethylamino)propylamino)pyridin-4-yl)-1H-benzo[d]imidazol-4-ylcarbamoyl)benzyl)piperazine-1-carboxylate (37 mg, 0.06 mmol) in methanol (1.0 mL) was added 4M HCl/MeOH (2 mL). The mixture was stirred for 1h, and the precipitate was collected by filtration to give *N*-(2-(2-(3-(dimethylamino)propylamino)pyridin-4-yl)-1H-benzo[d]imidazol-4-yl)-4-(piperazin-1-ylmethyl)benzamide trihydrochloride as the HCl salt. (31 mg, 83% yield).MS (ESI) calcd for MS (ESI) calcd for $C_{29}H_{36}N_8O$: 512; found: 514 [M+H].

**Example 19 Preparation of 4-(piperazin-1-ylmethyl)-N-(2-(2-(propylamino)pyridin-4-yl)-1H-benzo[d]imidazol-4-yl)benzamide dihydrochloride (XVI):**

Step 1) Preparation of 2-(2-(propylamino)pyridin-4-yl)-1H-benzo[d]imidazol-4-amine:

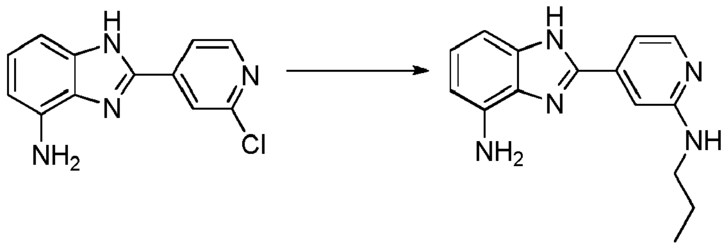

A solution of 2-(2-chloropyridin-4-yl)-1H-benzo[d]imidazol-4-amine (70 mg, 0.29 mmol), and propylamine (0.11 mL, 1.43 mmol) in NMP (1 mL) was microwave heated (250 °C x 20 min). The mixture was poured into water, extracted with ethyl acetate, washed with brine and dried. The residue was purified by prep-TLC to obtain 2-(2-(propylamino)pyridin-4-yl)-1H-benzo[d]imidazol-4-amine as a yellow solid (48 mg, 62% yield). MS (ESI) calcd for MS (ESI) calcd for $C_{15}H_{17}N_5$: 267; found: 268 [M+H].

Step 2) Preparation of *tert*-butyl 4-(4-(2-(2-(propylamino)pyridin-4-yl)-1H-benzo[d]imidazol-4-ylcarbamoyl)benzyl)piperazine-1-carboxylate:

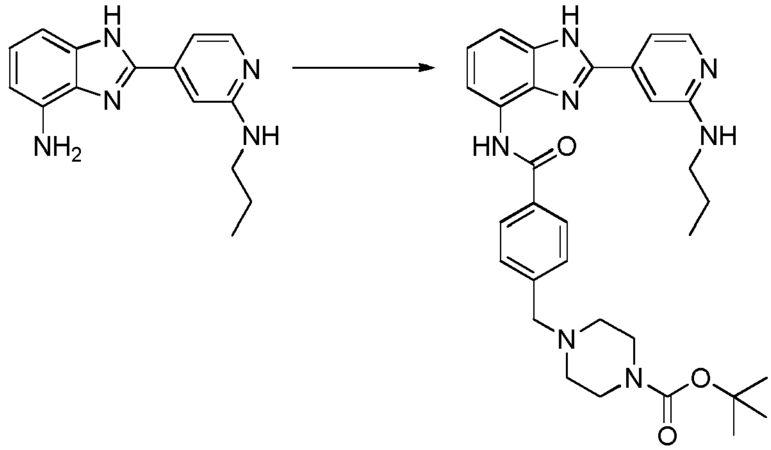

To a solution of 2-(2-(propylamino)pyridin-4-yl)-1H-benzo[d]imidazol-4-amine (28 mg, 0.1 mmol), 4-((4-(tert-butoxycarbonyl)piperazin-1-yl)methyl)benzoic acid (20.2 mg, 0.13 mmol), and HATU (60 mg, 0.16 mmol) in DMF (5 mL) was added N,N'-diisopropylethylamine (0.05 mL, 0.31 mmol). The mixture was stirred 18h at room temperature, diluted with water, and the resulting precipitate was collected by filtration and dried. The solid was purified by prep-TLC (6.25% MeOH in $CH_2Cl_2$) to obtain *tert*-butyl 4-(4-(2-(2-(propylamino)pyridin-4-yl)-1H-benzo[d]imidazol-4-ylcarbamoyl)benzyl)piperazine-1-carboxylate as a yellow solid (30 mg, 53% yield). MS (ESI) calcd for MS (ESI) calcd for $C_{32}H_{39}N_7O_3$: 569; found: 570 [M+H].

Step 3) Preparation of 4-(piperazin-1-ylmethyl)-N-(2-(2-(propylamino)pyridin-4-yl)-1H-benzo[d]imidazol-4-yl)benzamide dihydrochloride **(XVI):**

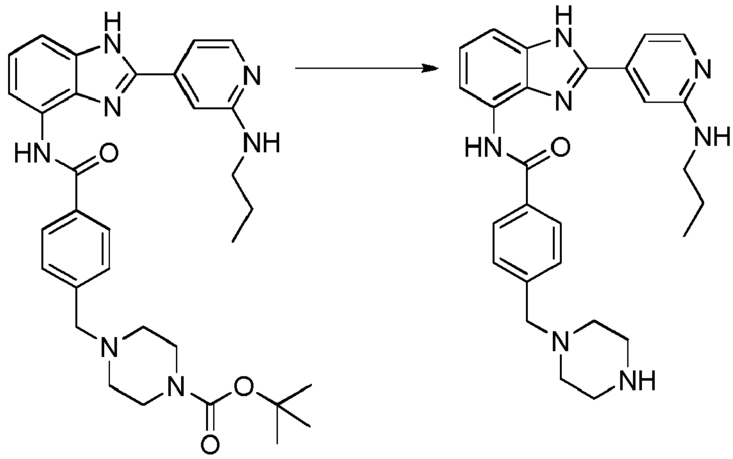

XVI

A solution of *tert*-butyl 4-(4-(2-(2-(propylamino)pyridin-4-yl)-1H-benzo[d]imidazol-4-ylcarbamoyl)benzyl)piperazine-1-carboxylate (30 mg, 0.05 mmol) in HCl/MeOH (2 mL) was stirred for 0.5h. The precipitate that formed was collected by filtration, washed with acetone and dried to obtain 4-(piperazin-1-ylmethyl)-N-(2-(2-(propylamino)pyridin-4-yl)-1H-benzo[d]imidazol-4-yl)benzamide dihydrochloride as the HCl salt. (20 mg, 97% yield). MS (ESI) calcd for MS (ESI) calcd for $C_{27}H_{31}N_7O$: 469; found: 471 [M+H].

**Example 20 Preparation of *N*-(2-(2-(propylamino)pyridin-4-yl)-1H-benzo [d] imidazol-4-yl)-3-(pyrrolidin-3-yl)benzamide hydrochloride (XV):**

Step 1) Preparation of *tert*-butyl 3-(3-(2-(2-chloropyridin-4-yl)-1H-benzo[d]imidazol-4-ylcarbamoyl)phenyl)pyrrolidine-1-carboxylate:

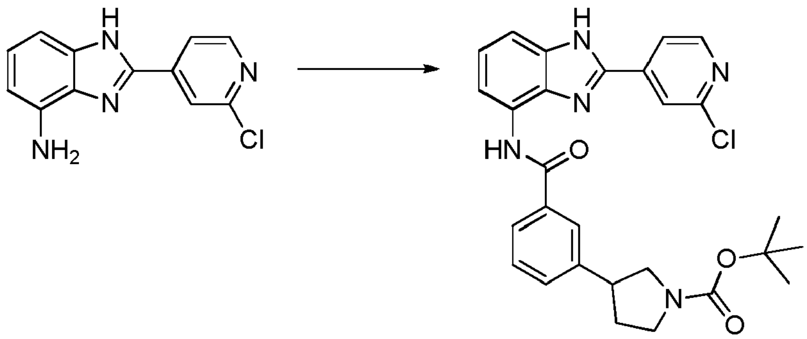

To a solution of 2-(2-chloropyridin-4-yl)-1H-benzo[d]imidazol-4-amine (200 mg, 0.82 mmol), 3-(1-(*tert*-butoxycarbonyl)pyrrolidin-3-yl)benzoic acid (262 mg, 0.9 mmol), and HATU (466 mg, 1.23 mmol) in DMF (5 mL) was added *N*,*N'*-diisopropylethylamine (0.4 mL, 2.45 mmol). The mixture was stirred 18h at room temperature, diluted with water, and the resulting precipitate was collected by filtration. The solid was purified by silica gel column chromatography (25% to 50% ethyl acetate in petroleum ether) to obtain *tert*-butyl 3-(3-(2-(2-chloropyridin-4-yl)-1H-benzo[d]imidazol-4-ylcarbamoyl)phenyl)pyrrolidine-1-carboxylate as a yellow solid (345 mg, 81% yield). ). MS (ESI) calcd for MS (ESI) calcd for $C_{28}H_{28}ClN_5O_3$: 517; found: 518 [M+H].

Step 2) Preparation of *tert*-butyl 3-(3-(2-(2-(propylamino)pyridin-4-yl)-1H-benzo[d]imidazol-4-ylcarbamoyl)phenyl)pyrrolidine-1-carboxylate:

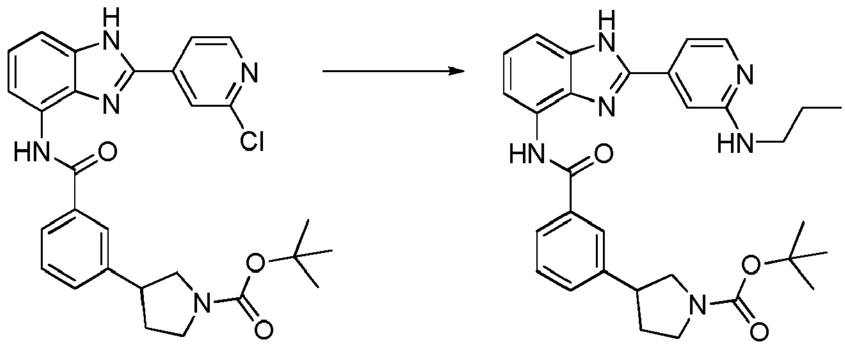

A solution of *tert*-butyl 3-(3-(2-(2-chloropyridin-4-yl)-1H-benzo[d]imidazol-4-ylcarbamoyl)phenyl)pyrrolidine-1-carboxylate (100 mg, 0.19 mmol), and propylamine (0.16 mL, 1.93 mmol) in DMSO (2 mL) in a sealed tube was heated 140°C for 24h. The reaction mixture was poured into water, extracted with $CH_2Cl_2$, and the organic phase was washed with water, dried and concentrated to dryness. The residue was purified by prep-tlc to obtain *tert*-butyl 3-(3-(2-(2-(propylamino)pyridin-4-yl)-1H-benzo[d]imidazol-4-ylcarbamoyl)phenyl)pyrrolidine-1-carboxylate as a brown solid (31 mg, 38% yield). MS (ESI) calcd for MS (ESI) calcd for $C_{31}H_{36}N_6O_3$: 540; found: 541 [M+H].

Step 3) Preparation of *N*-(2-(2-(propylamino)pyridin-4-yl)-1H-benzo[d]imidazol-4-yl)-3-(pyrrolidin-3-yl)benzamide hydrochloride **(XV):**

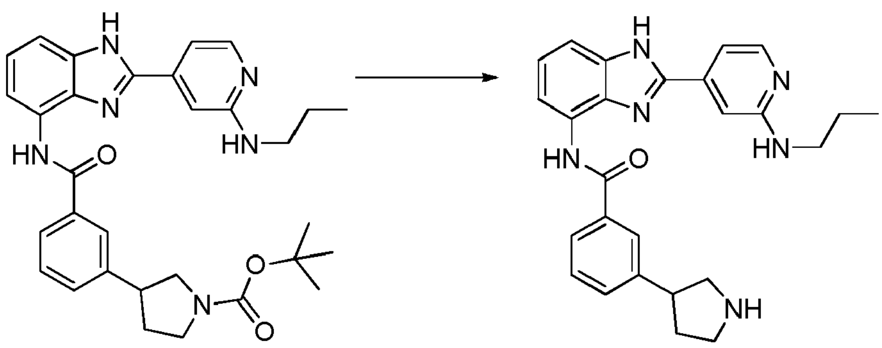

XV

A solution of tert-butyl 3-(3-(2-(2-(propylamino)pyridin-4-yl)-1H-benzo[d]imidazol-4-ylcarbamoyl)phenyl)pyrrolidine-1-carboxylate (39 mg, 0.07 mmol) in HCl/MeOH (2 mL) was stirred for 0.5 h. The mixture was concentrated and the residue was triturated with acetone to obtain *N*-(2-(2-(propylamino)pyridin-4-yl)-1H-benzo[d]imidazol-4-yl)-3-(pyrrolidin-3-yl)benzamide hydrochloride as the HCl salt. (30 mg, 94% yield). MS (ESI) calcd for $C_{26}H_{28}N_6O$: 440; found: 442 [M+H].

**Example 21 Biological activity**

A mass spectrometry based assay was used to identify modulators of SIRTI activity. The mass spectrometry based assay utilizes a peptide having 20 amino acid residues as follows: Ac-EE-K(biotin)-GQSTSSHSK(Ac)NleSTEG-K(5TMR)-EE-$NH_2$ (SEQ ID NO: 1) wherein K(Ac) is an acetylated lysine residue and Nle is a norleucine. The peptide is labeled with the fluorophore 5TMR (excitation 540 nrnlemission 580 nm) at the C-terminus. The sequence of the peptide substrate is based on p53 with several modifications. In addition, the methionine residue naturally present in the sequence was replaced with the norleucine because the methionine may be susceptible to oxidation during synthesis and purification.

The mass spectrometry assay is conducted as follows: 0.5 J-1M peptide substrate and 120 μM $\beta NAD^+$ is incubated with 10 nM SIRTI for 25 minutes at 25°C in a reaction buffer (50 mM Tris-acetate pH 8, 137 mM NaCl, 2.7 mM KCl, 1 mM MgClz, 5 mM DTT, 0.05% BSA). Test compounds may be added to the reaction as described above. The SirTI gene is cloned into a T7-promoter containing vector and transformed into BL21 (DE3). After the 25 minute incubation with SIRT1, 10 μL of 10% formic acid is added to stop the reaction. Reactions are sealed and frozen for later mass spec analysis. Determination of the mass of the substrate peptide allows for precise determination of the degree of acetylation (i.e.starting material) as compared to deacetylated peptide (product).

A control for inhibition of sirtuin activity is conducted by adding 1 μL of 500 mM nicotinamide as a negative control at the start of the reaction (e.g., permits determination of maximum sirtuin inhibition). A control for activation of sirtuin activity is conducted using 10 nM of sirtuin protein, with 1□ μL of DMSO in place of compound, to determine the amount of deacetylation of the substrate at a given timepoint within the linear range of the assay. This timepoint is the same as that used for test compounds and, within the linear range, the endpoint represents a change in velocity.

For the above assay, SIRTI protein was expressed and purified as follows. The SirT1 gene was cloned into a T7-promoter containing vector and transformed into BL21 (DE3). The protein was expressed by induction with 1 mM IPTG as an N-terminal His-tag fusion protein at 18 °C overnight and harvested at 30,000 x g. Cells were lysed with lysozyme in lysis buffer (50 mM Tris-HCl, 2 mM Tris[2-carboxyethyl] phosphine (TCEP), 10 μM $ZnCl_2$, 200 mM NaCl) and further treated with sonication for 10 min for complete lysis. The protein was purified over a Ni-NTA column (Amersham) and fractions containing pure protein were pooled, concentrated and run over a sizing column (Sephadex S200 26/60 global). The peak containing soluble protein was collected and run on an Ion-exchange column (MonoQ). Gradient elution (200 mM - 500 mM NaCl) yielded pure 20 protein. This protein was concentrated and dialyzed against dialysis buffer (20 mM TrisHCl, 2 mM TCEP) overnight. The protein was aliquoted and frozen at -80 °C until further use.

Sirtuin modulating compounds that activated SIRT1 were identified using the assay described above and are shown below in Table 4. The $EC_{1.5}$ values represent the concentration of test compounds that result in 150% activation of SIRT1. The $EC_{1.5}$ values for the activating compounds are represented by A ($EC_{1.5}$ <1 μM), B ($EC_{1.5}$ > 1 μM),. The percent maximum fold activation is represented by A (Fold activation ≥200%) or B (Fold Activation <200%).

Table 6

| COMPOUND NO. | [M+H]+ | STRUCTURE | EC1.5 uM | % FOLD ACT. |
|---|---|---|---|---|
| 1 (I) | 495 | 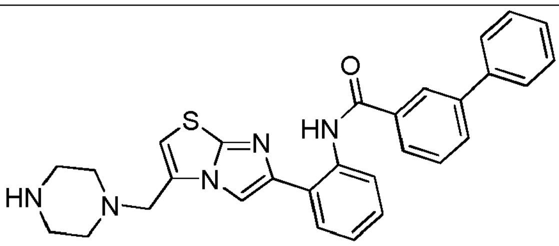 | A | A |
| 2 (II) | 442 | 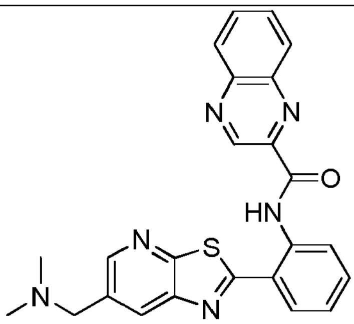 | B | B |

| | | | | |
|---|---|---|---|---|
| 3 (III) | 461 | 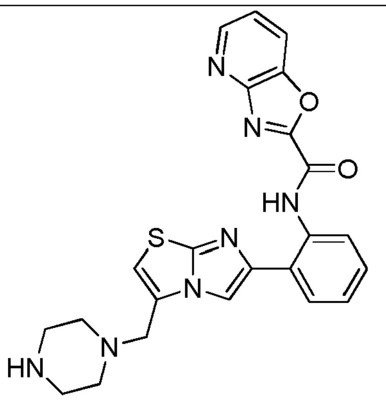 | B | B |
| 4 (IV) | 529 | 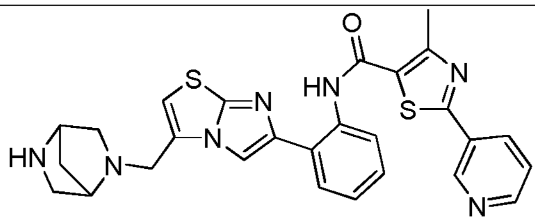 | A | A |
| 5 (V) | 559 | 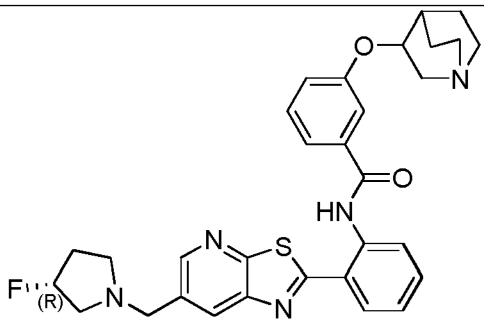 | B | A |
| 6 (VI) | 480 | 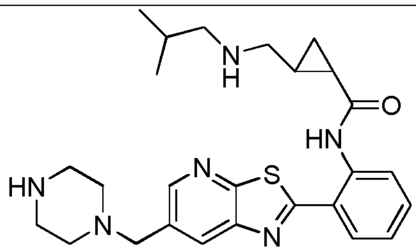 | B | |
| 7 (VII) | 513 | 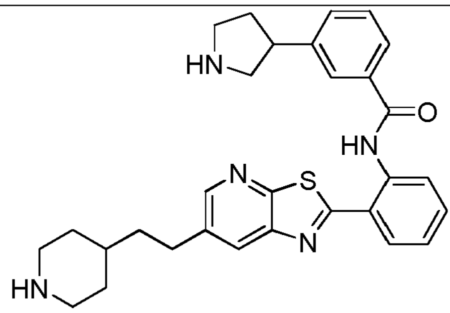 | A | A |

| | | | | |
|---|---|---|---|---|
| 8 (VIII) | 506 | 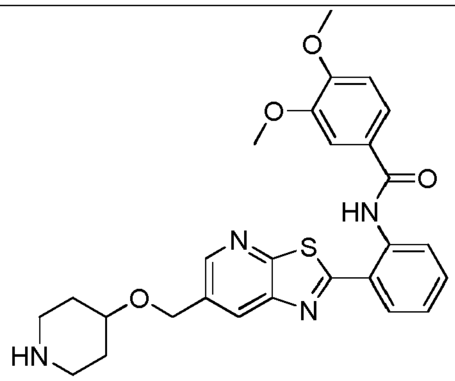 | A | A |
| 9 (IX) | 546 | 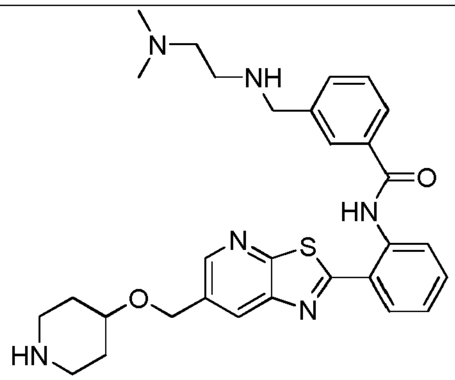 | B | B |
| 10 (X) | 602 | 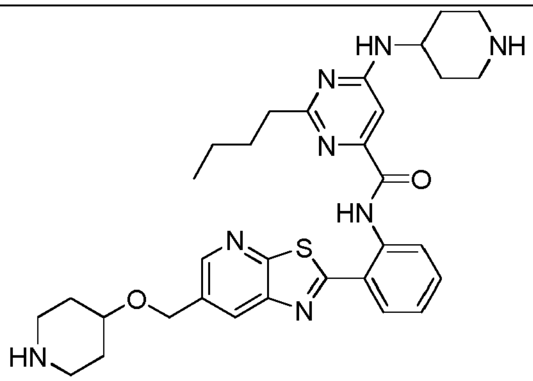 | A | A |
| 11 (XI) | 602 | 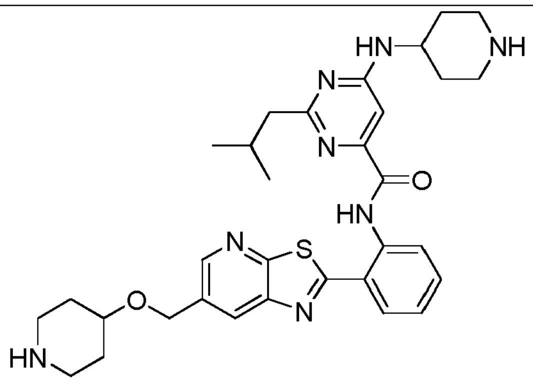 | A | A |

| | | | | |
|---|---|---|---|---|
| 12 (XII) | 576 | 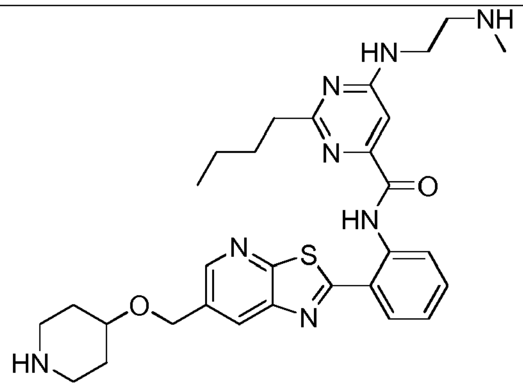 | A | A |
| 13 (XIII) | 471 | 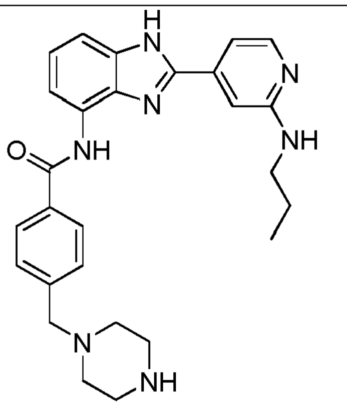 | A | A |
| 14 (XIV) | 514 | 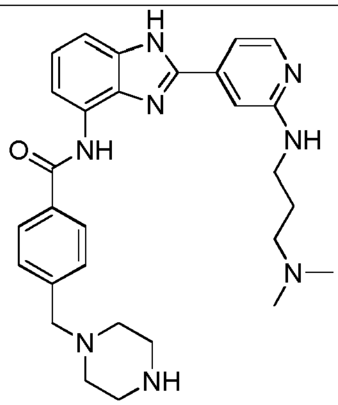 | A | A |
| 15 (XV) | 442 | 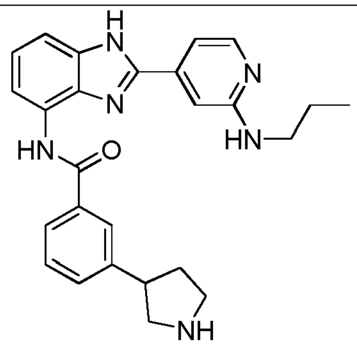 | A | A |

| | | | | |
|---|---|---|---|---|
| 16 (XVI) | 471 | 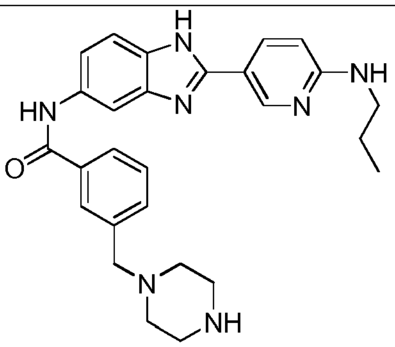 | B | B |
| 17 (XVII) | 514 | 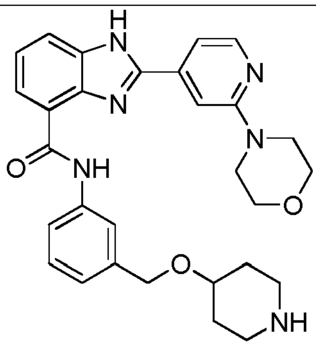 | A | A |
| 18 (XVIII) | 514 | 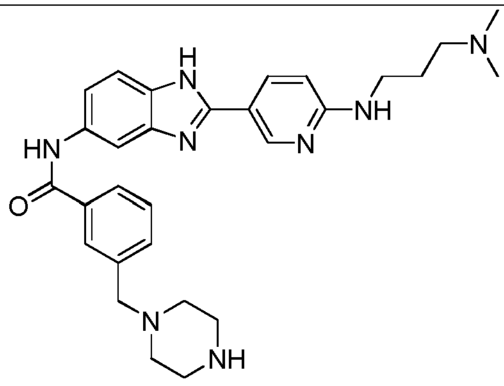 | B | B |
| 19 (XIX) | 487 | 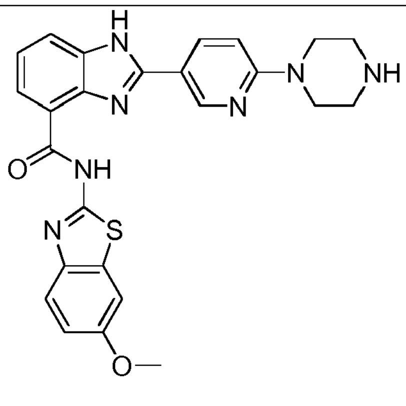 | A | A |

| | | | | |
|---|---|---|---|---|
| 20 (XX) | 493 | 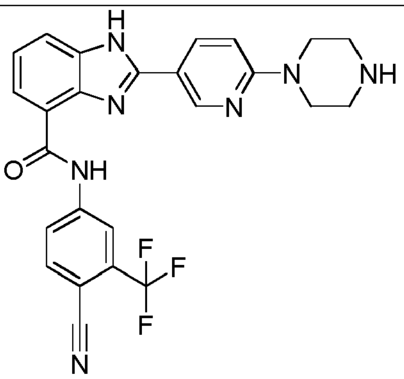 | A | A |
| 21 (XXI) | 556 | 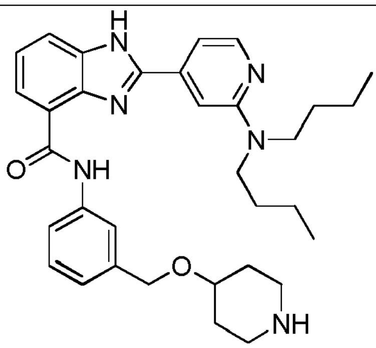 | A | A |

**EQUIVALENTS**

The present invention provides among other things sirtuin-activating compounds and methods of use thereof. While specific embodiments of the subject invention have been discussed, the above specification is illustrative and not restrictive. Many variations of the invention will become apparent to those skilled in the art upon review of this specification. The full scope of the invention should be determined by reference to the claims, along with their full scope of equivalents, and the specification, along with such variations.

**INCORPORATION BY REFERENCE**

All publications and patents mentioned herein are hereby incorporated by reference in their entirety as if each individual publication or patent was specifically and individually indicated to be incorporated by reference. In case of conflict, the present application, including any definitions herein, will control.

Also incorporated by reference in their entirety are any polynucleotide and polypeptide sequences which reference an accession number correlating to an entry in a public database, such as those maintained by The Institute for Genomic Research (TIGR) (www.tigr.org) and/or the National Center for Biotechnology Information (NCBI) (www.ncbi.nlm.nih.gov).

The invention can be further defined as in the numbered sentences below:

1. A method of detecting a compound that activates a sirtuin deacetylase activity on a fluorescent-free activation substrate *in vitro* comprising:

   contacting a sirtuin deacetylase with a candidate compound and a fluorescent-free activation substrate comprising an acetylated peptide, polypeptide, or protein substrate of the sirtuin;

   detecting the level of sirtuin deacetylase activity on the fluorescent-free activation substrate in the presence of the candidate compound; and

   comparing the level of sirtuin deacetylase activity on the fluorescent-free activation substrate in the presence of the candidate compound to the level of sirtuin deacetylase activity on the fluorescent-free activation substrate

in the absence of the candidate compound,

wherein an increase in the level of sirtuin deacetylase activity on the fluorescent-free activation substrate in the presence of the candidate compound compared to the level of sirtuin deacetylase activity on the fluorescent-free activation substrate in the absence of the candidate compound indicates that the compound is a sirtuin activator.

2. The method of claim 1, wherein the sirtuin deacetylase is SIRT1.

3. The method of claim 1, wherein the sirtuin deacetylase is selected from the group consisting of SIRT2, SIRT3, SIRT4 and SIRT5.

4. The method of sentence 1, wherein the candidate compound is a SIRT1 activator of an acetylated peptide or polypeptide substrate containing a fluorescent group.

5. The method of sentence 4, wherein the fluorescent group-containing peptide substrate is the TAMRA-peptide: Ac-EEK$^{(biotin)}$GQSTSSHSK$^{Ac}$NleSTEGK$^{(5\text{-}TMR)}$EE-$NH_2$.

6. The method of sentence 1, wherein the sirtuin deacetylase is contacted with a fluorescent-free activation substrate and a candidate compound in the presence of NAD.

7. The method of sentence 1, wherein the sirtuin deacetylase is contacted with a fluorescent-free activation substrate and a candidate compound in the presence of a hydrolysable NAD analog.

8. The method of sentence 1, wherein the fluorescent-free activation substrate is a biotinylated polypeptide.

9. The method of sentence 1, wherein the fluorescent-free activation substrate is the desTAMRA-peptide: Ac-EEK$^{(biotin)}$GQSTSSHSK$^{Ac}$NleSTEGKEE- $NH_2$.

10. The method of sentence 1, wherein the fluorescent-free activation substrate is an acetylated peptide or polypeptide free of the fluorescent groups TAMRA (tetramethyl-6-carboxyrhodamine) and AMC (7-amido-4-methyl coumarin).

11. The method of sentence 1, wherein the fluorescent-free activation substrate is an acetylated peptide selected from the group consisting of Ac-RHKK$^{Ac}$F-$NH_2$ and Ac-RHKK$^{Ac}$W-$NH_2$.

12. The method of sentence 1, wherein the fluorescent-free activation substrate comprises an acetylated peptide, polypeptide, or protein substrate of the sirtuin and an activation cofactor-bearing accessory protein.

13. The method of sentence 12, wherein the activation cofactor-bearing accessory protein is selected from the group consisting of DBC1 (deleted in breast cancer 1), HIC1 (hypermethylated in cancer 1), AROS (active regulator of SIRT1), and CLOCK.

14. The method of sentence 1, wherein the acetylated protein is selected from the group consisting of histone H1, histone H3, histone H4, p53, p300, FOXO 1, FOXO 3a, FOXO 4, p65, HIVTat, PGC-1$\alpha$, PCAF, MyoD, PPAR$\gamma$, and Ku70.

15. The method of sentence 1, wherein the level of sirtuin deacetylase activity is detected by measuring the rate of NAD hydrolysis.

16. The method of sentence 1, wherein the level of sirtuin deacetylase activity is detected by measuring the rate of fluorescent-free activation substrate deacetylation.

17. The method of sentence 1, wherein the compound is an activator of SIRT1 deacetylation of a fluorescent-free activation substrate.

18. The method of sentence 17, wherein the compound has the formula:

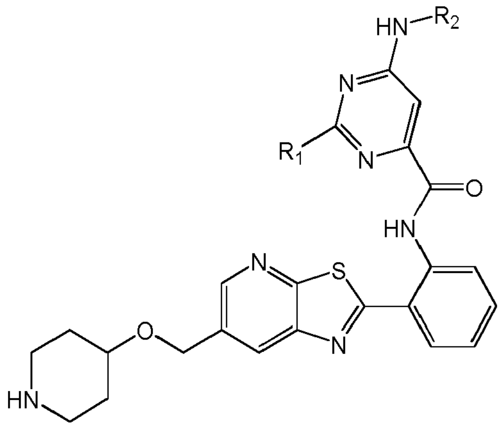

(XL),

or a salt thereof, wherein:

$R_1$ is selected from $-(CH_2)_3-CH_3$, and $-(CH_2)CH(CH_3)_2$; and

$R_2$ is selected from -piperidine and $-(CH_2)_2-NH-CH_3$.

19. The method of sentence 18, wherein the compound, or salt thereof, is selected from the group consisting of:

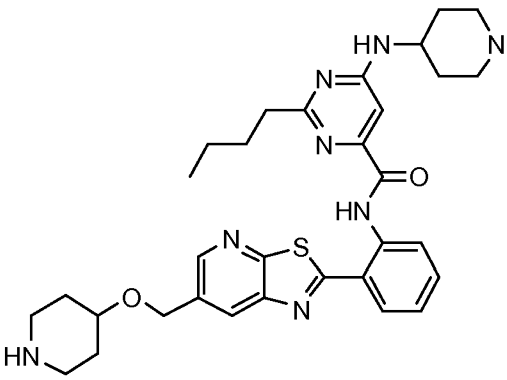

(XI),

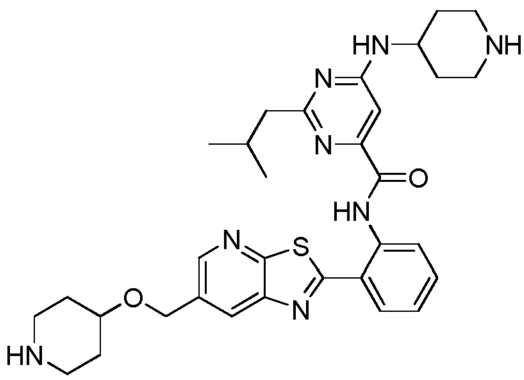

(XII), and

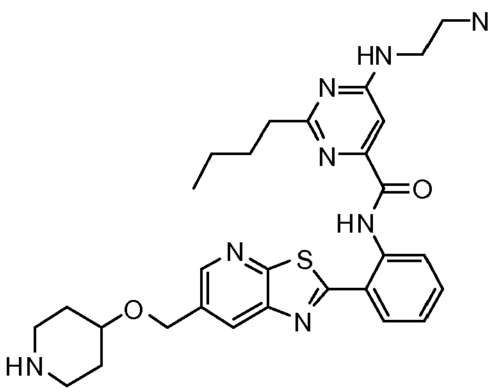

(XIII).

20. The method of sentence 1, further comprising:

selecting a candidate compound that is a SIRT1 activator, and

contacting the SIRT1 activator with a test cell and detecting a SIRT1 activation-specific change in the test cell.

21. The method of sentence 20, wherein the SIRT1 activation-specific change is an increase in FGF21 production.

22. The method of sentence 20, wherein the SIRT1 activation-specific change is a decrease in LPS-induced TNFα production.

23. The method of sentence 1, further comprising:

selecting a candidate compound that is a SIRT1 activator, and

administering the SIRT1 activator to a test subject and detecting a SIRT1 activation-specific change in the test subject.

24. The method of sentence 23, wherein the test subject is a diabetic model mouse and the SIRT1 activation specific change is a lowering of blood glucose.

25. The method of sentence 23, wherein the test subject is a neurodegenerative disease model mouse and the SIRT1 activation specific change is a decrease in a neurodegenerative disease process or marker.

26. The method of sentence 25, wherein the neurodegenerative disease is selected from the group consisting of Alzheimer's, Huntington's, amyotrophic lateral sclerosis (ALS), Parkinson's disease, Huntington's disease, and multiple sclerosis (MS).

27. A fluorescent-free sirtuin substrate having a structural formula selected from the group consisting of Ac-RHKK$^{Ac}$F-$NH_2$ and Ac-RHKK$^{Ac}$W-$NH_2$.

28. A compound of the formula (I):

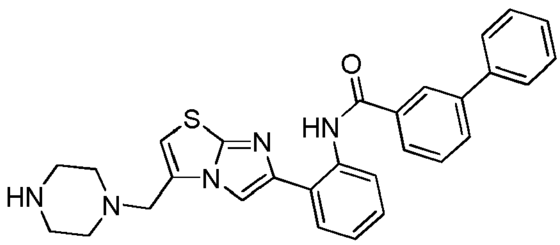

(I),

or a salt thereof.

29. A compound of the formula (II):

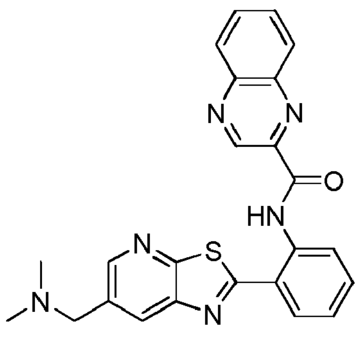

(II),

or a salt thereof.

30. A compound of the formula (III):

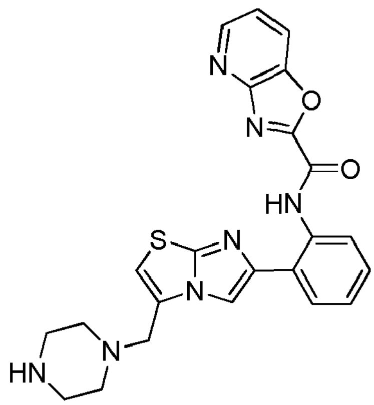

(III),

or a salt thereof.

31. A compound of the formula (IV):

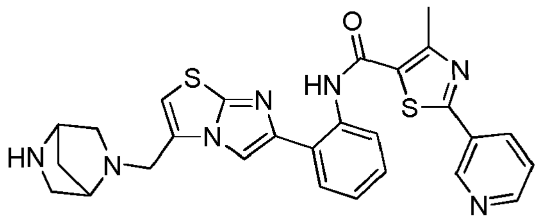

(IV),

or a salt thereof.

32. A compound of the formula (V):

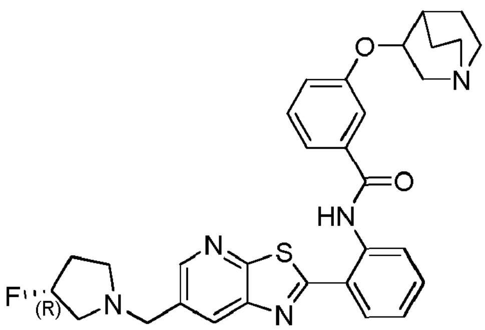

(V),

or a salt thereof.

33. A compound of the formula (VI):

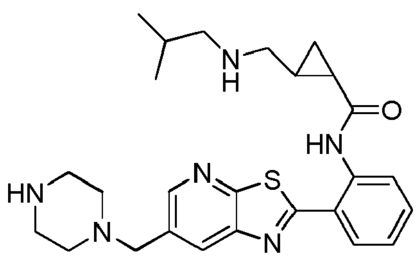

(VI),

or a salt thereof.

34. A compound of the formula (VII):

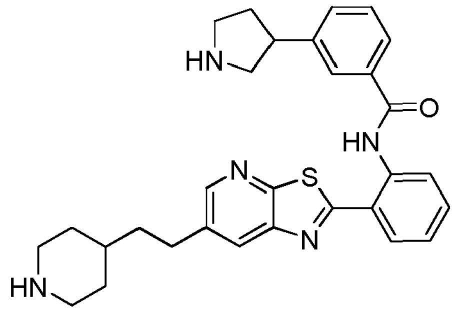

(VII),

or a salt thereof.

35. A compound of the formula (VIII):

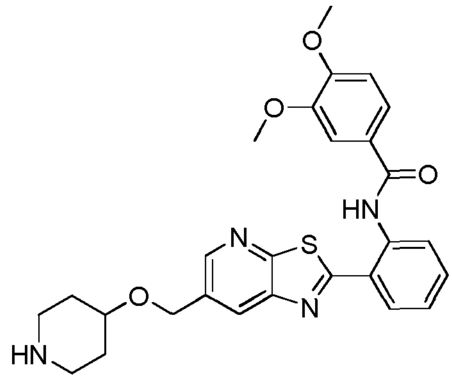

(VIII),

or a salt thereof.

36. A compound of the formula (IX):

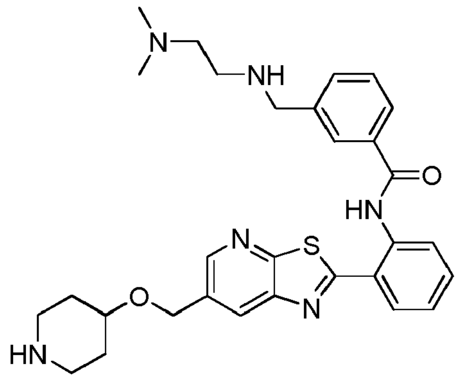

(IX),

or a salt thereof.

37. A compound of the formula (X):

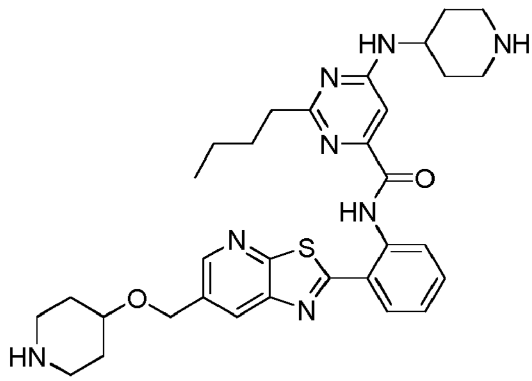

(X),

38. A pharmaceutical composition comprising a compound of any one of sentences 28 to 37, or a pharmaceutically acceptable salt thereof; and a pharmaceutically acceptable carrier.

39. The pharmaceutical composition of sentence 38, further comprising an additional active agent.

40. A method for treating a subject suffering from or susceptible to insulin resistance, a metabolic syndrome, diabetes, or complications thereof, or for increasing insulin sensitivity in a subject, comprising administering to the subject in need thereof a composition of sentence 38.

41. A compound of the formula (XXXI):

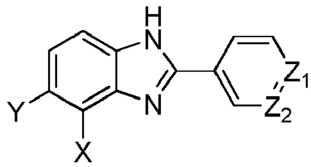

(XXXI),

or salt thereof wherein:

one of X and Y is selected from-NH-C(=O)-$R^1$ or -C(=O)-NH-$R^1$, and the other of X and Y is H, wherein

$R^1$ is selected from phenyl or a fused hetereocycle, and $R^1$ is optionally substituted with one to two substituents independently selected from -C≡N, $C_1$-$C_2$ fluoro-substituted alkyl and -($C_0$-$C_2$ alkyl)-saturated hetereocycle,

and when $R^1$ is phenyl, $R^1$ is optionally substituted with -$CH_2$-O-saturated hetereocycle, wherein the saturated hetereocycle is selected from piperidine, pyrrolidine or piperazine,

and when $R^1$ is selected from a fused hetereocycle, the fused hetereocycle is selected from benzo[d]thiazole and $R^1$ is also optionally substituted with -$OCH_3$; and

one of $Z^1$ and $Z^2$ is N, and the other is $CR^2$, wherein $R^2$ is selected from -NH($C_1$-$C_3$ alkyl), -NH($C_1$-$C_3$ alkyl)-N($CH_3$)$_2$, -($C_0$-$C_2$ alkyl)-morpholine, -($C_0$-$C_2$ alkyl)-piperazine or -N($C_1$-$C_4$ alkyl)$_2$, wherein when $R^2$ is -($C_0$-$C_2$ alkyl)-saturated hetereocycle, the saturated heterocycle is selected from morpholine or piperazine.

42. The compound of sentence 41 having formula (XXXI), or a salt thereof, selected from the group consisting of:

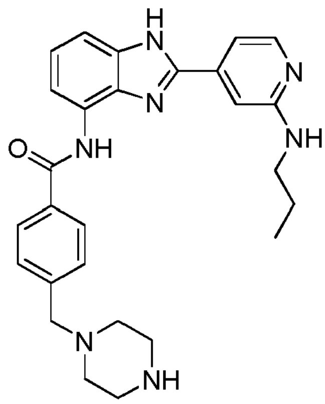 (XIII),

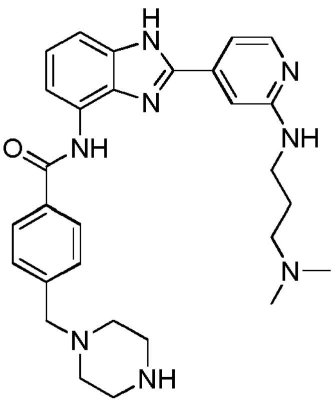 (XIV),

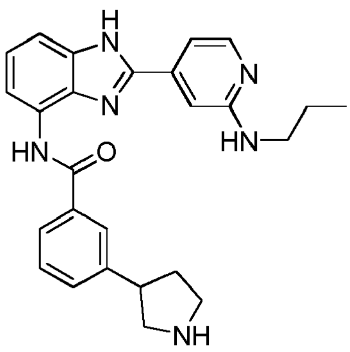 (XV),

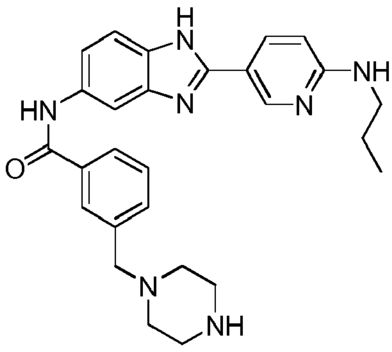 (XVI),

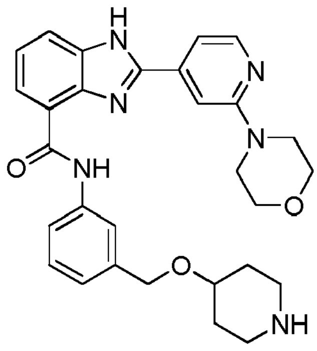 (XVII),

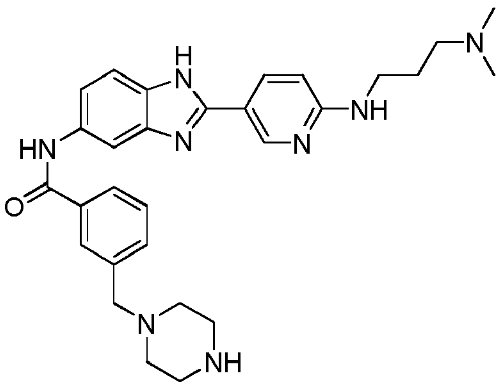 (XVIII),

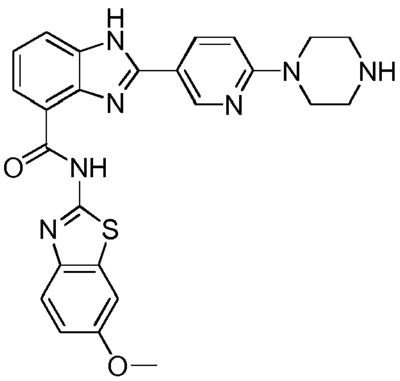 (XIX),

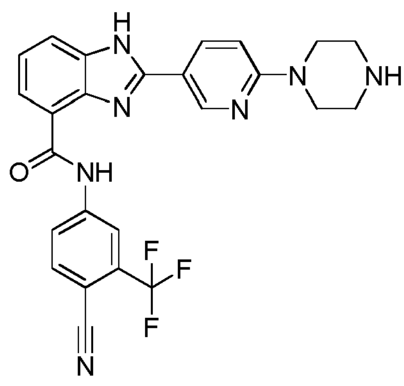 (XX),

and

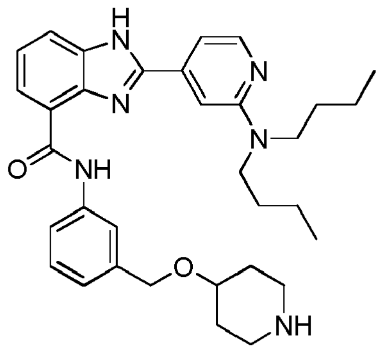

(XXI).

43. A compound of the formula (XXXII):

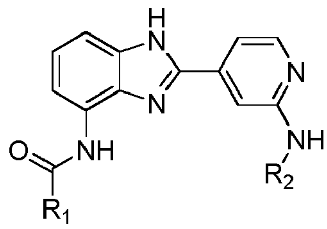

(XXXII),

or salt thereof wherein:

$R^1$ is phenyl optionally substituted with -($C_0$-$C_2$ alkyl)-pyrrolidine or -($C_0$-$C_2$ alkyl)-piperazine; and

$R^2$ is selected from -$C_1$-$C_3$ alkyl or -($C_1$-$C_3$ alkyl)-$N(CH_3)_2$.

44. The compound of sentence 43 having formula (XXXII), or a salt thereof, selected from the group consisting of:

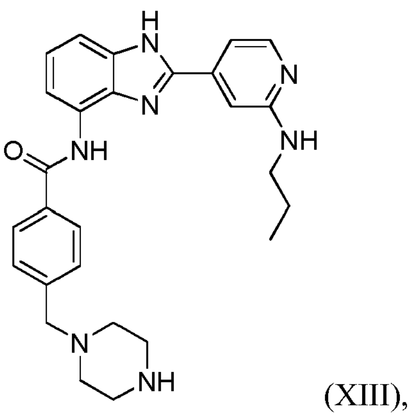

(XIII),

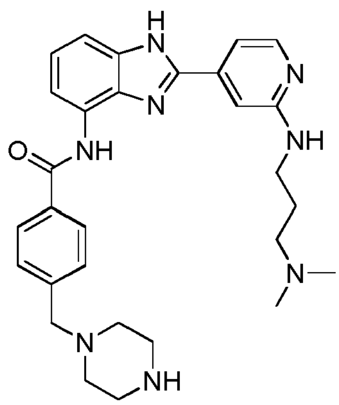

(XIV),

and

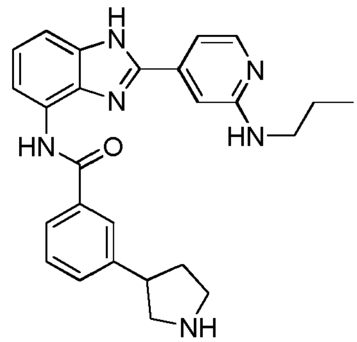

(XV).

45. A compound of the formula (XXXIII):

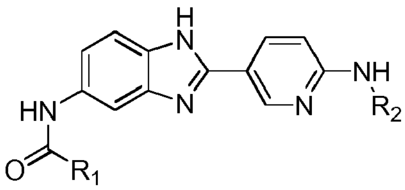

(XXXIII),

or salt thereof wherein:

$R^1$ is phenyl, optionally substituted with $-(C_0-C_2$ alkyl)-piperazine; and

$R^2$ is selected from $-C_1-C_3$ alkyl and $-(C_1-C_3)-N(CH_3)_2$.

46. The compound of sentence 45, having formula (XXXIII), or a salt thereof, selected from the group consisting of:

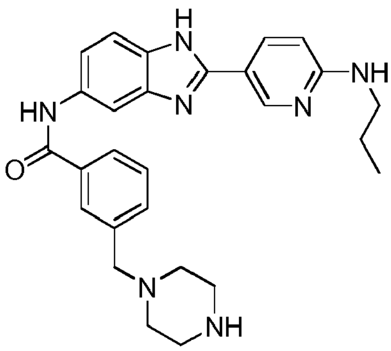

(XVI), and

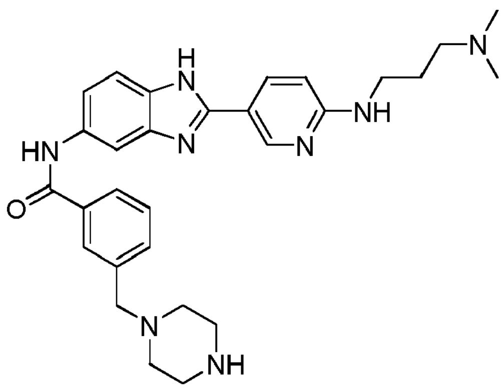

(XVIII).

47. A compound of the formula (XXXIV):

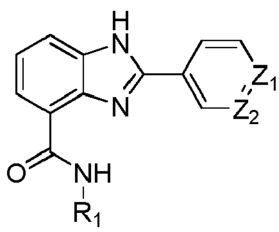

(XXXIV),

or salt thereof wherein:

one of $Z^1$ and $Z^2$ is N, and the other is selected from $CR^2$, wherein

$R^2$ is selected from -($C_0$-$C_2$ alkyl)-morpholine, -($C_0$-$C_2$ alkyl)-piperazine, and - N($C_1$-$C_4$)$_2$; and

$R^1$ is selected from phenyl and benzo[d]thiazole optionally substituted with -$OCH_3$, wherein $R^1$ is further optionally substituted with one to two substituents independently selected from-C≡N, $C_1$-$C_2$ fluoro-substituted alkyl, and when $R^1$ is phenyl, $R^1$ is further optionally substituted with -$CH_2$-O-piperidine.

48. The compound of sentence 58 having formula (XXXIV), or a salt thereof, selected from the group consisting of:

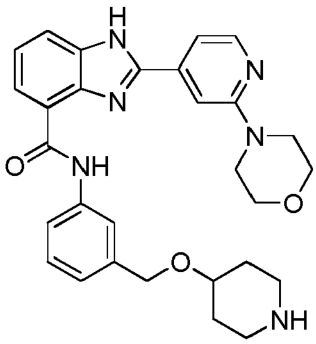
(XVII),

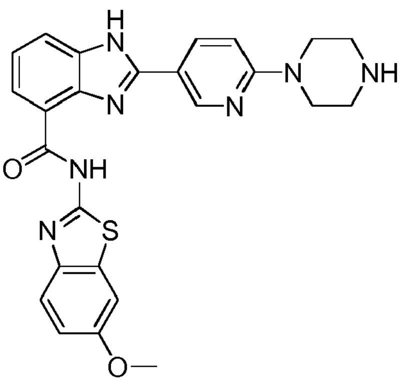
(XIX),

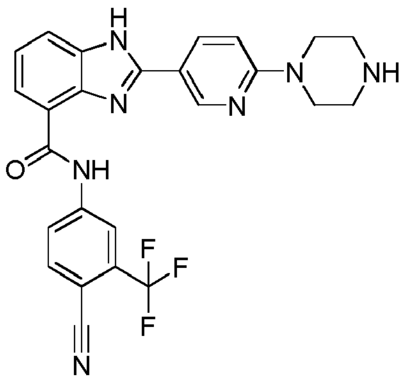
(XX), and

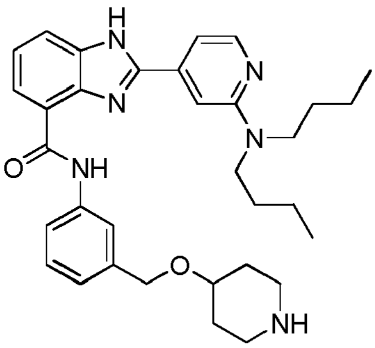
(XXI).

49. A pharmaceutical composition comprising a compound of any one of sentences 41 to 48, or a pharmaceutically acceptable salt thereof; and a pharmaceutically acceptable carrier.

50. The pharmaceutical composition of sentence 49, further comprising an additional active agent.

51. A method for treating a subject suffering from or susceptible to insulin resistance, a metabolic syndrome, diabetes, or complications thereof, or for increasing insulin sensitivity in a subject, comprising administering to the subject in need thereof a composition of sentence 50.

## Claims

1. A method of detecting a compound that activates a SIRT1 sirtuin deacetylase activity on a fluorescent-free activation substrate *in vitro* comprising:

   contacting a SIRT1 sirtuin deacetylase with a candidate compound and a fluorescent-free activation substrate comprising an acetylated peptide, polypeptide,
   or protein and an activation cofactor selected from tryptophan and phenylalanine;
   detecting the level of SIRT1 sirtuin deacetylase activity on the substrate in the presence of the candidate compound; and
   comparing the level of SIRT1 sirtuin deacetylase activity on the substrate in the presence of the candidate compound to the level of SIRT1 sirtuin deacetylase activity on the substrate in the absence of the candidate compound,

   wherein an increase in the level of SIRT1 sirtuin deacetylase activity on the substrate in the presence of the candidate compound compared to the level of SIRT1 sirtuin deacetylase activity on the substrate in the absence of the candidate compound indicates that the compound is a SIRT1 sirtuin activator.

2. The method of claim 1, wherein the candidate compound is a SIRT1 activator of an acetylated peptide or polypeptide substrate containing a fluorescent group.

3. The method of claim 1 or 2, wherein the SIRT1 sirtuin deacetylase is contacted with the substrate and a candidate compound in the presence of NAD or a hydrolysable NAD analog.

4. The method of claim 1-3, wherein the substrate is an acetylated peptide selected from the group consisting of Ac-$RHKK^{Ac}F\text{-}NH_2$ and Ac-$RHKK^{Ac}W\text{-}NH_2$.

5. The method of claim 1-3, wherein the acetylated peptide, polypeptide, or protein substrate of SIRT1 comprises an activation cofactor moiety selected from the group consisting of tryptophan or phenylalanine at +1 relative to the position of lysine acetylation.

6. The method of claim 5, wherein the acetylated peptide, polypeptide, or protein substrate of SIRT1 comprises a tryptophan activation cofactor moiety at +1 relative to the position of lysine acetylation.

7. The method of claim 1-3, wherein the substrate comprises an acetylated protein selected from the group consisting of histone H1, histone H3, histone H4, p53, p300, FOXO 1, FOXO 3a, FOXO 4, p65, HIVTat, PGC-1$\alpha$, PCAF, MyoD, PPAR$\gamma$, and Ku70.

8. The method of any preceding claim, wherein the level of SIRT1 sirtuin deacetylase activity is detected by measuring the rate of NAD hydrolysis.

**9.** The method of any of claims 1-7, wherein the level of SIRT1 sirtuin deacetylase activity is detected by measuring the rate of substrate deacetylation.

**10.** The method of any preceding claim, further comprising:

selecting a candidate compound that is a SIRT1 activator, and
contacting the SIRT1 activator with a test cell and detecting a SIRT1 activation-specific change in the test cell,

wherein the SIRT1 activation-specific change is an increase in FGF21 production or a decrease in LPS-induced TNF$\alpha$ production.

**11.** The method of any preceding claim, further comprising:

selecting a candidate compound that is a SIRT1 activator, and
administering the SIRT1 activator to a test subject and detecting a SIRT1 activation-specific change in the test subject,

wherein the test subject is a diabetic model mouse and the SIRT1 activation specific change is a lowering of blood glucose, or the test subject is a neurodegenerative disease model mouse of a neurodegenerative disease selected from the group consisting of Alzheimer's, Huntington's, amyotrophic lateral sclerosis (ALS), Parkinson's disease, Huntington's disease, and multiple sclerosis (MS) and the SIRT1 activation specific change is a decrease in a neurodegenerative disease process or marker.

**12.** A fluorescent-free sirtuin substrate having a structural formula selected from the group consisting of Ac-RHKK$^{Ac}$F-$NH_2$ and Ac-RHKK$^{Ac}$W-$NH_2$.

# FIGURE 1

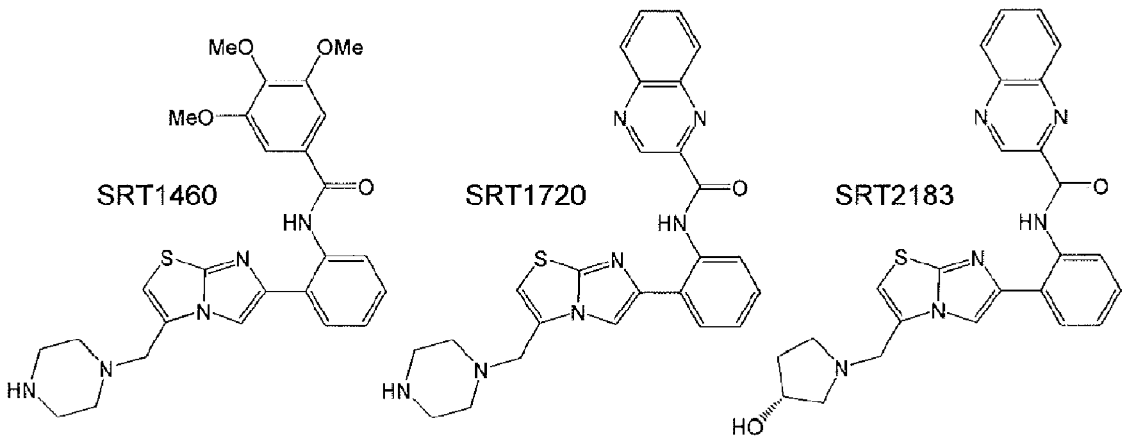

SRT1460 SRT1720 SRT2183

FIGURE 2

***A.*** *"Indirect" Mechanism*

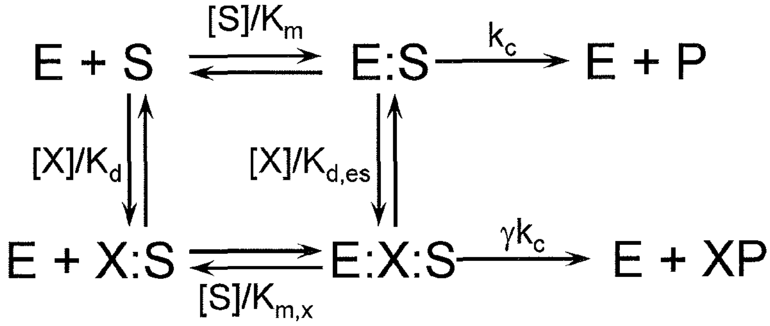


***B.*** *Allosteric Mechanism*

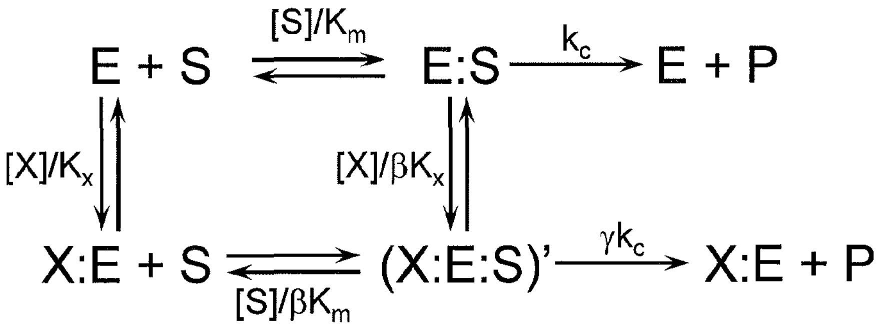

FIGURE 3

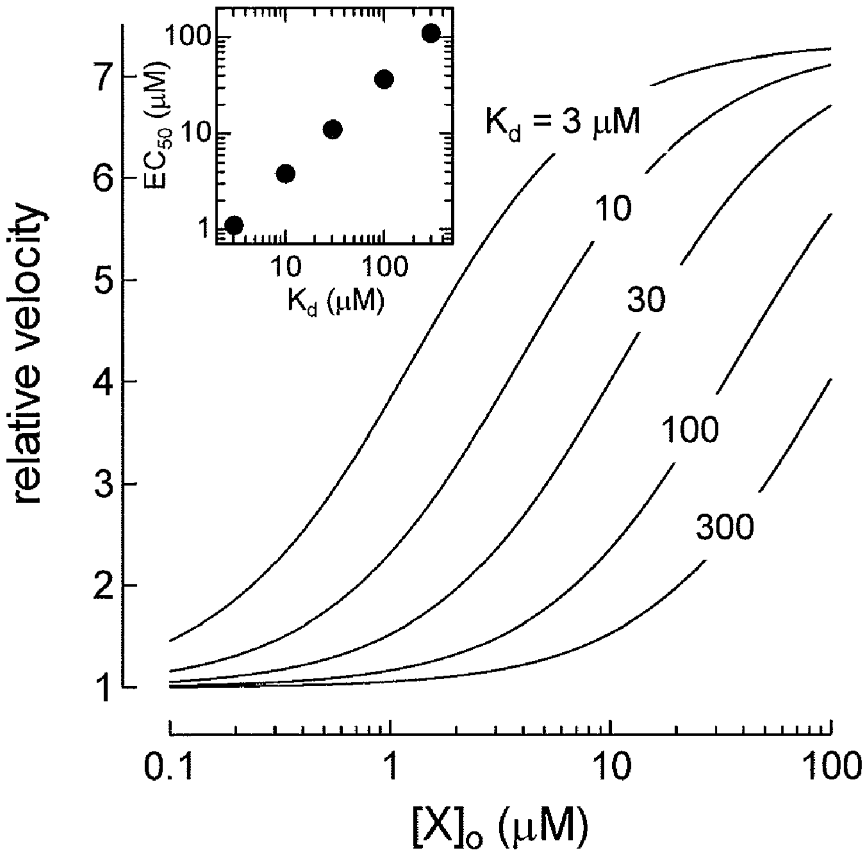

relative velocity
1
2
3
4
5
6
7
0.1
1
10
100
[X]o (μM)
Kd = 3 μM
10
30
100
300
EC50 (μM)
1
10
100
10
100
Kd (μM)

# FIGURE 4

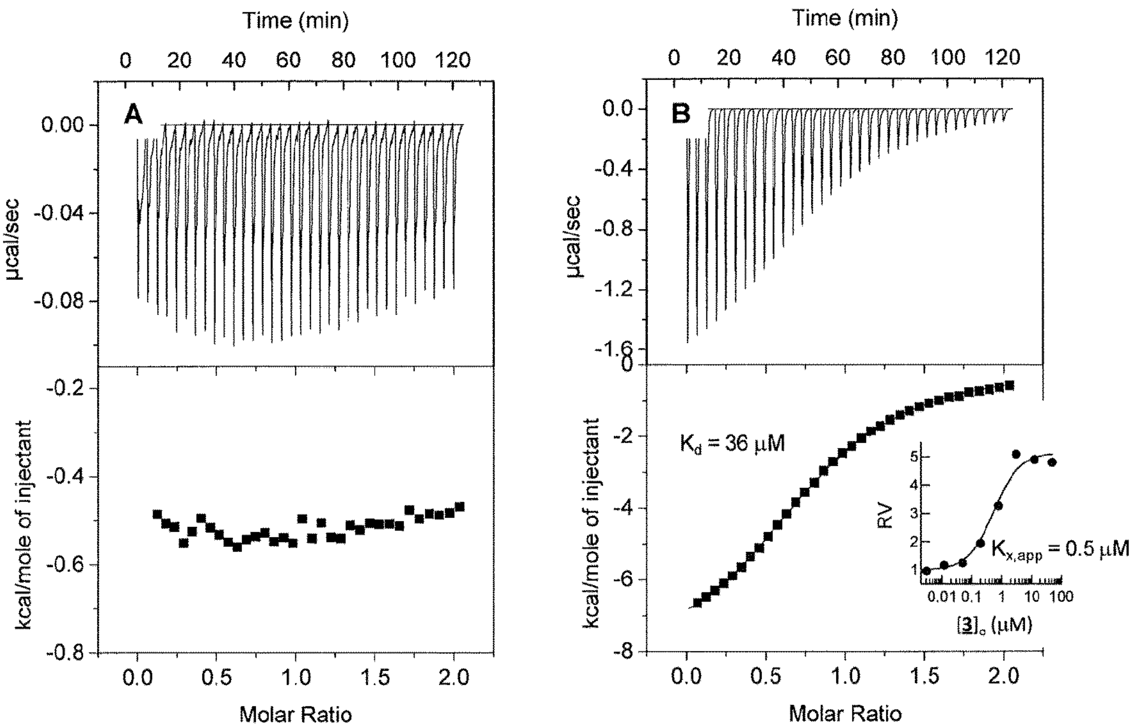
Time (min)
0 20 40 60 80 100 120
A
0.00
-0.04
-0.08
µcal/sec
-0.2
-0.4
-0.6
-0.8
kcal/mole of injectant
0.0 0.5 1.0 1.5 2.0
Molar Ratio
Time (min)
0 20 40 60 80 100 120
B
0.0
-0.4
-0.8
-1.2
-1.6
µcal/sec
0
-2
-4
-6
-8
kcal/mole of injectant
K$_d$ = 36 µM
RV
K$_{x,app}$ = 0.5 µM
0.01 0.1 1 10 100
[3]$_o$ (µM)
0.0 0.5 1.0 1.5 2.0
Molar Ratio

# FIGURE 5

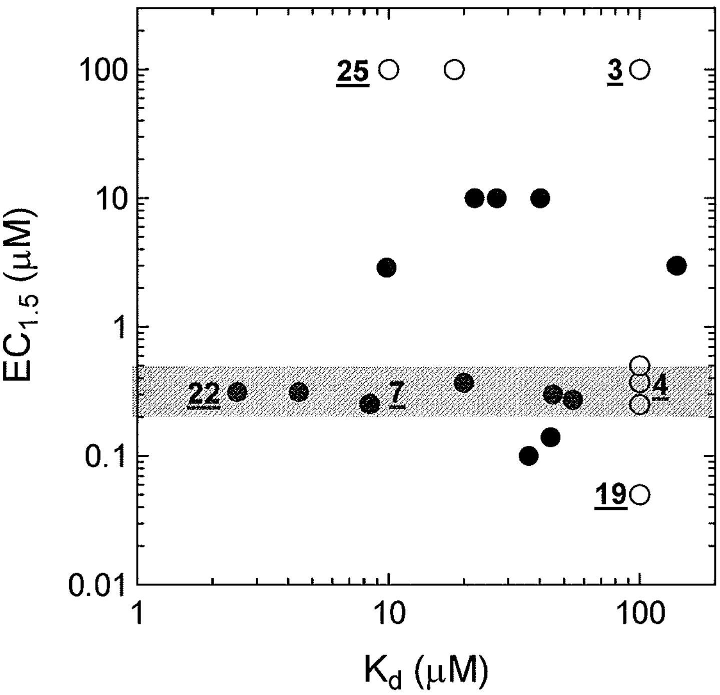


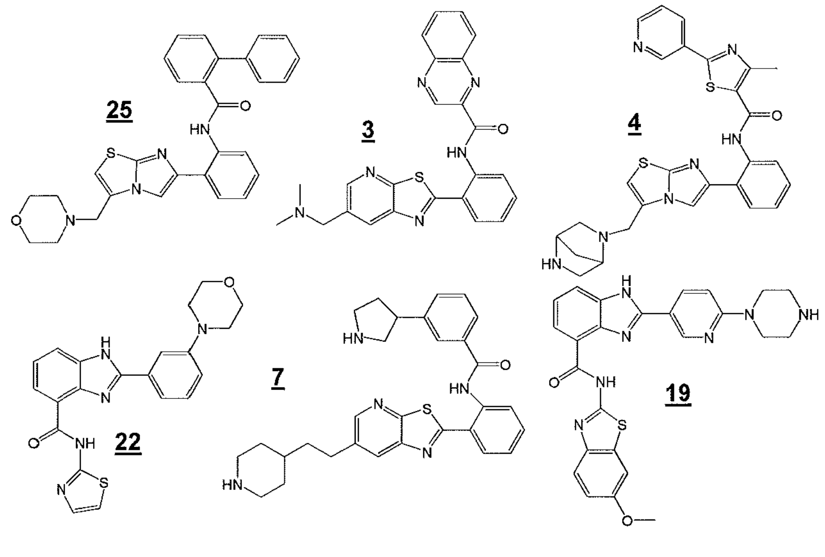

25

3

4

22

7

19

FIGURE 6

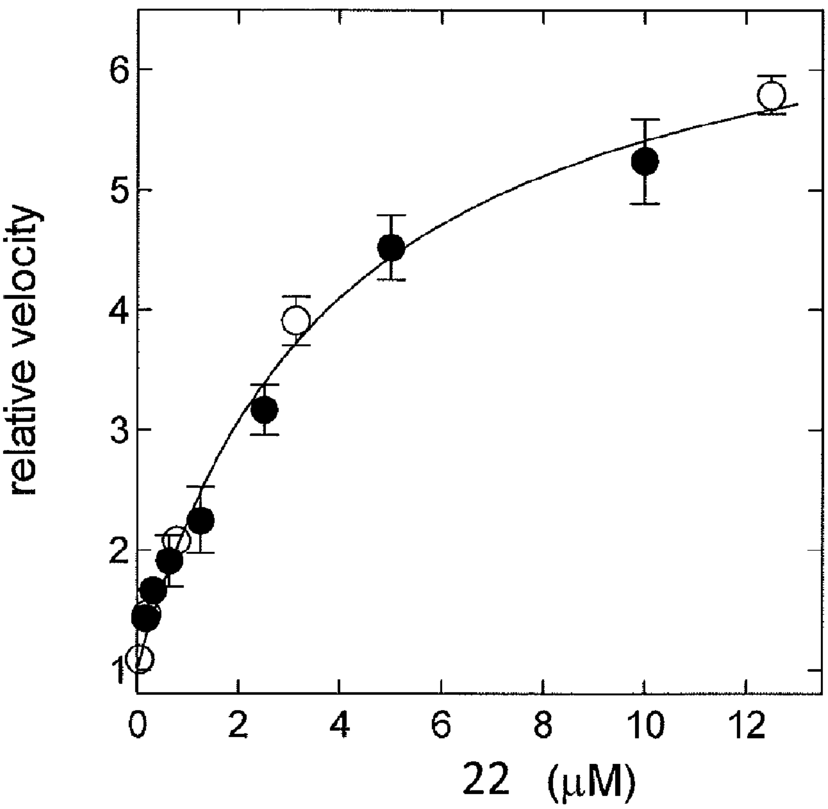

FIGURE 7

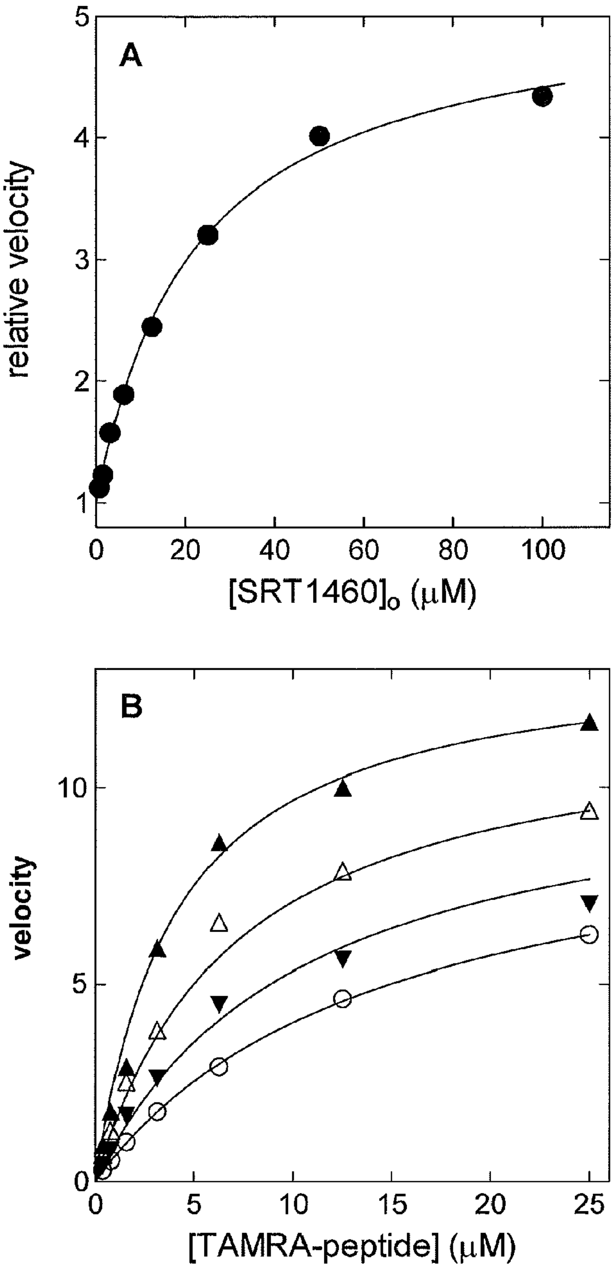
A
relative velocity
[SRT1460]o (μM)
B
velocity
[TAMRA-peptide] (μM)

# FIGURE 8

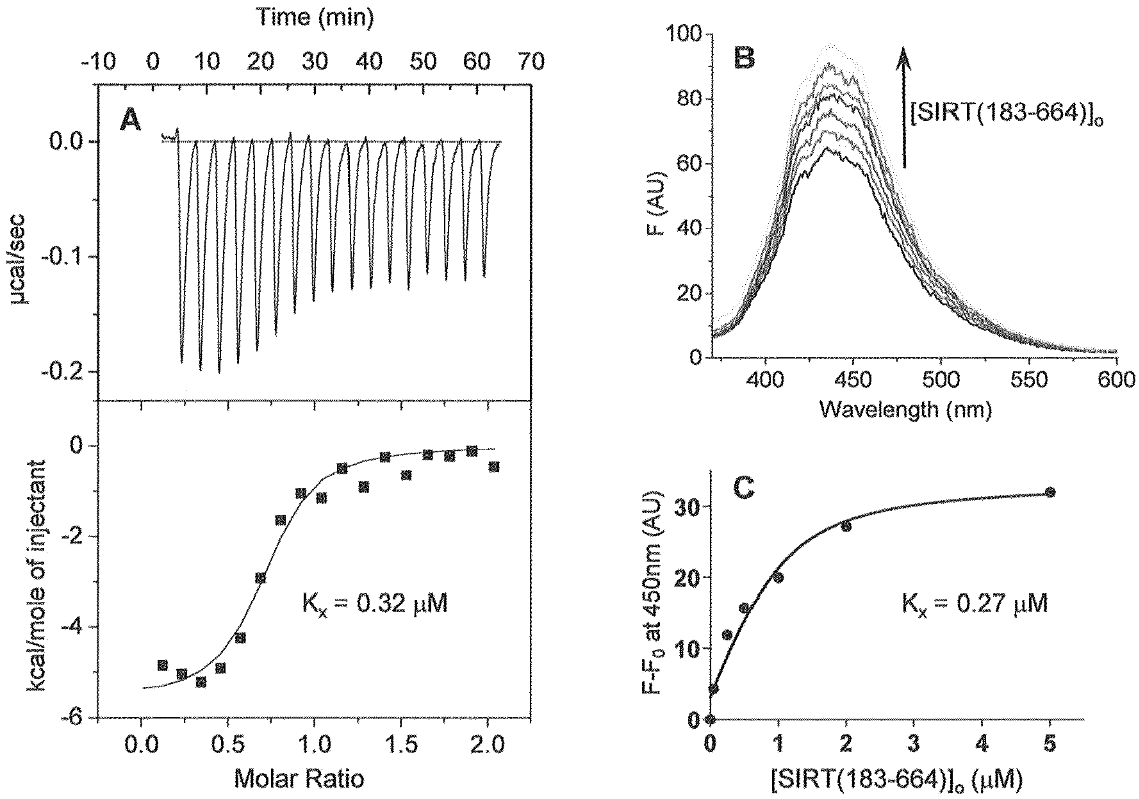
Time (min)
-10 0 10 20 30 40 50 60 70
A
0.0
-0.1
-0.2
μcal/sec
0
-2
-4
-6
kcal/mole of injectant
$K_x$ = 0.32 μM
0.0 0.5 1.0 1.5 2.0
Molar Ratio
100
80
60
40
20
0
B
[SIRT(183-664)]$_o$
F (AU)
400 450 500 550 600
Wavelength (nm)
C
30
20
10
0
F-$F_0$ at 450nm (AU)
$K_x$ = 0.27 μM
0 1 2 3 4 5
[SIRT(183-664)]$_o$ (μM)

FIGURE 9

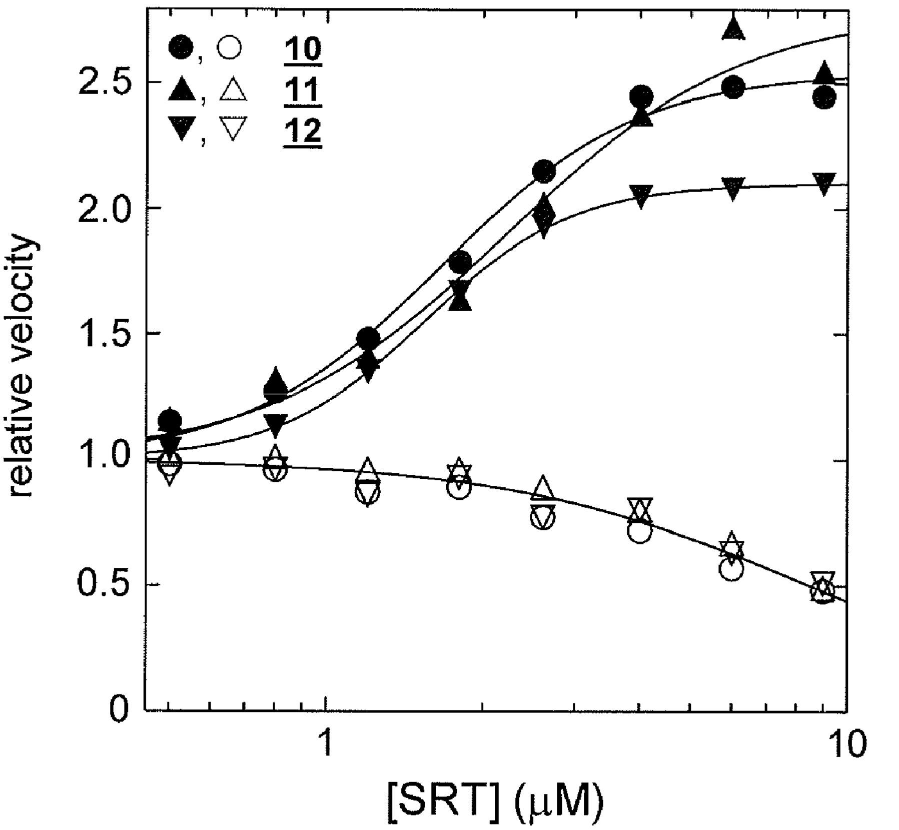


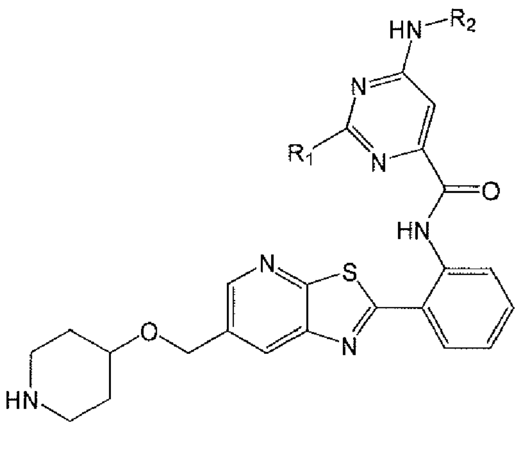

| | R1 | R2 |
| --- | --- | --- |
| **10** | $-(CH_2)_3CH_3$ | 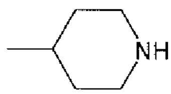 |
| **11** | $-CH_2CH(CH_3)_2$ | 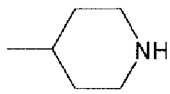 |
| **12** | $-(CH_2)_3CH_3$ | 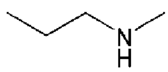 |

FIGURE 10

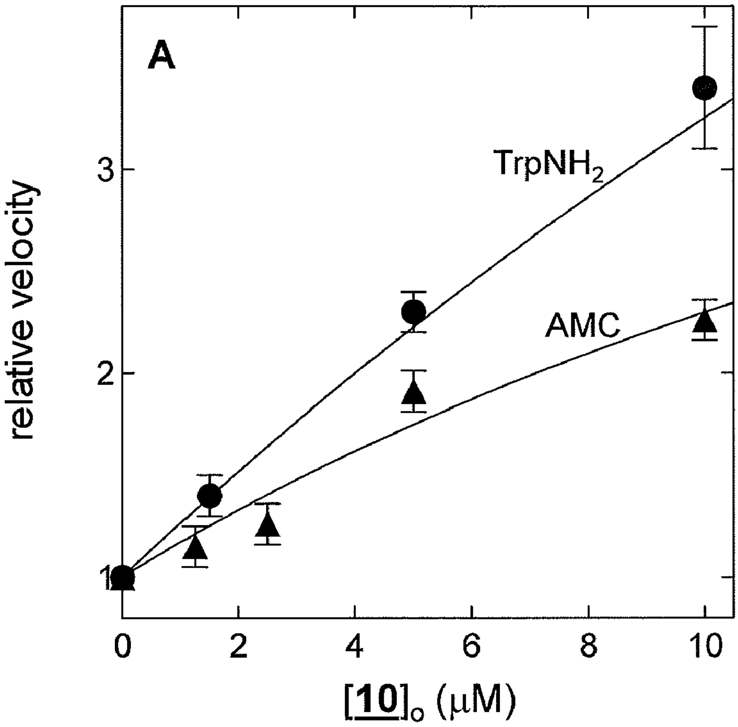
A
relative velocity
TrpNH$_2$
AMC
1
2
3
0
2
4
6
8
10
[10]$_o$ (μM)

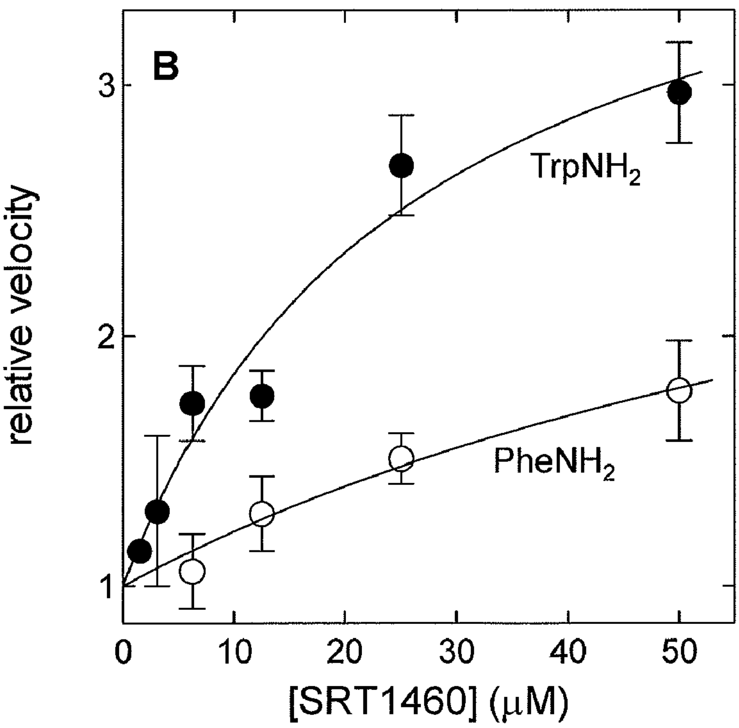
B
relative velocity
TrpNH$_2$
PheNH$_2$
1
2
3
0
10
20
30
40
50
[SRT1460] (μM)

**FIGURE 11**

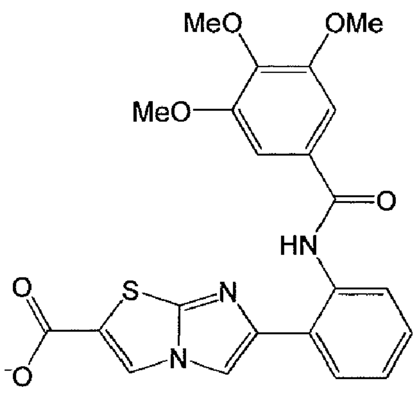

**23**

$EC_{1.5}$ = 0.5 μM

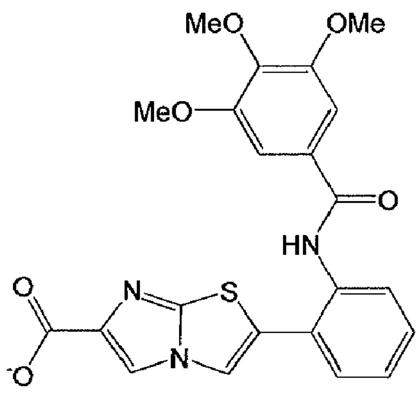

**24**

$EC_{1.5}$ > 50 μM

**FIGURE 12**

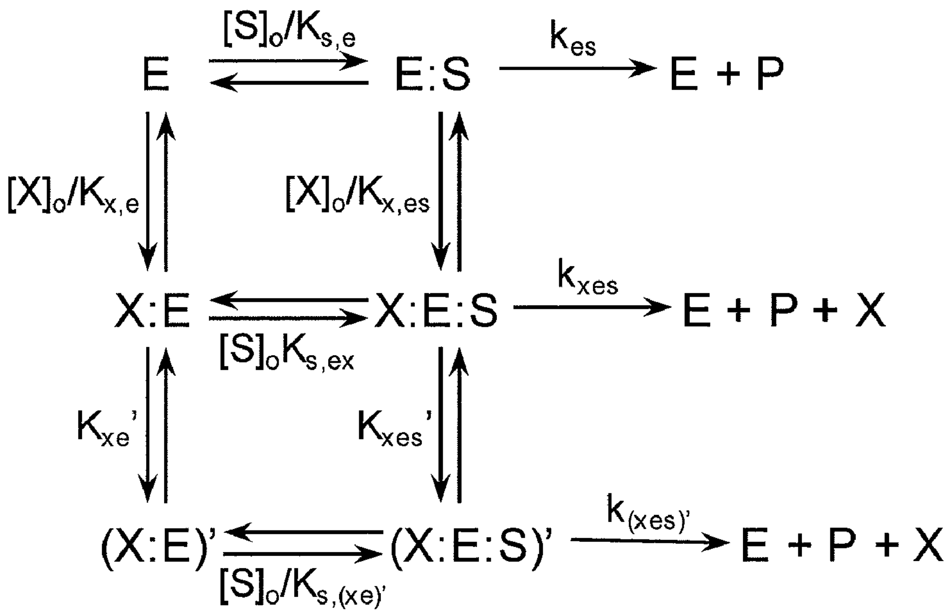
[S]o/Ks,e
E
E:S
kes
E + P
[X]o/Kx,e
[X]o/Kx,es
X:E
X:E:S
kxes
E + P + X
[S]oKs,ex
Kxe'
Kxes'
(X:E)'
(X:E:S)'
k(xes)'
E + P + X
[S]o/Ks,(xe)'

FIGURE 13

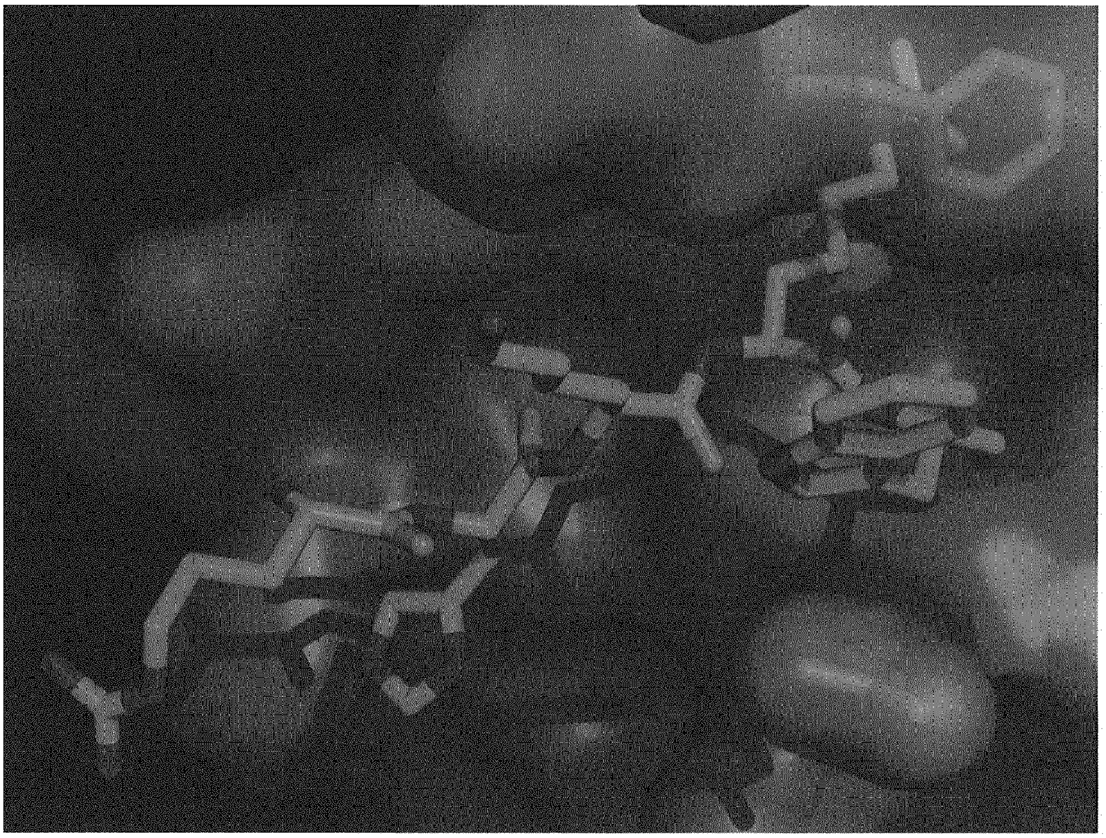

FIGURE 14

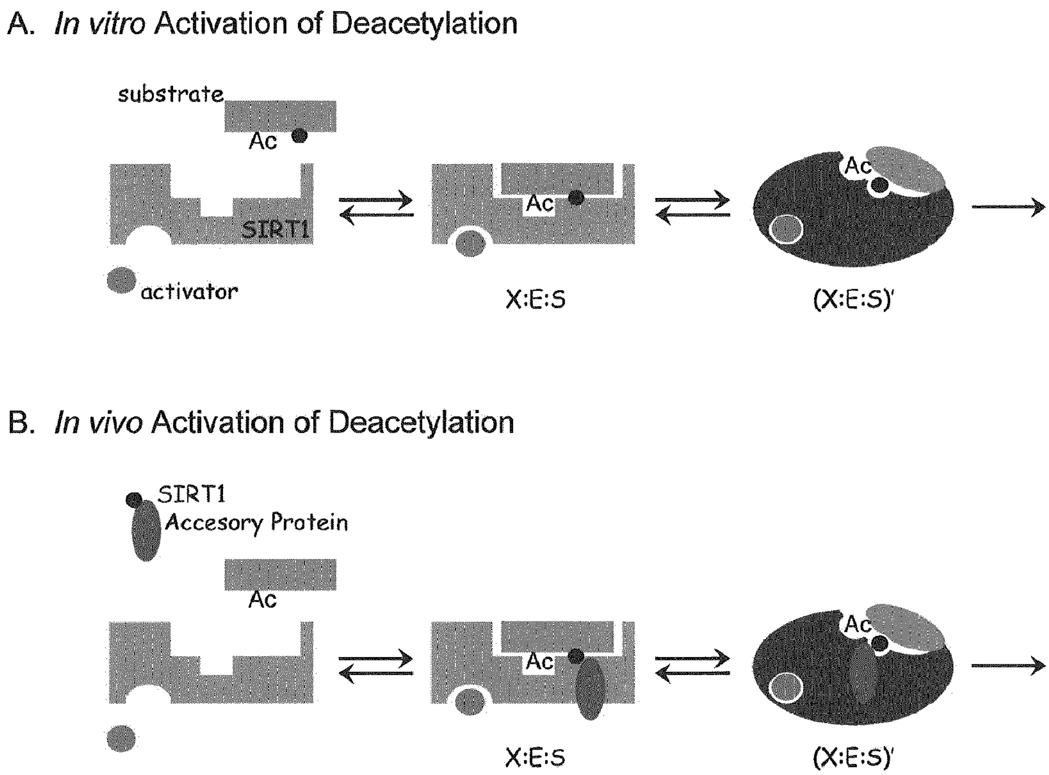
A. In vitro Activation of Deacetylation
substrate
Ac
SIRT1
activator
Ac
X:E:S
Ac
(X:E:S)'
B. In vivo Activation of Deacetylation
SIRT1
Accesory Protein
Ac
Ac
X:E:S
Ac
(X:E:S)'

Europäisches Patentamt
European Patent Office
Office européen des brevets

# EUROPEAN SEARCH REPORT

Application Number
EP 15 18 5484

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LIU Y ET AL: "High-throughput assays for sirtuin enzymes: A microfluidic mobility shift assay and a bioluminescence assay", ANALYTICAL BIOCHEMISTRY, ACADEMIC PRESS INC, NEW YORK, vol. 378, no. 1, 1 July 2008 (2008-07-01), pages 53-59, XP022679701, ISSN: 0003-2697, DOI: 10.1016/J.AB.2008.02.018 [retrieved on 2008-03-04] * the whole document * * In particular: Title; Abstract; Materials and methods section; p. 56-57, NAD+ bioluminescence assay; Fig. 4. * ----- | 1-11 | INV. C12Q1/37 |
| Y | M. KAEBERLEIN: "Substrate-specific Activation of Sirtuins by Resveratrol", JOURNAL OF BIOLOGICAL CHEMISTRY, vol. 280, no. 17, 11 February 2005 (2005-02-11), pages 17038-17045, XP055083761, ISSN: 0021-9258, DOI: 10.1074/jbc.M500655200 * the whole document * * In particular: Abstract; Materials and methods section; Fig. 4 * ----- | 1-11 | |
| | -/-- | | TECHNICAL FIELDS SEARCHED (IPC) |
| | | | C12Q |

The present search report has been drawn up for all claims

| Place of search | Date of completion of the search | Examiner |
|---|---|---|
| The Hague | 9 December 2015 | C.F. Angioni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 15 18 5484

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BLANDER GIL ET AL: "SIRT1 shows no substrate specificity in vitro", JOURNAL OF BIOLOGICAL CHEMISTRY, AMERICAN SOCIETY FOR BIOCHEMISTRY AND MOLECULAR BIOLOGY, vol. 280, no. 11, 1 March 2005 (2005-03-01), pages 9780-9785, XP002429821, ISSN: 0021-9258, DOI: 10.1074/JBC.M414080200 * the whole document * * In particular: Title; Abstract; Materials and methods section; Fig. 1. * ----- | 1-11 | |
| Y | DIRK BEHER ET AL: "Resveratrol is Not a Direct Activator of SIRT1 Enzyme Activity", CHEMICAL BIOLOGY & DRUG DESIGN, BLACKWELL PUBLISHING TD., OXFORD, GB, vol. 74, no. 6, 1 December 2009 (2009-12-01), pages 619-624, XP008162711, ISSN: 1747-0277, DOI: 10.1111/J.1747-0285.2009.00901.X [retrieved on 2009-10-20] * the whole document * * see in particular: experimental procedures section. * ----- -/-- | 1-11 | TECHNICAL FIELDS SEARCHED (IPC) |

The present search report has been drawn up for all claims

| Place of search | Date of completion of the search | Examiner |
|---|---|---|
| The Hague | 9 December 2015 | C.F. Angioni |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 18 5484

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MICHELLE PACHOLEC ET AL: "SRT1720, SRT2183, SRT1460, and Resveratrol Are Not Direct Activators of SIRT1", JOURNAL OF BIOLOGICAL CHEMISTRY, AMERICAN SOCIETY FOR BIOCHEMISTRY AND MOLECULAR BIOLOGY, US, vol. 385, no. 11, 12 March 2010 (2010-03-12), pages 8340-8351, XP008162683, ISSN: 0021-9258, DOI: 10.1074/JBC.M109.088682 [retrieved on 2010-01-08] * the whole document * * See in particular: materials and methods section. * | 1-11 | |
| X | US 2007/105109 A1 (GEESAMAN BARD J [US] ET AL) 10 May 2007 (2007-05-10) * the whole document * * See in particular: paragraphs 120-177. Of particular relevance are paragraphs 146 and 149. * | 1-11 | TECHNICAL FIELDS SEARCHED (IPC) |
| X | US 2005/136429 A1 (GUARENTE LEONARD P [US] ET AL) 23 June 2005 (2005-06-23) * the whole document * * See in particular: paragraphs 101, 117, 153, 181-220. Of particular relevance is paragraph 185. * | 1-11 | |
| X | US 2005/250794 A1 (NAPPER ANDREW [US] ET AL) 10 November 2005 (2005-11-10) * the whole document * * See in particular: paragraphs 235-267. Of particular relevance is paragraph 257. * | 1-11 | |

-/--

The present search report has been drawn up for all claims

2

| Place of search | Date of completion of the search | Examiner |
|---|---|---|
| The Hague | 9 December 2015 | C.F. Angioni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 18 5484

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZACHARY A. GURARD-LEVIN ET AL: "Combining Mass Spectrometry and Peptide Arrays to Profile the Specificities of Histone Deacetylases", CHEMBIOCHEM - A EUROPEAN JOURNAL OF CHEMICAL BIOLOGY., vol. 10, no. 13, 4 September 2009 (2009-09-04), pages 2159-2161, XP055235190, DE ISSN: 1439-4227, DOI: 10.1002/cbic.200900417 * the whole document * * In particular: P. 3, 2nd paragraph; Fig. 3. * ----- | 12 | |
| | | | TECHNICAL FIELDS SEARCHED (IPC) |

The present search report has been drawn up for all claims

2

| Place of search | Date of completion of the search | Examiner |
|---|---|---|
| The Hague | 9 December 2015 | C.F. Angioni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 18 5484

This annex lists the patent family members relating to the patent documents cited in the above-mentioned European search report. The members are as contained in the European Patent Office EDP file on
The European Patent Office is in no way liable for these particulars which are merely given for the purpose of information.

09-12-2015

| Patent document cited in search report | Publication date | Patent family member(s) | Publication date |
|---|---|---|---|
| US 2007105109 A1 | 10-05-2007 | US 2007105109 A1 | 10-05-2007 |
| | | WO 2005004814 A2 | 20-01-2005 |
| US 2005136429 A1 | 23-06-2005 | US 2005136429 A1 | 23-06-2005 |
| | | US 2011218229 A1 | 08-09-2011 |
| | | WO 2005002527 A2 | 13-01-2005 |
| US 2005250794 A1 | 10-11-2005 | CA 2550091 A1 | 07-07-2005 |
| | | EP 1694323 A2 | 30-08-2006 |
| | | JP 2007515429 A | 14-06-2007 |
| | | US 2005250794 A1 | 10-11-2005 |
| | | WO 2005060711 A2 | 07-07-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4727064 A, Pitha
- US 4683195 A, Mullis
- WO 2007019346 A, Nunes
- WO 2008156866 A, Bemis
- WO 2010003048 A, Vu


### Non-patent literature cited in the description


- **HAIGIS, M. C. ; SINCLAIR, D.** *Annu Rev Pathol,* 2010, vol. 5, 253-259
- **BAUR, J. A.** *Mech Aging Dev,* 2010
- **RUTANEN.** *Diabetes,* 2010
- **MILNE et al.** *Nature,* 2007, vol. 450, 712-716
- **BEMIS et al.** *Bioorg Med Chem Lett,* 2009, vol. 19, 2350-2353
- **VU et al.** *J. Med. Chem.,* 2009
- **FEIGE et al.** *Cell Metab,* 2008, vol. 8, 347-358
- **FUNK et al.** *J Pharmacol Exp Ther*
- **JIN et al.** *Protein Sci,* 2009, vol. 18, 514-525
- **LIU et al.** *Nature,* 2008, vol. 456, 269-273
- **SMITH et al.** *BMC Syst Biol,* 2009, vol. 3, 31
- **YAMAZAKI et al.** *Am J Physiol Endocrinol Metab,* 2009
- **YOSHIZAKI et al.** *Mol Cell Biol,* 2009, vol. 29, 1363-1374
- **HOWITZ et al.** *Nature,* 2003, vol. 425, 191-196
- **BORRA et al.** *J. Biol. Chem.,* 2005, vol. 280, 17187-17195
- **KAEBERLEIN et al.** *J. Biol. Chem.,* 2005, vol. 280, 17038-17045
- **BEHER et al.** *Chem Biol Drug Des,* 2009
- **PACHOLEC et al.** *J. Biol. Chem.,* 2010, vol. 285, 8340-8351
- **HIRAYMA et al.** *Nature,* 2007, vol. 450, 1086-90
- **SASSONE-CORSI et al.** *Cell,* 2008, vol. 134, 329-340
- **ISSELBACHER et al.** *Harrison's Principles of Internal Medicine,* 1994, 1814-1877
- **BITTERMAN et al.** *J. Biol. Chem.,* 2002, vol. 277, 45099
- **BEGLEY.** *Pharmacology & Therapeutics,* 2004, vol. 104, 29-45
- Encyclopedia of Pharmaceutical Technology. Marcel Dekker, 1992, vol. 9
- Cell Therapy: Stem Cell Transplantation, Gene Therapy, and Cellular Immunotherapy. Cambridge University Press, 1996
- **E. D. BALL ; J. LISTER ; P. LAW.** *Hematopoietic Stem Cell Therapy,* 2000
- Molecular Cloning A Laboratory Manual. Cold Spring Harbor Laboratory Press, 1989
- DNA Cloning. 1985, vol. I, II
- Oligonucleotide Synthesis. 1984
- Nucleic Acid Hybridization. 1984
- Transcription And Translation. 1984
- **R. I. FRESHNEY.** Culture Of Animal Cells. Alan R. Liss, Inc, 1987
- Immobilized Cells And Enzymes. IRL Press, 1986
- **B. PERBAL.** *A Practical Guide To Molecular Cloning,* 1984
- Methods In Enzymology. Academic Press, Inc,
- Gene Transfer Vectors For Mammalian Cells. Cold Spring Harbor Laboratory, 1987
- Methods In Enzymology. vol. 154, 155
- Immunochemical Methods In Cell And Molecular Biology. Academic Press, 1987
- Handbook Of Experimental Immunology. 1986, vol. I-IV
- Manipulating the Mouse Embryo. Cold Spring Harbor Laboratory Press, 1986
- **SMITH et al.** *Anal Biochem,* 2009, vol. 394, 101-109
- **VU et al.** *J Med Chem,* 2009
- **MARCOTTE et al.** *Anal Biochem,* 2004, vol. 332, 90-99
- **PACHOLEC et al.** *J. Biol. Chem.,* vol. 285, 8340-8351
- **BEHER.** *Chem Biol Drug Des,* 2009
- **JIN et al.** *J. Biol. Chem.,* 2009, vol. 284, 24394-24405
- **GUEX ; PEITSCH.** *Electrophoresis,* 1997, vol. 18, 2714-2723

- **AVALOS et al.** *Mol Cell,* 2002, vol. 10, 523-535
- **PERNI et al.** *J. Med. Chem.,* 2009, vol. 52, 1275-1283